# EUROPÄISCHE PATENTSCHRIFT

(11) **EP 1 173 044 B1**
(45) Veröffentlichungstag und Bekanntmachung des Hinweises auf die Patenterteilung: **29.08.2012**
(21) Anmeldenummer: 01115199.0
(22) Anmeldetag: 22.06.2001
(51) Int. Cl.: H04R 25/00, A61F 11/04, G10L 21/02, G10L 13/08

(54) **System zur Rehabilitation einer Hörstörung**
Implantable system for the rehabilitation of a hearing disorder
Système implantable de réhabilitation d'un trouble auditif

(30) Priorität: 30.06.2000 DE 10031832
(43) Veröffentlichungstag der Anmeldung: 16.01.2002
(73) Patentinhaber: Cochlear Limited, Lane Cove, NSW 2066 (AU)
(72) Erfinder: Leysieffer, Hans, Dr.-Ing., 82024 Taufkirchen (DE); Waldmann, Bernd, Dr. rer. nat., 81927 München (DE)
(74) Vertreter: Grünecker, Kinkeldey, Stockmair & Schwanhäusser

(56) Entgegenhaltungen:
- EP-A- 0 624 865
- EP-A- 1 006 511
- EP-A- 1 014 755
- EP-A- 1 083 769
- WO-A-90/07251
- WO-A-96/06586
- DE-A- 19 722 705
- US-A- 4 441 202
- US-A- 5 271 397
- ZADAK J ET AL: "AN APPLICATION OF MAPPING NEURAL NETWORKS AND A DIGITAL SIGNAL PROCESSOR FOR COCHLEAR NEUROPROSTHESES" BIOLOGICAL CYBERNETICS, SPRINGER VERLAG, HEIDELBERG, DE, Bd. 68, Nr. 6, 1. April 1993 (1993-04-01), Seiten 545-552, XP000362213 ISSN: 0340-1200
- LEISENBERG M: "Hearing aids for the profoundly deaf based on neural net speech processing" ACOUSTICS, SPEECH, AND SIGNAL PROCESSING, 1995. ICASSP-95., 1995 INTERNATIONAL CONFERENCE ON DETROIT, MI, USA 9-12 MAY 1995, NEW YORK, NY, USA,IEEE, US, Bd. 5, 9. Mai 1995 (1995-05-09), Seiten 3535-3538, XP010152110 ISBN: 0-7803-2431-5

## Beschreibung

Die vorliegende Erfindung betrifft ein System zur Rehabilitation einer Hörstörung, das mindestens einen schallaufnehmenden Sensor, eine elektronische Signalverarbeitungseinheit zur Audiosignalverarbeitung und -verstärkung, eine elektrische Energieversorgungseinheit, welche einzelne Komponenten des Systems mit Strom versorgt, und einen oder mehrere elektroakustische, elektromechanische oder rein elektrische ausgangsseitige Aktoren oder eine beliebige Kombination solcher Aktoren zur Stimulation des geschädigten Gehöres umfasst.

Unter Hörsystemen sollen vorliegend Systeme verstanden werden, bei denen das Schallsignal mit mindestens einem Sensor, der ein Schallsignal in ein elektrisches Signal umwandelt (Mikrofonfunktion), aufgenommen und elektronisch weiterverarbeitet und verstärkt wird und deren ausgangsseitiges Signal das geschädigte Gehör auf akustische, mechanische oder elektrische Weise oder durch eine beliebige Kombination dieser drei physikalischen Reizformen stimuliert.

Unter dem Begriff "Hörstörung" sollen vorliegend alle Arten von Innenohrschäden, kombinierten Innen- und Mittelohrschäden sowie auch zeitweise auftretende oder permanente Ohrgeräusche (Tinnitus) verstanden werden.

Das Hörsystem gemäß vorliegender Erfindung kann nichtimplantierbar oder mindestens teilweise implantierbar ausgebildet sein, und es umfasst mindestens einen schallaufnehmenden Sensor (Mikrofon), eine elektronische Anordnung zur Audiosignalverarbeitung und -verstärkung, eine elektrische Energieversorgungseinheit, welche einzelne Komponenten des Systems mit Strom versorgt, eine drahtlose, transkutane Ladevorrichtung im Falle eines Vollimplantates bei Verwendung eines implantatseitig sekundären elektrischen Speicherelements sowie eine ausgangsseitige aktorische Anordnung, die aus mindestens einem elektroakustischen Wandler bei einem konventionellen Hörgerät besteht oder aus mindestens einem elektromechanischen Wandler, der das geschädigte Innenohr direkt oder mittelbar über eine mechanische Ankopplung an Teile des Mittelohres mechanisch stimuliert oder einer intracochleären Reizelektrodenanordnung, die das geschädigte Innenohr durch elektrische Reizung mit mindestens einer Reizelektrode stimuliert, oder einer Reizelektrodenanordnung mit mindestens einer Reizelektrode, die den Hirnstamm, das heißt, Teile der höheren Hörbahn direkt elektrisch stimuliert ("Brainstem-Implant") oder einer beliebigen Kombination der genannten Stimulationsarten.

Konventionelle Hörgeräte mit ausgangsseitig akustischer Stimulation des geschädigten Gehörs, insbesondere des Innenohres, haben in den letzten Jahren bezüglich der elektronischen Signalverarbeitung erhebliche Verbesserungen erfahren, die insbesondere auf dem Einsatz moderner volldigitaler Signalprozessoren beruhen. Mit Hilfe dieser Prozessoren und der entsprechenden Signalverarbeitungssoftware kann die Rehabilitation der Hörstörung durch verfeinerte Anpassung an den individuellen Hörschaden optimiert werden. Insbesondere sind so erstmals rausch- beziehungsweise störsignalunterdrückende Algorithmen implementierbar, die besonders dem Umstand Rechnung tragen, dass vor allem Innenohrschwerhörige dann erhebliche Schwierigkeiten beim Sprachverstehen einzelner Personen haben, wenn sie sich in störlärmerfüllter Umgebung befinden.

Die Rehabilitation sensorischer Hörstörungen mit teilimplantierbaren, elektronischen Systemen hat in jüngerer Zeit ebenfalls einen bedeutenden Stellenwert erhalten. Insbesondere gilt dies für den Patientenkreis, bei dem das Gehör durch Unfall, Krankheit oder sonstige Einflüsse vollständig ausgefallen oder bereits von Geburt an nicht funktionsfähig ist. Ist in diesen Fällen nur das Innenohr (Cochlea) und nicht die nach zentral führende neuronale Hörbahn betroffen, kann mit elektrischen Reizsignalen der verbliebene Hörnerv stimuliert und somit ein Höreindruck erzeugt werden, der bis zu einem offenen Sprachverständnis führen kann. Bei diesen sogenannten Cochlea Implantaten wird in die Cochlea ein Reizelektroden-Array eingeführt, das von einem elektronischen System angesteuert wird, wobei dieses hermetisch dichte und biokompatibel eingekapselte Elektronikmodul operativ im knöchernen Bereich hinter dem Ohr (Mastoid) eingebettet ist. Das elektronische System enthält bei derzeit verfügbaren Systemen im wesentlichen nur Decodier- und Treiberschaltungen für die Reizelektroden. Die akustische Schallaufnahme, die Wandlung dieses Schallsignals in elektrische Signale und deren weitere Verarbeitung erfolgen extern in einem sogenannten Sprachprozessor, der außen am Körper getragen wird. Der Sprachprozessor setzt die vorverarbeiteten Signale entsprechend codiert auf ein hochfrequentes Trägersignal um, das über eine induktive Kopplung durch die geschlossene Haut (transkutan) zu dem Implantat übertragen wird. Das schallaufnehmende Mikrofon befindet sich außerhalb des Körpers und in den meisten Anwendungen in einem an der Ohrmuschel getragenen Gehäuse eines Hinter-dem-Ohr-Hörgerätes (HdO), und es ist über ein Kabel mit dem Sprachprozessor verbunden. Solche Cochlea-Implantat-Systeme sowie deren Komponenten und Prinzipien der transkutanen Signalübertragung sind beispielhaft in folgenden Patentschriften beschrieben: : US-A-5 070 535, US-A-4 441 210, EP-A-0 200 321, US-A-5 626 629. Verfahren zur Sprachaufbereitung und -Codierung bei Cochlea-Implantaten sind beispielsweise in folgenden Patentschriften angegeben: EP-A-0 823 188, EP-A-0 190 836, US-A-5 597 380, US-A-5 271 397, US-A-5 095 904, US-A-5 601 617, US-A-5 603 726.

Neben der Rehabilitation gehörloser beziehungsweise ertaubter Patienten mit Cochlea-Implantaten existieren seit geraumer Zeit Ansätze, Patienten mit einer sensorineuralen Hörstörung, die operativ nicht behebbar ist, mit teil- beziehungsweise vollimplantierbaren Hörgeräten eine bessere Rehabilitation als mit konventionellen Hörgeräten zu bieten. Das Prinzip besteht in den überwiegenden Ausführungsformen darin, ein Ossikel des Mittelohres oder das Innenohr direkt über einen mechanischen beziehungsweise hydromechanischen Reiz zu stimulieren und nicht über das verstärkte akustische Signal eines konventionellen Hörgerätes, bei dem das verstärkte Schallsignal dem äußeren Gehörgang zugeführt wird. Der aktorische Stimulus dieser elektromechanischen Systeme wird mit verschiedenen physikalischen Wandlerprinzipien realisiert wie zum Beispiel durch elektromagnetische und piezoelektrische Systeme. Der Vorteil dieser Geräte wird hauptsächlich in der gegenüber konventionellen Hörgeräten verbesserten Klangqualität und bei vollimplantierten Systemen in der Unsichtbarkeit der Hörprothese gesehen. Solche teil- und vollimplantierbaren elektromechanischen Hörgeräte sind beispielhaft von Yanigahara und Suzuki et al. (Arch Otolaryngol Head Neck, Surg-Vol 113, 1987, Seiten 869872; Hoke, M. (ed), Advances in Audiology, Vol. 4, Karger Basel, 1988), H.P. Zenner et al. "Erste Implantationen eines vollständig implantierbaren elektronischen Hörsystems bei Patienten mit Innenohrschwerhörigkeit", HNO 46:844-852; H. Leysieffer et al. "Ein vollständig implantierbares Hörsystem für Innenohrschwerhörige: TICA LZ 3001" HNO 46:853-863; H.P. Zenner et al. "Aktive elektronische Hörimplantate für Mittel- und Innenohrschwerhörige - eine neue Ära der Ohrchirurgie", HNO 45:749-774; H.P. Zenner et al. "Totally implantable hearing device for sensorineural hearing loss", The Lancet Vol. 352, No. 9142, Seite 1751; und in zahlreichen Patentschriften beschrieben, so unter anderem in: EP-A-0 263 254, EP-A-0 400 630, EP-A-0 499 940, US-A-3 557 775, US-A-3 712 962, US-A-3 764 748, US-A-5 411 467, US-A-4 352 960, US-A-4 988 333, US-A-5 015 224, US-A-5 015 225, US-A-5 360 388, US-A-5 772 575, US-A-5 814 095, US-A-5 951 601, US-A-5 977 689 und US-A-5 984 859. Dabei ist das Einbringen eines elektromechanischen Wandlers durch eine Eröffnung im Promontorium zur direkten Flüssigkeitsanregung im Innenohr in US-A-5 772 575, US-A-5 951 601, US-A-5 977 689 und US-A-5 984 859 beschrieben.

Seit kurzem sind solche teil- und vollimplantierbare Hörsysteme zur Rehabilitation eines Innenohrschadens in der klinischen Anwendung. Dabei zeigt sich je nach verwendetem physikalischen Prinzip des ausgangsseitigen elektromechanischen Wandlers und insbesondere dessen Ankopplungsart an die Ossikel des Mittelohres, dass die erreichten Ergebnisse der Verbesserung des Sprachverständnisses sehr unterschiedlich sein können. Dazu kommt, dass bei manchen Patienten kein ausreichender Lautstärkepegel erreicht werden kann. Dieser Aspekt ist spektral sehr unterschiedlich, was bedeuten kann, dass bei zum Beispiel mittleren und hohen Frequenzen die erzeugte Lautheit zwar ausreichend ist, jedoch nicht bei tiefen Frequenzen beziehungsweise umgekehrt.

Weiterhin kann die übertragbare spektrale Bandbreite begrenzt sein, so beispielsweise bei elektromagnetischen Wandlern auf tiefe und mittlere Frequenzen oder bei piezoelektrischen Wandlern auf mittlere und hohe Frequenzen. Darüber hinaus können sich nichtlineare Verzerrungen, die insbesondere bei elektromagnetischen Wandlern ausgeprägt sind, negativ auf die resultierende Klangqualität auswirken. Die mangelnde Lautheit führt insbesondere dazu, dass der audiologische Indikationsbereich für die Implantation eines elektromechanischen Hörsystems sehr eingeschränkt ist. Das bedeutet, dass Patienten zum Beispiel mit einem sensorineuralen Hörverlust von größer 50 dB HL (= hearing loss, Hörverlust) im Tieftonbereich mit einem piezoelektrischen System nur unzureichend versorgbar sind. Demgegenüber sind ausgeprägte Hochtonverluste mit elektromagnetischen Wandlern nur schwer versorgbar.

Viele Patienten mit einem Innenohrschaden leiden zusätzlich unter zeitweise auftretenden oder permanenten Ohrgeräuschen (Tinnitus), die operativ nicht behebbar sind und gegen die bis heute keine zugelassenen medikamentösen Behandlungsformen existieren. Daher sind sogenannte Tinnitus-Maskierer bekannt (WO-A-90/07251, EP-A-0 537 385, DE-U-296 16 956). Dies sind kleine, batteriebetriebene Geräte, die ähnlich einem Hörgerät hinter oder im Ohr getragen werden und durch artifizielle Schalle, die beispielsweise über einen Hörgeräte-Lautsprecher in den Gehörgang abgestrahlt werden, den Tinnitus auf psychoakustisch wirkende Weise verdecken ("maskieren") und das störende Ohrgeräusch so möglichst unter die Wahrnehmungsschwelle absenken. Die artifiziellen Schalle sind häufig Schmalbandgeräusche (zum Beispiel Terzrauschen), die in ihrer spektralen Lage und ihrem Lautstärkepegel über ein Programmiergerät einstellbar sind, um eine möglichst optimale Anpassung an die individuelle Ohrgeräuschsituation zu ermöglichen. Darüber hinaus existiert seit kurzem die sogenannte "Retraining-Methode", wobei durch die Kombination eines mentalen Trainingsprogrammes und die Darbietung eines breitbandigen Schalles (Rauschen) nahe der Ruhehörschwelle die Wahrnehmbarkeit des Tinnitus ebenfalls weitgehend unterdrückt werden soll (H. Knör, "Tinnitus-Retraining-Therapie und Hörakustik" Zeitschrift "Hörakustik" 2/97, Seiten 26 und 27). Diese Geräte werden auch als "Noiser" bezeichnet.

Bei beiden oben genannten Methoden zur apparativen Therapie des Tinnitus sind hörgeräteähnliche, technische Geräte außen am Körper im Ohrbereich sichtbar mitzuführen, die den Träger stigmatisieren und daher nicht gerne getragen werden.

In US-A-5 795 287 wird ein implantierbarer Tinnitusmaskierer mit "Direktantrieb" ("direct drive") des Mittelohres zum Beispiel über einen an die Ossikelkette angekoppelten elektromechanischen Wandler beschrieben. Dieser direkt gekoppelte Wandler kann vorzugsweise ein sogenannter "Floating Mass Transducer" (FMT) sein. Dieser FMT entspricht dem Wandler für implantierbare Hörgeräte, der in US-A-5 624 376 beschrieben ist.

In DE-C-198 58 398 und in DE-C-198 59 171 werden implantierbare Systeme zur Behandlung eines Tinnitus durch Maskierung und/oder Noiserfunktionen beschrieben, bei denen der signalverarbeitende elektronische Pfad eines teil- oder vollimplantierbaren Hörsystems durch entsprechende elektronische Module so ergänzt wird, dass die zur Tinnitusmaskierung oder zur Noiser-funktion notwendigen Signale in den Signalverarbeitungsweg der Hörgerätefunktion einspeisbar sind und die zugehörigen Signalparameter durch weitere elektronische Maßnahmen individuell an die pathologischen Bedürfnisse anpassbar sind. Diese Anpassbarkeit kann dadurch realisiert werden, dass die notwendigen Einstelldaten der Signalerzeugungs- und Einspeiselektronik in dem selben physikalischen und logischen Datenspeicherbereich des Implantatsystems hard- und softwaremäßig abgelegt beziehungsweise programmiert werden und über entsprechende elektronische Stellglieder die Einspeisung des Maskierer- beziehungsweise Noisersignals in den Audiopfad des Hörimplantats steuern.

Die oben beschriebenen mindestens teilweise implantierbaren Hörsysteme zur Rehabilitation einer Innenohrschädigung, die auf einem ausgangsseitigen elektromechanischen Wandler basieren, unterscheiden sich von herkömmlichen, konventionellen Hörgeräten wesentlich nur dadurch, dass der ausgangsseitige akustische Stimulus (verstärktes Schallsignal vor dem Trommelfell) durch einen verstärkten mechanischen Stimulus des Mittel- beziehungsweise Innenohres ersetzt wird. Der akustische Stimulus eines konventionellen Hörgerätes führt schließlich über die mechanische Anregung des Trommelfells und des sich anschließenden Mittelohres auch zu einem vibratorischen, das heißt mechanischen, Reiz des Innenohres. Bezüglich der sinnvollen Audiosignalvorverarbeitung bestehen grundlegend ähnliche beziehungsweise gleiche Anforderungen. Weiterhin wird in beiden Ausführungsformen letztendlich ausgangsseitig ein örtlich lokalisierter vibratorischer Stimulus an das geschädigte Innenohr geleitet (zum Beispiel verstärkte mechanische Schwingung des Steigbügels im ovalen Fenster des Innenohres).

Grundsätzlich besteht bei diesen heute klinisch eingesetzten Rehabilitationen eines Innenohrschadens durch aktive Hörsysteme mit externer akustischer oder implantiert elektromechanischer Stimulation ein weiterer gravierender Nachteil, der im folgenden zum Verständnis der vorliegenden Erfindung zusammenfassend beschrieben wird: Die überwiegende Mehrheit der Innenohrschwerhörigkeiten basiert auf einer mehr oder weniger ausgeprägten Schädigung der äußeren Haarzellen im Innenohr. Diese äußeren Haarzellen, die in einer Vielzahl im Cortischen Organ entlang der Basilarmembran angeordnet sind, bilden einen Teil des sogenannten cochleären Verstärkers, der je nach örtlicher Anregung der Basilarmembran aufgrund einer Wandlerwellenausbildung diesen örtlichen Anregungsbereich bei kleinen Pegeln und damit kleinen Wandlerwellenamplituden mechanisch aktiv entdämpft und somit zu einer Empfindlichkeitssteigerung führt. Diese aktive Entdämpfung beruht auf einem sehr komplexen, efferent gesteuerten Prozess, der hier nicht näher beschrieben wird. Es wird weiterhin angenommen, dass sich bei sehr hohen Pegeln der Innenohranregung aufgrund hoher Lautstärke dieser Effekt in seiner Wirkung umkehrt und damit die Wandlerwellenamplitude örtlich herabsetzt und damit aktiv bedämpft. Diese nichtlineare Kennlinie des cochleären Verstärkers, der in mehreren hundert örtlich begrenzt wirkenden Funktionseinheiten entlang des Cortischen Organs angeordnet ist, ist für die Funktion des gesunden Innenohres von maßgeblicher Bedeutung. Bei teilweisem oder vollständigem Ausfall der äußeren Haarzellen entstehen neben dem Empfindlichkeitsverlust, der zu einer Hörschwellenanhebung führt, jedoch noch weitere Nachteile: die beschriebene aktive Entdämpfung der Basilarmembran führt zu hohen Güten der Einhüllenden der Wandlerwellen, die wesentlich für das Frequenzunterscheidungsvermögen (Tonhöhenunterschiede) verantwortlich sind. Fehlt diese hohe Güte durch Ausfall oder Teilschädigung der äußeren Haarzellen, kann der Betroffene deutlich schlechter Tonhöhenunterschiede wahrnehmen. Die Anhebung der Hörschwelle führt darüber hinaus zu einer Verringerung des Dynamikbereiches, da sich die obere Empfindungsgrenze (Unbehaglichkeitsschwelle) bei einer Innenohrschwerhörigkeit nicht mit anhebt. Diese Reduktion der Dynamik führt zu einer Versteilerung des Lautstärkeempfindens, das als positives Recruitment bezeichnet wird. Die beschriebenen Effekte, die durch Schädigung oder Ausfall der äußeren Haarzellen hervorgerufen werden, führen in der Gesamtwirkung für den Betroffenen zur Minderung der Sprachverständlichkeit insbesondere in störlärmerfüllter Umgebung (zusammenfassende Darstellung in Zenner, H.P.: "Hören", Georg Thieme Verlag Stuttgart, New York, 1994, Seiten 20-23, 107 und 108, und LePage, E.W., Johnstone, M.B.: "Non-linear mechanical behaviour of the basilar membrane in the basal turn of the guinea pig cochlea". Hearing Research 2 (1980), 183-189).

Wesentliche Konsequenz dieses beschriebenen Mechanismus ist, dass sowohl bei konventionellen, akustischen Hörgeräten wie auch bei teil- oder vollimplantierbaren Hörsystemen die wichtigen Funktionen der geschädigten äußeren Haarzellen und damit des cochleären Verstärkers nicht ersetzt oder zumindest teilweise wiederhergestellt werden können. Aus DE-C-198 40 211 ist eine Wandleranordnung für teil- oder vollimplantierbare Hörgeräte zur direkten mechanischen Anregung des Mittel- oder Innenohres bekannt, die mit einem piezoelektrischen Wandlerelement und zusätzlich mit einem elektromagnetischen Wandler versehen ist, die in einem gemeinsamen Gehäuse untergebracht sind und die beide über dasselbe Koppelelement mit einem Mittelohr-Ossikel oder direkt mit dem Innenohr koppelbar sind. Es sind ferner implantierbare Hörsysteme bekannt (WO-A-99/08476 entsprechend US-A-5 997 466, WO-A-99/08480 entsprechend US-A-6 005 955), die mit zwei oder mehr ausgangsseitigen elektromechanischen Wandlern in einer Anordnung oder in örtlich getrennten Anordnungen arbeiten. Diese Ausführungsformen sind aber eindeutig dahingehend beschrieben, dass die Systemauslegung mit mehr als einem Wandler eine lineare Superposition der Auslenkungsfrequenzgänge der einzelnen Wandler ermöglicht, die im Ergebnis eine spektral möglichst optimierte beziehungsweise gezielt frequenzabhängig einstellbare oder programmierbare ausgangsseitige Anregungsform der Cochlea ermöglicht und somit zu einem spektral ausgeglichenen und ausreichenden Lautstärkeeindruck des Implantatsystems führen soll. Mit diesen Maßnahmen soll also der beschriebene Nachteil unzureichender Lautheit beziehungsweise zu eingeschränkter audiologischer Indikationsbereich bei implantierbaren, elektromechanischen Hörsystemen gemindert beziehungsweise umgangen werden. Eine Rehabilitation des cochleären Verstärkers mit den oben beschriebenen Merkmalen ist durch diese Ausführungsformen beziehungsweise beschriebenen Signalvorverarbeitungsmethoden jedoch nicht möglich.

Bei den Cochlea Implantaten (CI) werden als aktorische Stimuli heute ausschließlich elektrische Reizsignale verwendet. Nach der Implantation eines CI bei völlig ertaubten beziehungsweise gehörlosen Patienten ist im Regelfall ein Training zur Hörrehabilitation notwendig, da die artifiziellen Reize gelernt werden müssen, weil sie prinzipiell nicht der biologisch adäquaten Reizform des Innenohres entsprechen. Dem gegenüber entfällt diese Rehabilitationsphase nach Implantation eines elektromechanischen Hörsystems bei Schwerhörigen, da die mechanische Reizform, wie oben beschrieben, biologisch adäquat ist und letztendlich einer Versorgung mit einem Hörgerät zumindest bezüglich der grundsätzlichen Funktion weitgehend entspricht, das heißt, der stimulierende Reiz am Eingang des Innenohres ist vibratorischer Natur.

Seit kurzer Zeit ist wissenschaftlich aus CI-Implantationen bekannt, dass auch bei nicht vollständiger Taubheit CIs erfolgreich angewendet werden können, wenn mit einem konventionellen Hörgerät keine ausreichende Sprachdiskrimination mehr erreicht werden kann. Interessanterweise konnte nachgewiesen werden, dass die wesentlichen Innenohrstrukturen, die die akustische Resthörigkeit ermöglichen, zum Teil oder weitgehend langzeitstabil erhalten werden können, wenn eine CI-Elektrode in die Cochlea eingeführt wird (Ruh, S. et al.: "Cochlear Implant bei resthörigen Patienten", Laryngo-Rhino-Otol. 76 (1997), 347-350; Müller-Deile, J. et al.: "Cochlear-Implant-Versorgung bei nicht tauben Patienten?", Laryngo-Rhino-Otol. 77 (1998), 136-143; Lehnhardt, E.: "Intracochlear placement of cochlear implant electrodes in soft surgery technique", HNO 41 (1993), 356-359). Daraus kann geschlossen werden, dass bei erwartungsgemäß weitergehender klinischer und audiologischer Forschung in absehbarer Zeit CI-Elektroden bei Resthörigkeit klinisch sicher so intracochleär plaziert werden können, dass die verbleibenden Innenohrstrukturen langzeitstabil erhaltbar sind und somit auch auf biologisch adäquatem Weg, das heißt vibratorisch weiterhin stimulierbar sind und zu einem verwertbaren Höreindruck führen. Daher finden sich in der jüngeren Patentliteratur neue Ansätze, den geschädigten cochleären Verstärker durch ein mehrkanaliges, intracochleäres Wandlerarray mit rein mechanischer oder gemischt mechanisch/elektrischer Reizform zu "ersetzen" (ältere EP-Patentanmeldungen 01 109 192.3, 01 109 191.5 und 00 119 195.6). Ob diese Lösungen zu einer deutlichen Verbesserung des Sprachverständnisses insbesondere in Störschallumgebung führen werden, ist heute jedoch noch nicht absehbar.

Bei allen beschriebenen Verfahren bleibt also bei den heute verfügbaren Systemen der gravierende Nachteil bestehen, dass insbesondere das Sprachverständnis in störlärmerfüllter Umgebung erheblich reduziert ist. Dies gilt insbesondere bei Cochlea-Implantaten, bei denen die Patienten aufgrund der physiologisch nicht adäquaten elektrischen Reizform quasi eine neue Sprache erlernen müssen, deren Verständnis beziehungsweise Interpretation naturgemäß anfälliger ist gegen Störsignalanteile.

Insbesondere bei vollimplantierbaren Hörsystemen kann dieser Nachteil durch eine optimierte Mikrofonposition nahe dem Trommelfell (EP-A-0 831 673, EP-A-0 831 674, US-A-5 814 095) gemindert werden, da so die natürliche Richtwirkung des Außenohres genutzt wird. Solche Richtwirkungseffekte werden bei konventionellen Hörgeräten durch die geeignete Verschaltung mehrerer, örtlich getrennt angeordneter Mikrofone angenähert. Diese Maßnahmen sind jedoch relativ wirkungslos, wenn Nutz- und Störschall aus der gleichen Richtung kommen.

Diesen gravierenden Nachteil können moderne digitale Sprachverarbeitungsalgorithmen mit Störsignalminderung nur bis zu einem gewissen Maß reduzieren. Beispielsweise wird das Eingangssignal in mehrere Frequenzbänder unterteilt. In jedem Band wird anhand einfacher signalstatistischer Kriterien (zum Beispiel Modulationsparameter) bewertet, ob es sich in diesem Band um ein Sprach- oder Störsignal handelt. Bänder, in denen der Algorithmus Sprachsignale detektiert, werden in ihrer Verstärkung angehoben, wohingegen Bänder, in denen vorwiegend Störschall dominiert, abgeschwächt werden. Wesentlich ist hier, dass dieses heute vielfach eingesetzte Verfahren das Signal-Stör-Verhältnis in einem Band nicht verändern und damit auch nicht verbessern kann. Allen eingesetzten Analyseverfahren ist gemeinsam, dass sie nur elementare signalstatistische Parameter (zum Beispiel Amplitudenmodulationstiefe und -frequenz) verwenden.

Weiterhin ist allen beschriebenen Rehabilitationsverfahren gemeinsam, dass sie das Audiobeziehungsweise Sprachsignal zwar analysieren, bearbeiten und verstärken und bei Cochlea-Implantaten in eine neue Reizform codieren. Die Übertragung von direkter Sprachinformation in quasi Echtzeit bleibt jedoch erhalten. Daraus resultiert, dass auch Störsignalanteile durch Analyseverfahren zwar näherungsweise erkannt und reduziert werden können. Eine echte Separierung von Sprach- und Störinformation findet jedoch nicht statt.

Das Dokument EP 1 006 511 A1 zeigt ein System zur Rehabilitation einer Hörstörung, das mindestens einen Schallsensor eine elektronische Signalverarbeitungseinheit, eine elektrische Energieversorgungseinheit, und eine Aktoranordnung umfasst, die mit mehreren elektroakustischen Aktoren zur Stimulation eines geschädigten Gehörs versehen ist, wobei die Signalverarbeitungseinheit ein Sprachanalyse - beziehungsweise - erkennungsmodul und ein Sprachsynthesemodul aufweist.

Der Erfindung liegt die Aufgabe zugrunde, ein System zur Rehabilitation einer Hörstörung zu schaffen, das in der Lage ist, am Ausgang des Systems eine von Störschall mindestens weitgehend befreite Sprache anzubieten.

Diese Aufgabe wird duch ein System zur Rehabilitation einer Hörstörung gemäβ Anspruch 1 gelöst.

Bei dem erfindungsgemäßen System werden auf elektronische Weise eine Sprachanalyse beziehungsweise Spracherkennung und eine Sprachsynthese zur Verbesserung der Übertragung von Sprachinformation insbesondere in störlärmerfüllter Umgebung durchgeführt. Damit werden die vorstehend geschilderten Nachteile grundsätzlich umgangen. Ein elektronisches Signalverarbeitungssystem nimmt eine echte Sprachinformationsanalyse (Sprachinformationssegmentierung oder -erkennung) vor. Aufgrund der dadurch gewonnen Informationen wird eine Sprachausgabe erzeugt, die auf einer echten Synthese beruht. Somit wird ein rein artifiziell erzeugtes, synthetisiertes Sprachsignal ausgegeben, das praktisch ohne irgendwelche eingangsseitige Störsignalanteile ist. Es erfolgt mindestens näherungsweise eine vollständige Störsignalbefreiung, was unter anderem zu einer wesentlichen Verbesserung der Übertragung von Sprachinformation in störlärmerfüllter Umgebung führt. Ein weiterer Vorteil der vorliegenden Lösung besteht darin, dass insbesondere bei Hörsystemen mit akustischer und/oder mechanischer ausgangsseitiger Stimulation des Gehörs Rückkopplungen auf den schallaufnehmenden Sensor beziehungsweise das Mikrofon weitgehend vermieden beziehungsweise vollständig eliminiert werden.

Vorteilhafte weitere Ausgestaltungen der Erfindung ergeben sich aus den Unteransprüchen.

Die Signalverarbeitungseinheit weist bevorzugt einen digitalen Signalprozessor auf, der in Software realisierte Module zur Sprachanalyse und -synthese enthält. Diese Module sind vorteilhaft lernfähig aufgebaut und bedarfsweise umprogrammierbar, insbesondere um ein Optimieren der Sprachanalyse und -synthese zu ermöglichen.

Vorzugsweise ist zur Analyse des Eingangssignals ein digital realisiertes, mit automatischen Algorithmen arbeitendes neuronales Netzwerk vorgesehen, das im Zuge der Analyse das Eingangssignal phonetischen oder lexikalischen Kategorien zuordnet. Diese phonetischen oder lexikalischen Informationen stellen die Eingangssignale für ein nachgeordnetes Sprachsynthesemodul dar, dessen Ausgangssignal anschließend näherungsweise rauschfrei auf akustische, mechanische, elektrische oder beliebig kombinatorische Weise dem geschädigten Gehör übermittelt wird. Zur Vermeidung eines ausgeprägt monotonen Eindrucks der resynthetisierten Sprache ist es sehr sinnvoll, eine Anordnung vorzusehen, die Informationen über den sierten Sprache ist es sinnvoll, eine Anordnung vorzusehen, die Informationen über den emotionalen Zustand des Sprechers oder seine Intention bei zum Beispiel Fragestellungen oder Aufforderungen extrahiert und mitüberträgt, das heißt, im vorliegenden Fall geeignet synthetisiert. Solche zusätzlichen Informationen werden allgemein als Prosodie der Sprache bezeichnet. Die Erkennung dieser prosodischen Merkmale kann zum Beispiel in der Extraktion der Höhe und des Verlaufs der Sprachgrundfrequenz bei stimmhaften Lauten wie Vokalen bestehen. Auf der Syntheseseite des Systems wird das Ausgangssignal entsprechend moduliert, um diese Information zu übermitteln.

Weiterhin kann es sinnvoll sein, das Analyse- und Synthesesystem zeitweise auszuschalten und das Sprachsignal beziehungsweise das Audiosignal wie zum Beispiel Musik auf bekannte Weise zu übertragen, wenn kein oder nur sehr wenig Störschall vorliegt. Diese Funktion kann automatisch erfolgen oder durch eine Fernbedienungsfunktion durch den Anwender.

Grundsätzliche Verfahren zur Sprachanalyse, -erkennung und -synthese, die in vorliegender Erfindung zur Anwendung kommen können, sind beschrieben in: WO 99/59134, US-A-5 483 617, US-A-4 820 059, US-A 4 813 076, US-A-5 749 065, US-A-5 305 420, US-A-6 029 131 und insbesondere von Eric Mankin: "Machine demonstrates superhuman speech recognition abilities" in USC News Service, Release number: 0999025, Release date: September 30, 1999.

In weiterer Ausgestaltung der Erfindung kann die Signalverarbeitungseinheit Softwaremodule enthalten, die parallel zum Hörgerätebetrieb die Maskierung eines Tinnitus ermöglichen. Insbesondere im Falle der nachstehend beschriebenen mehrkanaligen Hörsysteme kann ein zumindest peripher zu lokalisierender Tinnitus effektiver maskiert werden als mit konventionellen Tinnitus-Maskern.

Die Signalverarbeitungseinheit weist zweckmäßig eine Vorverarbeitungsanordnung zum Vorverstärken und/oder Filtern sowie zum Analog/Digital- (A/D-) Wandeln der Schallsensorsignale auf. Sie kann insbesondere einen Antialiasing-Filter umfassen. Bei Vorhandensein von mehreren Schallsensoren und/oder von mehreren ausgangsseitigen Aktoren ist zweckmäßig jedem Schallsensor ein eigener Analog/Digital-Wandler nachgeschaltet beziehungsweise jedem ausgangsseitigen Aktor ein eigener Digital/Analog-Wandler vorgeschaltet.

Die Signalverarbeitungseinheit weist vorteilhaft einen digitalen Signalprozessor zum Verarbeiten der A/D-gewandelten und gegebenenfalls mittels der Vorverarbeitungsanordnung vorverarbeiteten Schallsensorsignale und/oder zum Generieren von digitalen Signalen für eine Tinnitusmaskierung auf.

Das vorliegende Hörsystem kann nichtimplantierbar aufgebaut sein. Vorzugsweise ist es jedoch mindestens teilweise implantierbar ausgebildet, wobei dann eine bevorzugt PC-basierte, drahtlose Telemetrieeinrichtung zur Übertragung von Daten zwischen einem implantierten Teil des Systems und einer externen Einheit, insbesondere einem externen Programmiersystem, vorgesehen ist.

Die vorhandenen Softwaremodule des Hörsystems können statisch in der Weise ausgelegt sein, dass sie aufgrund wissenschaftlicher Erkenntnisse einmalig in einem Programmspeicher des digitalen Signalprozessors abgelegt werden und unverändert bleiben. Liegen dann aber später zum Beispiel aufgrund neuerer wissenschaftlicher Erkenntnisse verbesserte Algorithmen zur Sprachsignalaufbereitung und -verarbeitung vor und sollen diese genutzt werden, muss bei einem implantierten System durch einen invasiven, operativen Patienteneingriff das gesamte Implantat oder das Implantatmodul, das die entsprechende Signalverarbeitungseinheit enthält, gegen ein neues mit der veränderten Betriebssoftware ausgetauscht werden. Dieser Eingriff birgt erneute medizinische Risiken für den Patienten und ist mit hohem Aufwand verbunden.

Diesem Problem kann in weiterer Ausgestaltung der Erfindung dadurch begegnet werden, dass bei einem mindestens teilimplantierbaren und mit der erwähnten Telemetrieeinrichtung versehenen Hörsystem dem Signalprozessor zur Aufnahme und Wiedergabe eines Betriebsprogramms eine wiederholt beschreibbare, implantierbare Speicheranordnung zugeordnet ist und mindestens Teile des Betriebsprogramms durch von der externen Einheit über die Telemetrieeinrichtung übermittelte Daten geändert oder ausgetauscht werden können. Auf diese Weise lässt sich nach Implantation des implantierbaren Systems die Betriebssoftware als solche verändern oder auch vollständig austauschen, wie dies für im übrigen bekannte Systeme zur Rehabilitation von Hörstörungen in DE-C-199 15 846 erläutert ist.

Bevorzugt ist die Auslegung so beschaffen, dass darüber hinaus bei vollimplantierbaren Systemen auch in an sich bekannter Weise Betriebsparameter, das heißt patientenspezifische Daten, wie beispielsweise audiologische Anpassdaten, oder veränderbare Implantatsystemparameter (zum Beispiel als Variable in einem Softwareprogramm zur Regelung einer Batterienachladung) nach der Implantation transkutan, das heißt drahtlos durch die geschlossene Haut, in das Implantat übertragen und damit verändert werden können. Dabei sind bevorzugt nicht nur das Sprachanalyse- oder -erkennungsmodul und das Sprachsynthesemodul, sondern auch weitere in der Signalverarbeitungseinheit und/oder in der Energieversorgungseinheit implementierte Softwaremodule dynamisch (lernfähig) oder umprogrammierbar ausgelegt. Insbesondere können die Softwaremodule adaptiv ausgelegt sein, und eine Parameteranpassung kann durch "Training" durch den Implantatträger und weitere Hilfsmittel vorgenommen werden.

Die Speicheranordnung zum Speichern von Betriebsparametern und die Speicheranordnung zur Aufnahme und Wiedergabe des Betriebsprogramms können als voneinander unabhängige Speicher implementiert sein; es kann sich jedoch auch um einen einzigen Speicher handeln, in dem sowohl Betriebsparameter als auch Betriebsprogramme abgelegt werden können.

Die erläuterte Ausgestaltung erlaubt eine Anpassung des Systems an Gegebenheiten, die erst nach Implantation eines implantierbaren Systems zu erfassen sind. So sind beispielsweise bei einem mindestens teilweise implantierbaren Hörsystem zur Rehabilitation einer monauralen oder binauralen Innenohrstörung sowie eines Tinnitus mit mechanischer Stimulation des Innenohres die sensorischen (Schallsensor beziehungsweise Mikrofon) und aktorischen (Ausgangsstimulator) biologischen Schnittstellen immer abhängig von den anatomischen, biologischen und neurophysiologischen Gegebenheiten, z.B. von dem interindividuellen Einheilprozess. Diese Schnittstellenparameter können individuell insbesondere auch zeitvariant sein. So können beispielsweise das Übertragungsverhalten eines implantierten Mikrofons aufgrund von Gewebebelägen und das Übertragungsverhalten eines an das Innenohr angekoppelten elektromechanischen Wandlers aufgrund unterschiedlicher Ankopplungsqualität interindividuell und individuell variieren. Solche Unterschiede der Schnittstellenparameter, die sich bei den aus dem Stand der Technik bekannten Vorrichtungen nicht einmal durch den Austausch des Implantats mindern beziehungsweise eliminieren ließen, können vorliegend durch Veränderung beziehungsweise Verbesserung der Signalverarbeitung des Implantats optimiert werden.

Bei einem mindestens teilweise implantierbaren Hörsystem kann es sinnvoll oder notwendig werden, nach Implantation verbesserte Signalverarbeitungsalgorithmen zu implementieren. Dabei sind insbesondere zu nennen:
- Sprachanalyse- und Sprachsyntheseverfahren (z.B. Optimierung einer Fast-FourierTransformation (FFT)),
- statische oder adaptive Störschallerkennungsverfahren,
- statische oder adaptive Störschallunterdrückungsverfahren,
- Verfahren zur Optimierung des systeminternen Signal-Rauschabstandes,
- optimierte Signalverarbeitungsstrategien bei progredienter Hörstörung,
- ausgangspegelbegrenzende Verfahren zum Schutz des Patienten bei Implantatfehlfunktionen beziehungsweise externen Fehlprogrammierungen,
- Verfahren zur Vorverarbeitung mehrerer Sensor-(Mikrofon-)signale, insbesondere bei binauraler Positionierung der Sensoren,
- Verfahren zur binauralen Verarbeitung zweier oder mehrerer Sensorsignale bei binauraler Sensorpositionierung z.B. Optimierung des räumlichen Hörens beziehungsweise Raumorientierung,
- Phasen- beziehungsweise Gruppenlaufzeit-Optimierung bei binauraler Signalverarbeitung,
- Verfahren zur optimierten Ansteuerung der Ausgangsstimulatoren, insbesondere bei binauraler Positionierung der Stimulatoren

Mit dem vorliegenden System lassen sich auch nach der Implantation unter anderem die folgenden Signalverarbeitungsalgorithmen implementieren:
- Verfahren zur Sprachanalyse und zur Sprachsynthese,
- Verfahren zur Rückkopplungsunterdrückung beziehungsweise -minderung,
- Verfahren zur Optimierung des Betriebsverhaltens des beziehungsweise der ausgangsseitigen Aktoren (z.B. Frequenz- und Phasengangoptimierung, Verbesserung des Impulsübertragungsverhaltens),
- Dynamik-Kompressionsverfahren, Datenreduktionsverfahren,
- Signalverarbeitungsmethoden zur Recruitment-Kompensation bei Innenohrschwerhörigkeiten.

Des weiteren ist bei Implantatsystemen mit einer sekundären Energieversorgungseinheit, das heißt einem nachladbaren Akkumulatorsystem, aber auch bei Systemen mit primärer Batterieversorgung davon auszugehen, dass diese elektrischen Energiespeicher mit voranschreitender Technologie immer größere Lebensdauern und damit steigende Verweilzeiten im Patienten ermöglichen. Es ist davon auszugehen, dass die Grundlagen- und Applikationsforschung für Signalverarbeitungsalgorithmen schnelle Fortschritte macht. Die Notwendigkeit oder der Patientenwunsch einer Betriebssoftwareanpassung beziehungsweise -veränderung wird daher voraussichtlich vor Ablauf der Lebensdauer der implantatinternen Energiequelle eintreten. Bevorzugte Ausführungsbeispiele des vorliegend beschriebenen Systems erlauben eine derartige Anpassung der Betriebsprogramme des Implantats auch im bereits implantierten Zustand.

Vorzugsweise ist ferner eine Zwischenspeicheranordnung vorgesehen, in welcher von der externen Einheit über die Telemetrieeinrichtung übermittelte Daten vor dem Weiterleiten an den Signalprozessor zwischengespeichert werden können. Auf diese Weise lässt sich der Übertragungsvorgang von der externen Einheit zu dem implantierten System abschließen, bevor die über die Telemetrieeinrichtung übermittelten Daten an den Signalprozessor weitergeleitet werden.

Des weiteren kann eine Überprüfungslogik vorgesehen sein, die in der Zwischenspeicheranordnung gespeicherte Daten vor dem Weiterleiten an den Signalprozessor einer Überprüfung unterzieht, um Fehlfunktionen vorzubeugen. Ferner kann ein Mikroprozessorbaustein, insbesondere ein Microcontroller, zum implantatinternen Steuern der A/D-Wandler und/oder der D/A-Wandler und/oder des Signalprozessors über einen Datenbus vorgesehen sein, wobei zweckmäßig die Überprüfungslogik und die Zwischenspeicheranordnung in dem Mikroprozessorbaustein implementiert sind und wobei über den Datenbus und die Telemetrieeinrichtung auch Programmteile oder ganze Softwaremodule zwischen der Außenwelt, dem Mikroprozessorbaustein und dem Signalprozessor übermittelt werden können.

Dem Mikroprozessorbaustein ist vorzugsweise eine implantierbare Speicheranordnung zum Speichern eines Arbeitsprogramms für den Mikroprozessorbaustein zugeordnet, und mindestens Teile des Arbeitsprogramms für den Mikroprozessorbaustein können durch von der externen Einheit über die Telemetrieeinrichtung übermittelte Daten geändert oder ausgetauscht werden.

In weiterer Ausgestaltung der Erfindung können mindestens zwei Speicherbereiche zur Aufnahme und Wiedergabe mindestens des Betriebsprogramms des Signalprozessors vorgesehen sein. Dies trägt zur Fehlersicherheit des Systems bei, indem durch das mehrfache Vorhandensein des Speicherbereichs, welcher das beziehungsweise die Betriebsprogramme enthält, beispielsweise nach einer Übertragung von extern oder aber beim Einschalten des Implantates eine Überprüfung der Fehlerfreiheit der Software durchgeführt werden kann

Analog hierzu kann auch die Zwischenspeicheranordnung mindestens zwei Speicherbereiche zur Aufnahme und Wiedergabe von von der externen Einheit über die Telemetrieeinrichtung übermittelten Daten aufweisen, so dass nach einer Datenübertragung von der externen Einheit noch im Bereich des Zwischenspeichers eine Überprüfung auf Fehlerfreiheit der übermittelten Daten vorgenommen werden kann. Die Speicherbereiche können zur beispielsweise komplementären Ablage der von der externen Einheit übermittelten Daten ausgelegt sein. Mindestens einer der Speicherbereiche der Zwischenspeicheranordnung kann aber auch zur Aufnahme nur eines Teils der von der externen Einheit übermittelten Daten ausgelegt sein, wobei in diesem Fall die Überprüfung der Fehlerfreiheit der übermittelten Daten abschnittsweise erfolgt.

Um zu gewährleisten, dass bei Übertragungsfehlern ein erneuter Übertragungsvorgang gestartet werden kann, kann dem Signalprozessor ferner ein vorprogrammierter, nicht überschreibbarer Festspeicherbereich zugeordnet sein, in welchem die für einen "Minimalbetrieb" des Systems erforderlichen Anweisungen und Parameter gespeichert sind, beispielsweise Anweisungen, die nach einem "Systemabsturz" zumindest einen fehlerfreien Betrieb der Telemetrieeinrichtung zum Empfang eines Betriebsprogramms sowie Anweisungen zum Einspeichern desselben in die Steuerlogik gewährleisten.

Wie bereits erwähnt, ist die Telemetrieeinrichtung in vorteilhafter Weise außer zum Empfang von Betriebsprogrammen von der externen Einheit auch zur Übermittlung von Betriebsparametern zwischen dem implantierbaren Teil des Systems und der externen Einheit ausgelegt, so dass einerseits solche Parameter von einem Arzt, einem Hörgeräteakustiker oder dem Träger des Systems selbst eingestellt werden können (z.B. Lautstärke), andererseits das System aber auch Parameter an die externe Einheit übermitteln kann, beispielsweise um den Status des Systems zu überprüfen.

Ein vollständig implantierbares Hörsystem der vorliegend erläuterten Art kann implantatseitig neben der aktorischen Stimulationsanordnung und der Signalverarbeitungseinheit mindestens einen implantierbaren Schallsensor und zur Energieversorgung ein nachladbares elektrisches Speicherelement aufweisen, wobei in einem solchen Fall eine drahtlose, transkutane Ladevorrichtung zum Laden des Speicherelements vorgesehen sein kann. Es versteht sich jedoch, dass zur Energieversorgung auch eine Primärzelle oder eine andere Energieversorgungseinheit vorhanden sein kann, die keine transkutane Nachladung benötigt. Dies gilt insbesondere, wenn man berücksichtigt, dass in naher Zukunft vor allem durch Weiterentwicklung der Prozessortechnologie mit wesentlicher Verminderung des Energiebedarfs für elektronische Signalverarbeitung zu rechnen ist, so dass für implantierbare Hörsysteme neue Energieversorgungsformen praktisch anwendbar werden, zum Beispiel eine den Seebeck-Effekt nutzende Energieversorgung, wie sie in DE-C-198 27 898 beschrieben ist. Vorzugsweise ist auch eine drahtlose Fernbedienung zur Steuerung der Implantatfunktionen durch den Implantatträger vorhanden.

Bei teilimplantierbarer Ausbildung des Hörsystems sind zweckmäßig mindestens ein Schallsensor, die elektronische Anordnung zur Audiosignalverarbeitung und -verstärkung, die Energieversorgungseinheit sowie eine Modulator/Sender-Einheit in einem extern am Körper, vorzugsweise am Kopf über dem Implantat, zu tragenden externen Modul enthalten. Das Implantat weist die intracochleäre aktorische Stimulatoranordnung auf, ist aber energetisch passiv und empfängt seine Betriebsenergie sowie Steuerdaten für die intracochleäre aktorische Stimulatoranordnung über die Einheit im externen Modul.

In weiterer Ausgestaltung der Erfindung können als ausgangsseitige Aktoren elektromechanische Wandler zur Anregung der flüssigkeitsgefüllten Innenohrräume des geschädigten Innenohrs vorgesehen sein, und die Signalverarbeitungseinheit kann eine treibende Signalverarbeitungselektronik aufweisen, die jeden der Wandler derart elektrisch ansteuert, dass auf der Basilarmembran des geschädigten Innenohres eine Wanderwellenkonfiguration entsteht, die die Art der Wanderwellenausbildung eines gesunden, nicht geschädigten Innenohres approximiert.

Vorzugsweise sind die ausgangsseitigen elektromechanischen Wandler für eine direkte Anregung der flüssigkeitsgefüllten Innenohrräume des geschädigten Innenohrs ausgelegt. Durch eine solche direkte Stimulation der Cochlea wird das Auftreten von Rückkopplungen, das heißt die Einkopplung des Ausgangssignals in den Sensor (Mikrofon), vermieden oder weitgehend reduziert, weil die Ossikelkette und damit das Trommelfell nicht beziehungsweise deutlich reduziert zu Schwingungen angeregt wird. Dies ist insbesondere dann von Vorteil, wenn ein Schallsensor (Mikrofonfunktion) in unmittelbarer Nähe zum Trommelfell appliziert wird, wie dies aus US-A-5 814 095 und US-A 5 999 632 bekannt ist.

Eine direkte Anregung der flüssigkeitsgefüllten Innenohrräume des geschädigten Innenohrs kann insbesondere durch ein intracochleäres Array von ausgangsseitigen Aktoren in Form von elektromechanischen Wandlern erreicht werden. Ein derartiges Wandler-Array wird unmittelbar in einen flüssigkeitsgefüllten Raum des Innenohres (scala tympani oder scala vestibuli) implantiert.

Das intracochleäre Wandler-Array hat vorzugsweise einen Gesamtdurchmesser im Bereich von 0,4 mm (apikaler Bereich) bis 2,0 mm (basaler Bereich) und eine Gesamtlänge zwischen 5 mm und 50 mm. Es weist zweckmäßig einen Träger aus einem biokompatiblen und im Innenohr biostabilen Material, vorzugsweise einem Polymer, insbesondere einem Silikon, auf. Die einzelnen ausgangsseitigen elektromechanischen Wandler können dabei aus Biokompatibilitätsgründen so in den Träger eingebettet sein, dass sie von einer dünnen Schicht des Trägermaterials vollständig umgeben sind.

Um eine mechanische Wellenausbreitung von einem Wandler innerhalb des Trägers zu benachbarten Wandlern zu minimieren, können zwischen den einzelnen ausgangsseitigen elektromechanischen Wandlern mechanische Dämpfungselemente in den Träger eingebettet sein, wobei das Material der Dämpfungselemente bei ähnlicher Querschnittsgeometrie wie die des Trägers vorzugsweise so gewählt ist, dass zur Erzielung hoher Dämpfungswerte ein großer mechanischer Impedanzunterschied zu dem Trägermaterial besteht.

Entsprechend einer abgewandelten Ausführungsform, kann aber auch ein extracochleäres mehrkanaliges Array von ausgangsseitigen Aktoren in Gestalt von elektromechanischen Wandlern vorgesehen sein, das von außen auf der Cochlea fixiert wird. Ein solches extracochleäres Wandler-Array kann als Ganzes einfach und mit hoher Genauigkeit in aus der Halbleiterfertigung üblichen Verfahren mikrosystemtechnisch, zum Beispiel fotolithographisch, entwickelt und hergestellt werden. Solche Verfahren gestatten eine hohe Miniaturisierung und eine exzellente Reproduzierbarkeit der einzelnen Wandler auf einem Array. Die Eigenschaften der mikrosystemtechnischen Erzeugung sind vorliegend besonders vorteilhaft, weil es bei der beabsichtigten Funktion des Arrays sehr auf einen Phasengleichlauf der einzelnen Wandler auf dem Array ankommt. Einzelheiten von mikrosystemtechnischen Verfahren sind unter anderem in WO-A-99/03146 beschrieben und bedürfen vorliegend keiner näheren Erläuterung.

Für das extracochleäre Wandler-Array kann vorteilhaft ein Substrat vorgesehen sein, das ein elektrisches Anschlussfeld enthält, das mikrosystemtechnisch mitgefertigt und für den Anschluss einer mehrpoligen, biokompatiblen Implantatleitung zu einem Modul ausgelegt ist, das die treibende Signalverarbeitungselektronik enthält. Das Substrat des extracochleären Wandler-Arrays kann ferner mit einem mikrosystemtechnisch mitgefertigten Elektronikmodul versehen sein, das insbesondere Treiberstufen zum Ansteuern der ausgangsseitigen elektromechanischen Wandler und/oder eine Decodierlogik und Umsetzerbausteine für den Anschluss einer polreduzierten Implantatleitung enthalten kann. So kann der Array-Anschluss aus nur drei Leitungen bestehen, und zwar insbesondere einer Masseleitung, einer Datenleitung und einer Taktsignalleitung, wobei die Versorgung mit elektrischer Betriebsenergie durch Phantomspeisung auf der Taktsignalleitung erfolgen kann.

Das Elektronikmodul kann des weiteren einen Schnittstellenbaustein für eine digitale Datenübertragung über die Implantatleitung, vorzugsweise mittels einer Lichtleitfaser, und/oder bei serieller Datenübertragung auf der Implantatzuleitung den Wandlern zugeordnete D/A-Wandler und Treiberbausteine enthalten. Zweckmäßig ist das extracochleäre Wandler-Array einschließlich Trägeraufbau (Substrat) mit einer biokompatiblen Ummantelung ausgestattet, die vorzugsweise aus von der Implantattechnologie bekannten Polymeren, insbesondere Polytetrafluorethylen, Polyurethan, oder Silikonen, besteht.

Für eine direkte Anregung der flüssigkeitsgefüllten Innenohrräume kann bei Verwendung eines extracochleären Wandler-Arrays dadurch gesorgt werden, dass die Wandler jeweils ein ausgangsseitiges Koppelelement aufweisen, das so ausgebildet ist, dass es durch einen artifiziellen Zugang zum Innenohr (Eröffnungen beziehungsweise Bohrungen in der knöchernen Außenwand der Cochlea, sogenannte "Cochleostomien") hindurchragt.

Das Elektronikmodul kann des weiteren einen Schnittstellenbaustein für eine digitale Datenübertragung über die Implantatleitung, vorzugsweise mittels einer Lichtleitfaser, und/oder bei serieller Datenübertragung auf der Implantatzuleitung den Wandlern zugeordnete D/A-Wandler und Treiberbausteine enthalten.

Zweckmäßig ist das extracochleäre Wandler-Array einschließlich Trägeraufbau (Substrat) mit einer biokompatiblen Ummantelung ausgestattet, die vorzugsweise aus von der Implantattechnologie bekannten Polymeren, insbesondere Polytetrafluorethylen, Polyurethan, oder Silikonen, besteht.

Vorteilhaft sind die ausgangsseitigen elektromechanischen Wandler in dem betreffenden Wandler-Array äquidistant oder in logarithmischen Distanzen - entsprechend der tonotopen Frequenz-Orts-Zuordnung längs der Basilarmembran des Innenohres - verteilt angeordnet, wobei im Falle einer tonotopen Anordnung eine Wandleranzahl von 20 bis 24 gemäß den psychoakustischen Frequenzgruppen zu besonders günstigen Ergebnissen führen kann.

Entsprechend einer abgewandelten Ausführungsform der Erfindung weist die Aktoranordnung in Kombination einen elektromechanischen Wandler zur mechanischen Stimulation des Mittel- oder Innenohres und ein intracochleäres, elektrisch wirkendes Reizelektrodenarray mit mindestens einer Reizelektrode zur elektrischen Stimulation des Innenohres auf.

Als aktorische Stimulationsanordnung kann ferner vorteilhaft eine duale intracochleäre Anordnung vorgesehen sein, die in Kombination eine Stimulatoranordnung mit mindestens einem Stimulatorelement zur mindestens mittelbaren mechanischen Stimulation des Innenohres und eine elektrisch wirkende Reizelektrodenanordnung mit mindestens einer Cochlea-Implant-Elektrode zur elektrischen Stimulation des Innenohres aufweist.

Bei den seit kurzem klinisch eingesetzten, teil- und vollimplantierbaren Hörsystemen zur Rehabilitation eines Innenohrschadens, bei denen ein an die Ossikelkette angekoppelter elektromechanischer Aktor vorgesehen ist, zeigt sich je nach verwendetem physikalischem Prinzip des ausgangsseitigen elektromechanischen Wandlers und insbesondere dessen Ankopplungsart an die Ossikel des Mittelohres, dass die erreichten Ergebnisse der Verbesserung des Sprachverständnisses sehr unterschiedlich sein können. Dazu kommt, dass bei manchen Patienten kein ausreichender Lautstärkepegel erreicht werden kann. Dieser Aspekt ist spektral sehr unterschiedlich, was bedeuten kann, dass bei zum Beispiel mittleren und hohen Frequenzen die erzeugte Lautheit zwar ausreichend ist, jedoch nicht bei tiefen Frequenzen beziehungsweise umgekehrt. Weiterhin kann die übertragbare spektrale Bandbreite begrenzt sein, so zum Beispiel bei elektromagnetischen Wandlern auf tiefe und mittlere Frequenzen oder bei piezoelektrischen Wandlern auf mittlere und hohe Frequenzen. Darüber hinaus können sich nichtlineare Verzerrungen, die insbesondere bei elektromagnetischen Wandlern ausgeprägt sind, negativ auf die resultierende Klangqualität auswirken. Die mangelnde Lautheit führt insbesondere dazu, dass der audiologische Indikationsbereich für die Implantation eines elektromechanischen Hörsystems sehr eingeschränkt sein kann. Das bedeutet, dass Patienten beispielsweise mit einem sensorineuralen Hörverlust von größer 50 dB HL (= hearing loss, Hörverlust) im Tieftonbereich mit einem piezoelektrischen System nur unzureichend versorgt werden können. Demgegenüber sind ausgeprägte Hochtonverluste mit elektromagnetischen Wandlern nur schwer versorgbar. Bei an Taubheit grenzender Schwerhörigkeit sind aus den genannten Gründen implantierbare, elektromechanische Systeme bislang nicht einsetzbar. Hier kommen Cochlea-Implantate mit rein elektrischer Reizung des Innenohres in Frage, die aber naturgemäß keine Klangqualität erwarten lassen, die zum Beispiel eine akzeptable Musikübertragung ermöglicht, sondern vorrangig zur Erlangung beziehungsweise Wiederherstellung eines ausreichenden Sprachverständnisses möglichst ohne Lippenablesen konzipiert sind. Diese Nachteile der heute verfügbaren implantierbaren, elektromechanischen Hörsysteme einerseits und der Cochlea-Implantate andererseits werden bei den zuletzt genannten abgewandelten Ausführungsformen der Erfindung zumindest teilweise umgangen, weil beide Stimulationsarten, d.h. mechanische und elektrische Reize, in einem einzigen Implantat-System zur Anwendung kommen und je nach individueller pathologischer und audiologischer Situation patientenspezifisch appliziert werden können. Durch eine kombinatorische Wirkung beider Reizformen und individuell maximierte Parametereinstellung des steuernden elektronischen Vorverarbeitungssystems können einerseits eine audiologisch möglichst weite Indikationsstellung und andererseits im individuellen Patienten ein möglichst optimales Ergebnis bezüglich Sprachdiskrimination, ausreichender Lautstärke in allen relevanten spektralen Bereichen und hoher Klangqualität erreicht werden.

Bei Verwendung der dualen intracochleären aktorischen Stimulationsanordnung kann die genannte Stimulatoranordnung vorteilhaft mindestens einen intracochleären elektromechanischen Wandler zur unmittelbaren mechanischen Stimulation des Innenohres und/oder mindestens eine intracochleäre Haarzellen-Reizelektrode zur mittelbaren mechanischen Stimulation des Innenohres durch elektrische Reizung von äußeren Haarzellen aufweisen. Die Cochlea-Implant-Elektroden können zugleich als Dämpfungselemente zwischen benachbarten elektromechanischen Wandlern genutzt werden.

Das beschriebene System kann monaural oder binaural ausgelegt sein. Ein binaurales System zur Rehabilitation einer Hörstörung beider Ohren weist zwei Systemeinheiten auf, die jeweils einem der beiden Ohren zugeordnet sind. Dabei können die beiden Systemeinheiten einander im wesentlichen gleich sein. Es kann aber auch die eine Systemeinheit als Master-Einheit und die andere Systemeinheit als von der Master-Einheit gesteuerte Slave-Einheit ausgelegt sein. Die Signalverarbeitungsmodule der beiden Systemeinheiten können auf beliebige Weise, insbesondere über eine drahtgebundene implantierbare Leitungsverbindung oder über eine drahtlose Verbindung, vorzugsweise eine bidirektionale Hochfrequenzstrecke, eine körperschallgekoppelte Ultraschallstrecke oder eine die elektrische Leitfähigkeit des Gewebes des Implantatträgers ausnutzende Datenübertragungsstrecke, so miteinander kommunizieren, dass in beiden Systemeinheiten eine optimierte binaurale Signalverarbeitung und Wandler-Array- Ansteuerung erreicht wird.

Bevorzugte Ausführungsbeispiele des erfindungsgemäßen Hörsystems beziehungsweise möglicher teil- und vollimplantierbarer Gesamtsysteme sind nachstehend unter Bezugnahme auf die beiliegenden Zeichnungen näher beschrieben. Es zeigen:
- Fig. 1: ein Blockschaltbild eines ausgangsseitig elektroakustischen Hörgerätes mit einem digitalen Signalprozessor zur Signalverarbeitung und mit drahtgebundenem Programmiersystem,
- Fig. 2: ein vollimplantierbares System mit einem elektromechanischen Wandler zur Mittelohranregung sowie mit Fernbedienung und Ladegerät,
- Fig. 3: ein vollimplantierbares System mit mehreren intracochleären, elektromechanischen Wandlern sowie mit Fernbedienung und Ladegerät,
- Fig. 4: ein Blockschaltbild eines teil- oder vollimplantierbaren Systems mit mehreren elektromechanischen Ausgangswandlern,
- Fig. 5: ein vollimplantierbares System mit einem elektromechanischen Wandler zur Mittelohranregung und mehreren, intracochleären elektrischen Reizelektroden sowie mit Fernbedienung und Ladegerät,
- Fig. 6: ein Blockschaltbild eines teil- oder vollimplantierbaren Systems mit einem elektromechanischen Wandler und mehreren elektrischen Reizelektroden,
- Fig. 7: ein vollimplantierbares System mit mehreren intracochleären, elektromechanischen Wandlern und mehreren intracochleären elektrischen Reizelektroden sowie mit Fernbedienung und Ladegerät,
- Fig. 8: ein Blockschaltbild eines teil- oder vollimplantierbaren Systems mit mehreren elektromechanischen Wandlern und mehreren elektrischen Reizelektroden,
- Fig. 9: ein teilimplantierbares System mit einem elektromechanischen Wandler zur
- Fig. 10: Mittelohranregung, ein teilimplantierbares System mit mehreren intracochleären, elektromechanischen Wandlern,
- Fig. 11: ein teilimplantierbares System mit einem elektromechanischen Wandler und mehreren intracochleären, elektrischen Reizelektroden,
- Fign. 12 bis 15: jeweils ein vollimplantierbares, binaurales System mit Leitungsverbindung zwischen beiden Elektronikmodulen und mit unterschiedlichen Aktoranordnungen,
- Fign. 16 bis 19: jeweils ein vollimplantierbares, binaurales System mit drahtloser Verbindung zwischen beiden Elektronikmodulen und mit unterschiedlichen Aktoranordnungen,
- Fign. 20 bis 23: jeweils ein vollimplantierbares, binaurales System mit Ultraschallkopplung zwischen beiden Elektronikmodulen und mit unterschiedlichen Aktoranordnungen,
- Fign. 24 bis 27: jeweils ein vollimplantierbares, binaurales System mit Kopplung beider Elektronikmodule mittels moduliertem Gewebestrom und mit unterschiedlichen Aktoranordnungen, sowie
- Fig. 28: schematisch ein Ausführungsbeispiel für den Aufbau eines extracochleären elektromechanischen Wandler-Arrays.

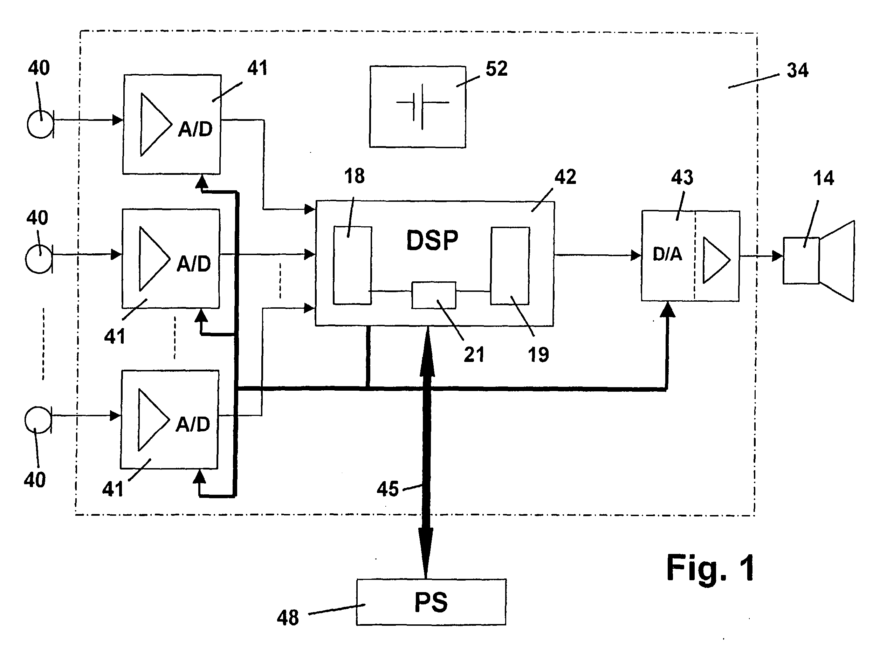

In Fig. 1 ist ein Blockschaltbild eines nichtimplantierbaren Hörgerätes veranschaulicht, bei dem zur Aufnahme des externen Schallsignals ein oder mehrere Schallsensoren (Mikrofone) 40 vorgesehen sind, welche das Schallsignal in ein analoges elektrisches Signal umwandeln. Jedem Schallsensor 40 ist ein Modul 41 nachgeschaltet, in dem das elektrische Ausgangssignal des betreffenden Schallsensors 40 vorverarbeitet, insbesondere vorverstärkt, und in ein digitales Signal umgewandelt (A/D) wird. Die Vorverstärkungs- und A/D-Wandler - Module 41 sind Teil einer insgesamt mit 34 bezeichneten elektronischen Signalverarbeitungseinheit. Zu der Signalverarbeitungseinheit 34 gehört ein digitaler Signalprozessor 42 (DSP), der die digitalisierten Sensorsignale weiterverarbeitet und der ein Digital/Analog-Wandler (D/A)-Endverstärkungs-Modul 43 ansteuert. In dem Modul 43 wird das digitale Ausgangssignal des Signalprozessors 42 in ein Analogsignal umgewandelt und verstärkt sowie dann einem in konventioneller Weise als elektroakustischer Wandler ausgebildeten Aktor 14 zugeführt. Der elektroakustische Wandler kann zum Beispiel vorteilhaft in der in DE-A-198 58 399.0 erläuterten Weise zur Schallabstrahlung in den äußeren Gehörgang aufgebaut sein und eine elektromechanische Wandler-Antriebseinheit aufweisen, die in einem allseitig hermetisch gasdichten Gehäuse untergebracht und mit einer Wandung diese Gehäuses derart gekoppelt ist, dass ausgangsseitige mechanische Schwingungen der Wandler-Antriebseinheit von innen an diese als biegefähige Membran ausgeführte Wandung mechanisch direkt angekoppelt werden und die Wandung zu Biegeschwingungen anregen, welche die Luftschallabstrahlung außerhalb des Wandlergehäuses bewirken. Dem Signalprozessor 42 ist ein Bedien- oder Programmiersystem 48 drahtgebunden zugeordnet. Zwischen dem Signalprozessor 42 und dem Bedien- oder Programmiersystem 48 können Daten über einen Datenbus 45 bidirektional übermittelt werden. Eine bei 52 angedeutete Energieversorgungseinheit versorgt die einzelnen Komponenten des Systems mit Strom.

Zu dem Signalprozessor 42 gehören erfindungsgemäß ein Sprachanalyse- beziehungsweise Spracherkennungsmodul 18 und ein Sprachsynthesemodul 19, die vorzugsweise beide in dem Signalprozessor 42 softwaremäßig realisiert und lernfähig und/oder umprogrammierbar aufgebaut sind. Das Sprachanalysemodul 18 nimmt eine echte Sprachinformationsanalyse (Sprachsegmentierung oder Spracherkennung) vor. Anhand der dadurch gewonnen Informationen wird in dem Sprachsynthesemodul 19 eine Sprachausgabe erzeugt, die auf einer echten Synthese beruht. Über den als elektroakustischer Wandler ausgebildeten Aktor 14 wird auf diese Weise ein rein artifiziell erzeugtes, synthetisiertes Sprachsignal ausgegeben, das praktisch ohne irgendwelche eingangsseitige Störsignalanteile ist. Es erfolgt mindestens näherungsweise eine vollständige Störsignalbefreiung, was unter anderem zu einer wesentlichen Verbesserung der Übertragung von Sprachinformation in störlärmerfüllter Umgebung führt. Verfahren und Vorrichtungen zur Sprachanalyse, -erkennung und -synthese, wie sie vorliegend grundsätzlich angewendet werden können, sind aus den dazu in der Beschreibungseinleitung genannten Literaturstellen bekannt, und sie bedürfen daher vorliegend keiner näheren Erläuterung.

Vorzugsweise wird in dem Sprachanalysemodul 18 das von den Vorverstärkungs- und A/D-Wandler-Modulen 41 kommende Eingangssignal durch automatische Algorithmen auf der Basis digital realisierter neuronaler Netzwerke analysiert und phonetischen oder lexikalischen Kategorien zugeordnet. Diese phonetischen oder lexikalischen Informationen stellen die Eingangssignale für das nachgeordnete Sprachsynthesemodul 19 dar. Das daraus von dem Sprachsynthesemodul 19 erzeugte Ausgangssignal geht nach D/A-Wandlung und zweckentsprechender Endverstärkung im Modul 43 an den Aktor 14, und es wird von diesem anschließend näherungsweise rauschfrei auf akustische Weise dem geschädigten Gehör übermittelt. Zur Vermeidung eines ausgeprägt monotonen Eindrucks der resynthetisierten Sprache ist es sehr sinnvoll, auf der Analyseseite Informationen über den emotionalen Zustand des Sprechers oder seine Intention bei zum Beispiel Fragestellungen oder Aufforderungen zu extrahieren und mit zu übertragen, das heißt, im vorliegenden Fall geeignet zu synthetisieren. Diese zusätzlichen Informationen werden allgemein als Prosodie der Sprache bezeichnet. Die Erkennung dieser prosodischen Merkmale kann zum Beispiel in der Extraktion der Höhe und des Verlaufs der Sprachgrundfrequenz bei stimmhaften Lauten wie Vokalen bestehen. Auf der Syntheseseite des Systems wird das Ausgangssignal entsprechend moduliert, um diese Information zu übermitteln.

Weiterhin kann es sinnvoll sein, ein Modul 21 vorzusehen, das es erlaubt, das Analyse- und Sysnthesesystem 18, 19 völlig auszuschalten und das Sprachsignal beziehungsweise das Audiosignal wie zum Beispiel Musik auf von herkömmlichen Hörgeräten bekannte Weise zu übertragen, wenn kein oder nur sehr wenig Störschall vorliegt. Das Modul 21 kann diese Funktion in Abhängigkeit von einer entsprechenden Störschallerfassung automatisch ausführen oder aber aufgrund einer Fernbedienungsfunktion durch den Anwender über das Bedien- oder Programmiersystem 48.

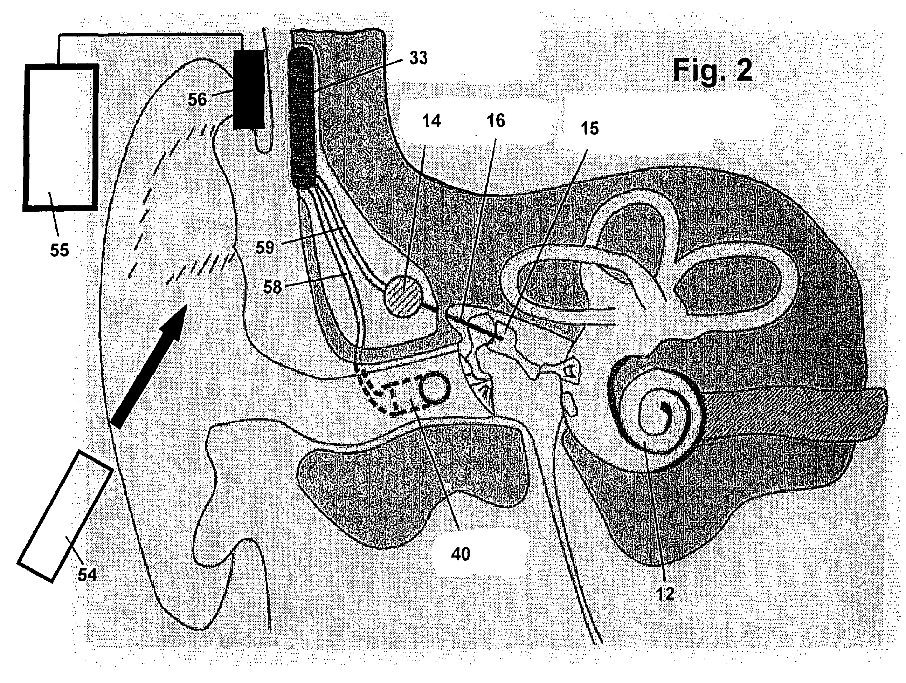

In Fig. 2 ist eine mögliche Ausführungsform eines vollimplantierbaren Hörsystems für Innenohrschwerhörige mit einen implantierbaren Schallsensor (Mikrofon) 40 und einem Aktor 14 in Form eines elektromechanischen Ausgangswandlers schematisch dargestellt. Dabei ist für eine mindestens bedarfsweise aktivierbare Sprachanalyse und -synthese entsprechend Fig. 1 gesorgt. Ferner kann bei diesem Hörsystem die Betriebssoftware transkutan verändert oder ausgetauscht werden.

Insbesondere weist das System ein in einem hermetisch dichten und biokompatiblen Implantatgehäuse untergebrachtes Elektronikmodul 33 auf, zu dem die anhand der Fig. 1 beschriebene Signalverarbeitungseinheit 34 einschließlich den Sprachanalyse- und Sprachsynthese-Modulen 18, 19 und vorzugsweise auch dem Schaltmodul 21 gehört. Weiterhin enthält das Implantatgehäuse als Energieversorgungseinheit 52 ein sekundäres, wiederaufladbares Element zur elektrischen Versorgung des Implantats und eine diesem Element zugeordnete Energieempfangsschaltung zur implantatseitigen Bereitstellung von Nachladeenergie. Die implantatseitige Energieempfangsschaltung wirkt mit einem Ladegerät 55 zum drahtlosen transkutanen Nachladen des im Implantat befindlichen wiederaufladbaren Elements 52 zusammen. Eine vorteilhafte Ausgestaltung einer implantierbaren Einheit mit einer Ladestromeinspeiseanordnung ist in EP-A-0 984 664 beschrieben Eine portable drahtlose Fernbedienung 54 dient der Steuerung der Implantatfunktionen durch den Implantatträger, wobei Daten zwischen der Fernbedienung 54 und dem Elektronikmodul 33 vorzugsweise auf induktivem Weg transferiert werden.

Das Mikrofon 40 kann vorteilhaft in der aus EP-A-0 831 673 bekannten Weise aufgebaut und mit einer Mikrofonkapsel, die in einem Gehäuse allseitig hermetisch dicht untergebracht ist, sowie mit einer elektrischen Durchführungsanordnung zum Durchführen mindestens eines elektrischen Anschlusses von dem Innenraum des Gehäuses zu dessen Außenseite versehen sein, wobei das Gehäuse mindestens zwei Schenkel aufweist, die in einem Winkel mit Bezug aufeinander ausgerichtet sind. Der eine Gehäuseschenkel nimmt dabei die Mikrofonkapsel auf und ist mit einer Schalleintrittsmembran versehen, während der andere Gehäuseschenkel die elektrische Durchführungsanordnung enthält und gegenüber der Ebene der Schalleintrittsmembran zurückversetzt ist. Dabei ist die Geometrie des Mikrofongehäuses so gewählt, dass bei Implantation des Mikrofons in der Mastoidhöhle der die Schalleintrittsmembran enthaltende Schenkel vom Mastoid aus in eine artifizielle Bohrung in der hinteren, knöchernen Gehörgangswand hineinragt und die Schalleintrittsmembran die Haut der Gehörgangswand berührt. Zur Festlegung des Mikrofons 40 kann zweckmäßig ein Fixationselement der aus US-A-5 999 632 bekannten Art vorgesehen sein, das eine Manschette aufweist, die mit einem zylindrischen Gehäuseteil den die Schalleintrittsmembran enthaltenden Schenkel umschließt und mit gegen die der Gehörgangshaut zugewendete Seite der Gehörgangswand anlegbaren, vorspringenden, elastischen Flanschteilen versehen ist. Dabei beinhaltet das Fixationselement vorzugsweise eine Halterung, welche die genannten Flanschteile vor der Implantation entgegen einer elastischen Rückstellkraft der Flanschteile in einer das Durchstecken durch die Bohrung der Gehörgangswand erlaubenden umgebogenen Stellung hält.

Zu dem Ladesystem gehört auch eine an den Ausgang des Ladegerätes 55 angeschlossene Ladespule 56, die vorzugsweise in der aus US-A-5 279 292 bekannten Art Teil eines Sende-Serienresonanzkreises bildet, der mit einem nicht veranschaulichten Empfangs-Serienresonanzkreis induktiv gekoppelt werden kann. Der Empfangs-Serienresonanzkreis kann bei der Ausführungsform gemäß Fig. 2 Teil des Elektronikmoduls 33 sein und entsprechend US-A-5 279 292 eine Konstantstromquelle für die Batterie 52 bilden. Dabei liegt der Empfangs-Serienresonanzkreis in einem Batterie-Ladestromkreis, der in Abhängigkeit von der jeweiligen Phase des in dem Ladestromkreis fließenden Ladestromes über den einen oder den anderen Zweig einer Vollweg-Gleichrichterbrücke geschlossen wird.

Das Elektronikmodul 33 ist bei der Anordnung nach Fig. 2 über eine Mikrofonleitung 58 an das Mikrofon 40 und über eine Aktor-Zuleitung 59 an den elektromechanischen Wandler 14 angeschlossen.

Der subkutan implantierte Schallsensor 40 (Mikrofon) nimmt den Schall auf und wandelt ihn in ein elektrisches Signal um, das über die Mikrofonleitung 58 der einen Teil des Elektronikmoduls 33 bildenden Signalverarbeitungseinheit 34 zugeführt wird. Das audiologisch bearbeitete und verstärkte Signal wird über die Aktorzuleitung 59 dem elektromechanischen Wandler (Aktor) 14 zugeleitet.

Der Wandler 14 kann insbesondere in der aus EP-A-0 499 940 bekannten Weise so aufgebaut sein, dass eine Gehäusewand eines hermetisch dichten Wandlergehäuses als schwingfähige Membran ausgeführt ist, die zusammen mit einer auf ihrer Innenseite aufgebrachten piezoelektrischen Keramikscheibe ein elektromechanisch aktives Heteromorph-Verbundelement darstellt und deren mechanische Schwingungen über eine auf der Außenseite der Membran fest angebrachte Koppelstange 16 und gegebenenfalls ein mit der Koppelstange verbundenes Koppelelement auf die Ossikelkette, im dargestellten Fall an den Amboss 15, übertragen werden. Die dort eingekoppelten Wandlerschwingungen gelangen über die Ossikelkette zum Innenohr und rufen dort den entsprechenden Höreindruck hervor.

Der Wandler 14 kann in der in DE-C-198 40 211 erläuterten Weise dahingehend modifiziert sein, dass an der Innenseite der piezoelektrischen Keramikscheibe ein Permanentmagnet angebracht ist, der nach Art eines elektromagnetischen Wandlers mit einer Elektromagnetspule zusammenwirkt. Ein solcher kombinierter piezoelektrischer elektromagnetischer Wandler ist besonders im Hinblick auf ein breites Frequenzband und die Erzielung relativ großer Schwingungsamplituden mit verhältnismäßig kleiner zugeführter Energie von Vorteil.

Bei dem Wandler 14 kann es sich auch um eine elektromagnetische Wandleranordnung handeln, wie sie in EP-A-0 984 663 beschrieben ist, das heißt eine mit einem am Implantationsort mit Bezug auf den Schädel fixierbaren Gehäuse und einem mit Bezug auf das Gehäuse beweglichen, mechanisch steifen Koppelelement versehene Wandleranordnung, bei der in dem Gehäuse ein elektromechanischer Wandler untergebracht ist, mit dem sich das Koppelelement in Schwingungen versetzen lässt, und der als Elektromagnetanordnung ausgebildet ist, die ein mit Bezug auf das Gehäuse fixiertes Bauteil sowie ein schwingfähiges Bauteil aufweist, welches mit dem Koppelelement derart in Verbindung steht, dass Schwingungen des schwingfähigen Bauteils auf das Koppelelement übertragen werden.

Zum Ankoppeln des elektromechanischen Wandlers 14 an das Mittel- oder Innenohr eignen sich besonders Koppelanordnungen gemäß US-A-5 941 814, bei denen ein Koppelelement außer einem Ankoppelteil für den betreffenden Ankoppelort eine Crimphülse aufweist, die zunächst lose auf einen mit rauher Oberfläche versehenen stabförmigen Teil der Koppelstange 16 aufgeschoben ist, die in der zuvor erläuterten Weise mit dem Wandler 14 verbunden ist. Beim Implantieren kann die Crimphülse gegenüber der Koppelstange 16 einfach verschoben und gedreht werden, um das Ankoppelteil des Koppelelementes mit dem beabsichtigten Ankoppelort exakt auszurichten. Dann wird die Crimphülse fixiert, indem sie mittels eines Crimpwerkzeuges plastisch kaltverformt wird. Alternativ kann das Koppelelement mit Bezug auf die Koppelstange 16 auch mittels einer zuziehbaren Bandschlaufe festgelegt werden.

Weitere vorliegend bevorzugt verwendbare Koppelanordnungen sind im einzelnen in DE-A-199 23 403, DE-A-199 35 029, DE-A-199 31 788, DE-A-199 48 336 und DE-A-199 48 375 beschrieben. So kann gemäß DE-A-199 23 403 das Koppelelement an seinem Ankoppelende eine Kontaktfläche aufweisen, die eine an die Oberflächenform der Ankoppelstelle anpassbare oder angepasste Oberflächenform sowie eine solche Oberflächenbeschaffenheit und Oberflächengröße aufweist, dass es durch Anlegen des Ankoppelendes an die Ankoppelstelle zu einer dynamischen Zug-Druck-Kraftkopplung von Koppelelement und Ossikelkette durch Oberflächenadhäsion kommt, die für eine sichere gegenseitige Verbindung von Koppelelement und Ossikelkette ausreicht. Das Koppelelement kann mit einem im implantierten Zustand an der Ankoppelstelle anliegenden Dämpfungsglied mit entropieelastischen Eigenschaften versehen sein, um eine optimale Schwingungsform der Steigbügelfußplatte oder einer das runde Fenster oder ein artifizielles Fenster in der Cochlea, im Vestibulum oder im Labyrinth abschließenden Membran zu erreichen und das Risiko einer Beschädigung der natürlichen Strukturen im Bereich der Ankoppelstelle während und nach der Implantation besonders gering zu halten (DE-A-199 35 029).

Das Koppelelement kann entsprechend DE-A-199 31 788 mit einer Stellvorrichtung zum wahlweisen Verstellen des Koppelelements zwischen einer Offenstellung, in welcher das Koppelelement in und außer Eingriff mit der Ankoppelstelle bringbar ist, und einer Schließstellung versehen sein, in welcher das Koppelelement im implantierten Zustand mit der Ankoppelstelle in Kraft- und/oder Formschlussverbindung steht.

Zum mechanischen Ankoppeln des als elektromechanischer Wandler ausgebildeten Aktors 14 an eine vorgewählte Ankoppelstelle an der Ossikelkette, der Steigbügelfußplatte oder einer das runde Fenster oder ein artifizielles Fenster in der Cochlea, im Vestibulum oder im Labyrinth (Gleichgewichtsorgan) abschließenden Membran eignet sich ferner eine Koppelanordnung (DE-A-199 48 336), die eine von dem Wandler in mechanische Schwingungen versetzbare Koppelstange 16 sowie ein mit der vorgewählten Ankoppelstelle in Verbindung bringbares Koppelelement aufweist, wobei die Koppelstange und das Koppelelement über wenigstens eine Kupplung miteinander verbunden sind und zumindest ein im implantierten Zustand an der Ankoppelstelle anliegender Abschnitt des Koppelelements zur verlustarmen Schwingungseinleitung in die Ankoppelstelle ausgelegt ist, wobei eine erste Kupplungshälfte der Kupplung eine Außenkontur mit mindestens näherungsweise der Gestalt einer Kugelkalotte aufweist, die in einer zur Außenkontur wenigstens teilweise komplementären Innenkontur einer zweiten Kupplungshälfte aufnehmbar ist, und wobei die Kupplung gegen Reibkräfte reversibel verschwenk- und/oder drehbar, jedoch bei im implantierten Zustand auftretenden dynamischen Kräften im Wesentlichen starr ist. Entsprechend einer abgewandelten Ausführungsform einer solchen Koppelanordnung (DE-A-199 48 375) hat eine erste Kupplungshälfte der Kupplung eine Außenkontur mit mindestens näherungsweise zylindrischer, vorzugsweise kreiszylindrischer, Gestalt, die in einer zur Außenkontur wenigstens teilweise komplementären Innenkontur einer zweiten Kupplungshälfte aufnehmbar ist, wobei ein im implantierten Zustand an der Ankoppelstelle anliegender Abschnitt des Koppelelements zur verlustarmen Schwingungseinleitung in die Ankoppelstelle ausgelegt ist, wobei im implantierten Zustand eine Übertragung von dynamischen Kräften zwischen den beiden Kupplungshälften der Kupplung im Wesentlichen in Richtung der Längsachse der ersten Kupplungshälfte erfolgt, und wobei die Kupplung reversibel an- und abkuppelbar sowie reversibel linear und/oder rotatorisch mit Bezug auf eine Längsachse der ersten Kupplungshälfte verstellbar, jedoch bei im implantierten Zustand auftretenden dynamischen Kräften starr ist.

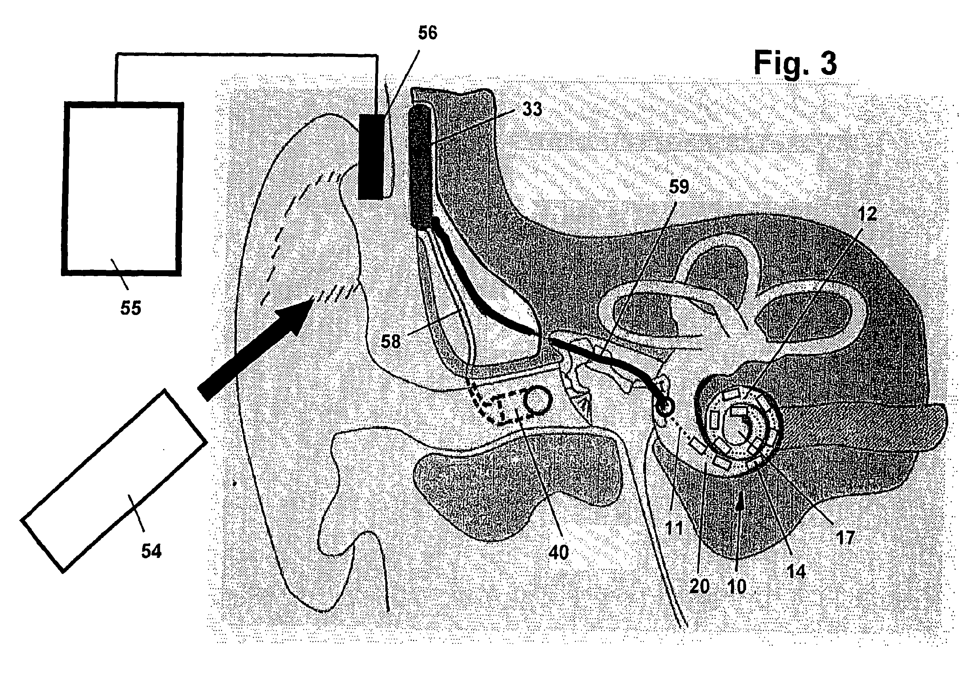

Fig. 3 zeigt schematisch den Aufbau eines vollständig implantierbaren Hörsystems ähnlich Fig. 2, bei dem jedoch Aktoren 14 vorgesehen sind, die ein intracochleäres elektromechanisches Wandler-Array 10 bilden. Das Wandler-Array 10 ist ähnlich einer mehrkanaligen, intracochleären Cochlea-Implantat-Elektrodenanordnung aufgebaut. Es weist einen mechanischen Träger 11 auf, der bevorzugt im wesentlichen aus einem flexiblen Formteil von vorzugsweise kreisförmigem Querschnitt besteht. Das Formteil wird durch eine artifizielle Eröffnung der Cochlea 12 oder das runde Fenster in das Innenohr geschoben. Statt einer Verteilung von elektrischen Reizelektroden einer Cochlea-Implantat-Elektrodenanordnung längs dieses Trägers 11 sind vorliegend als Aktoren 14 mehrere ausgangsseitige elektromechanische Wandler angeordnet, bei denen es sich beispielsweise um zylinderförmige Elemente mit kreisförmigem Querschnitt handeln kann. Innerhalb des Trägers 11 befinden sich elektrische Zuleitungen zu den Wandlern 14; diese Zuleitungen sind nicht dargestellt.

Die Aktoren 14 des Wandler-Arrays 10 arbeiten vorzugsweise nach dem Prinzip der dynamischen Volumenänderung aufgrund einer dynamischen Oberflächenvergrößerung beziehungsweise -verkleinerung entsprechend einem ansteuernden elektrischen Wandlerwechselspannungssignal. Die erforderlichen Volumenveränderungen für einen adäquaten, äquivalenten Schalldruckpegel von ca. 100 dB SPL ergeben sich zu etwa 2 10⁻⁴ Mikroliter (US-A-5 772 575).

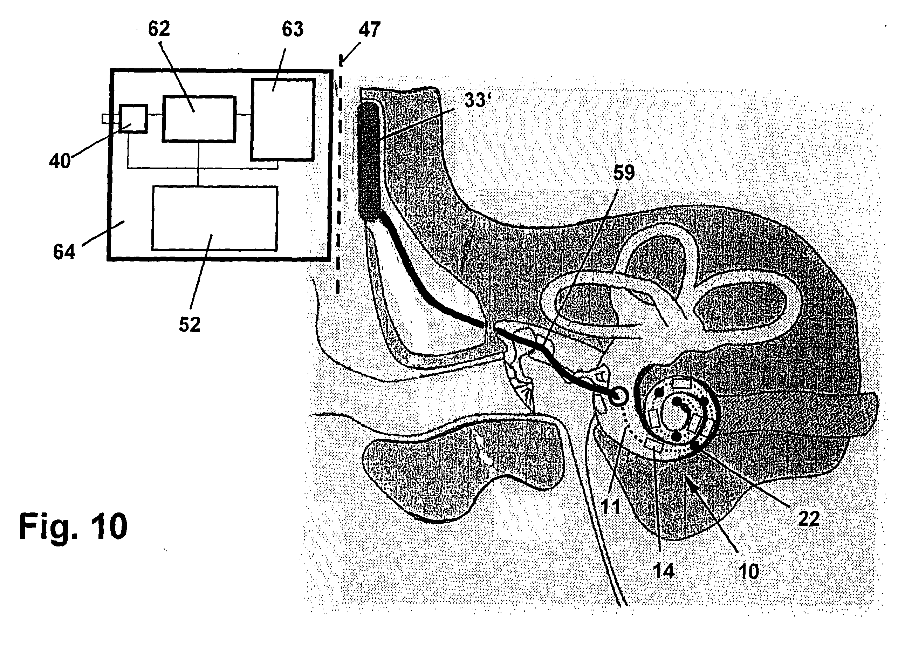

Die Aktoren 14 sind beispielsweise äquidistant längs des Trägers 11 verteilt oder in logarithmischen Distanzen entsprechend der tonotopen Orts-Frequenz-Zuordnung längs der Basilarmembran des Innenohres. Der Gesamtdurchmesser der Wandler-Array-Anordnung 10 liegt vorzugsweise im Bereich von 0,4 mm (apikaler Bereich 17 gemäß Fig. 3) bis 2,0 mm (basaler Bereich 20 gemäß Fig. 3). Die Gesamtlänge des Wandler-Arrays 10 liegt zweckmäßig zwischen 5 mm und 50 mm. Die Aktoren 14 sind aus Biokompatibilitätsgründen vorzugsweise so in den Träger 11 eingebettet, dass sie vollständig von einer dünnen Schicht des Trägermaterials umgeben sind. Der Träger 11 des Wandler-Arrays 10 besteht aus einem biokompatiblen und im Innenohr biostabilen Material, vorzugsweise Polymere wie entsprechende Silikone. Zwischen den einzelnen Aktoren 14 können in Fig. 10 bei 22 angedeutete mechanische Dämpfungselemente in den Träger 11 eingebettet sein, welche die mechanische Wellenausbreitung innerhalb des Trägers zu den benachbarten Wandlerelementen minimieren. Um hohe Dämpfungswerte zu erhalten, ist vorzugsweise das Material dieser Dämpfungselemente bei ähnlicher Querschnittsgeometrie wie die des Trägers 11 so gewählt, dass ein großer mechanischer Impedanzunterschied zu dem Trägermaterial besteht.

Die Aktoren 14 des Wandler-Arrays 10 können nach jedem bekannten elektromechanischen Wandlerprinzip arbeiten, nämlich elektromagnetisch, elektrodynamisch, piezoelektrisch, magnetostriktiv oder dielektrisch (kapazitiv). Die einzelnen elektromechanischen Wandler 14 werden vorzugsweise von der Signalverarbeitungseinheit 34 des Elektronikmoduls 33 so angesteuert, dass durch die jeweilige Wahl des spektralen Übertragungsbereiches je Wandler, der vibratorischen Amplitude und der Phasenlage der Wandler zueinander im aktorischen Gesamtergebnis der Innenohrstimulation eine Wanderwellenausbildung auf der Basilarmembran entsteht, die bei dem jeweiligen externen Schallereignis der Wanderwellenform möglichst nahekommt, die sich bei nicht geschädigtem cochleärem Verstärker und damit intakten äußeren Haarzellen ergeben würde.

Das die Signalverarbeitungseinheit 34 beinhaltende Elektronikmodul 33 ist bei der Anordnung nach Fig. 3 über die Mikrofonleitung 58 an das Mikrofon 40 und über die Aktorzuleitung 59 an das intracochleäre Wandler-Array 10 angeschlossen.

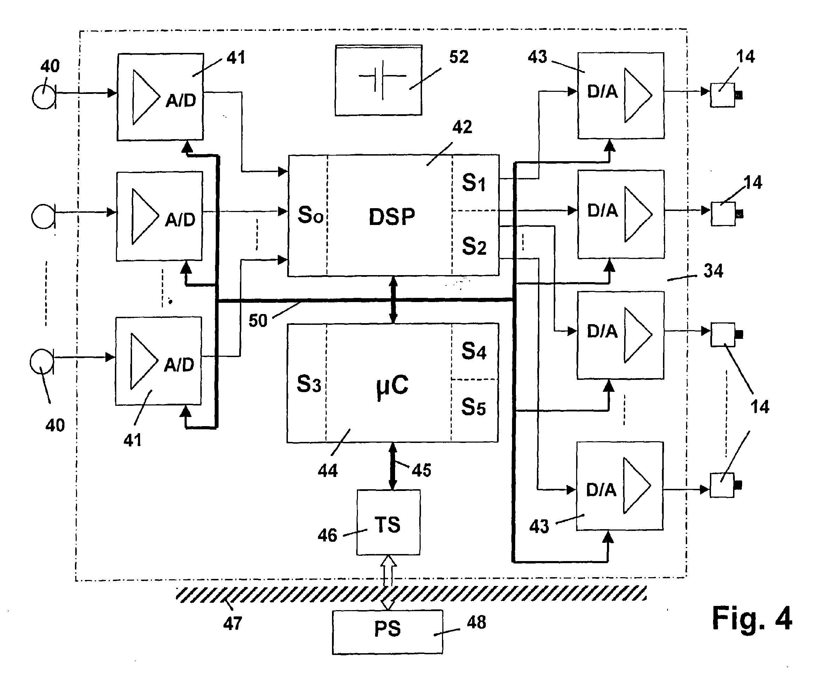

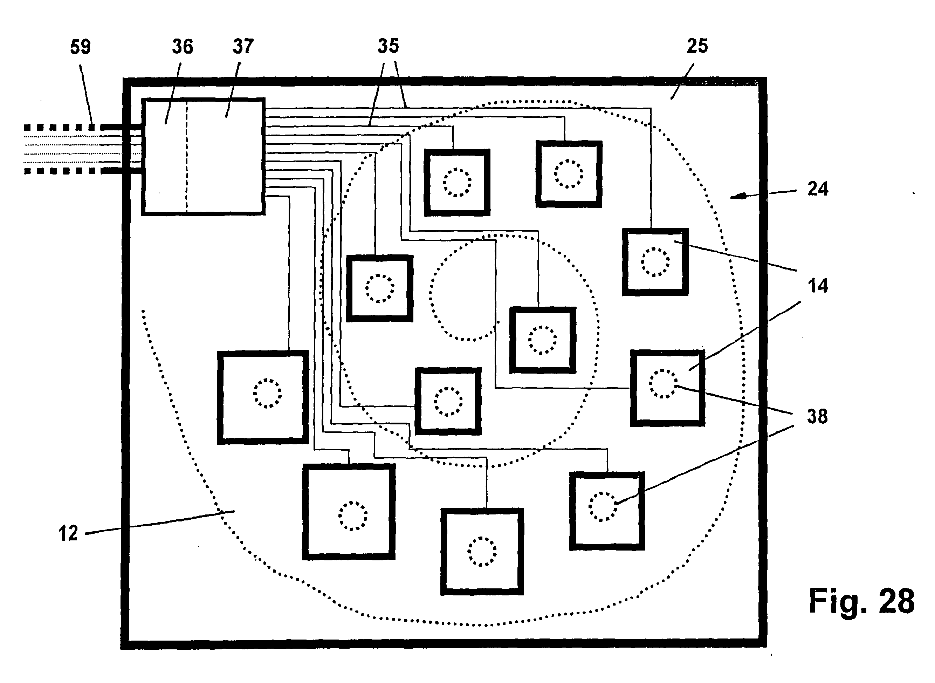

Anstelle des intracochleären Wandler-Arrays 10 kann auch ein extracochleäres Array von elektromechanischen Aktoren zur Anregung der flüssigkeitsgefüllten Innenohrräume vorgesehen sein, wie dies beispielshalber in Fig. 28 schematisch dargestellt ist. Bei einem solchen extracochleär zu applizierenden elektromechanischen Wandler-Array 24 sind mehrere miniaturisierte ausgangsseitige Aktoren 14 in Form von elektromechanischen Wandlern so über artifiziell in die die Cochlea 12 begrenzende knöcherne Wandung eingebrachte Öffnungen beziehungsweise Bohrungen (Cochleostomien) plaziert, dass wandlerausgangsseitig angebrachte Koppelelemente 28 durch die cochleären Eröffnungen in die lymphatischen Innenohrräume ragen. Mechanische Stimuli der Aktoren 14 werden intracochleär als Volumenverschiebungen erzeugt, die zu einem Höreindruck führen. Das Wandler-Array 24 kann bevorzugt mit mikrosystemtechnischen Methoden hergestellt werden. Auf einer Trägerplatte (Substrat) 25 sind dabei mehrere Wandlereinheiten 14 (zum Beispiel nach WO-A-99/3146) geometrisch so verteilt angeordnet, wie es dem statistischen Mittelwert der geometrischen Cochlea-Dimensionen entspricht (gepunktet unterlegter Umriss der Cochlea 12). Das Substrat 25 beinhaltet gleichzeitig elektrische Wandlerzuleitungen 35. Weiterhin ist ein elektrisches Anschlussfeld 36 vorgesehen, das mikrosystemtechnisch mitgefertigt wird und den adäquaten Anschluss einer mehrpoligen, biokompatiblen Aktorzuleitung 59 zu der Signalverarbeitungseinheit 34 (Fig. 4) ermöglicht. Darüber hinaus kann das Substrat 25 ein mikrosystemtechnisch mitgefertigtes Elektronikmodul 37 enthalten, das beispielhaft die Aktoren 14 ansteuernde Treiberstufen enthalten kann. Vorteilhaft kann dieses Modul 37 auch eine Decodierlogik und Umsetzerbausteine enthalten, die den Anschluss einer polreduzierten Implantatleitung ermöglichen. So kann beispielhaft der Arrayanschluss aus nur drei Leitungen bestehen (Masse, Daten und ein Taktsignal), wobei die notwendige Versorgung mit elektrischer Betriebsenergie durch Phantomspeisung auf der Taktsignalleitung erfolgen kann. Die wandlertreibenden Signale liegen dann in digital codierter Form vor, die mit hoher Taktrate seriell übertragen werden. Weiterhin kann ein Schnittstellenbaustein enthalten sein, der die digitale Datenübertragung über die Implantatleitung vorteilhaft mittels einer Lichtleitfaser ermöglicht. Bei serieller Datenübertragung sind in dem Elektronikmodul 37 entsprechende den Aktoren 24 zugeordnete D/A-Wandler und Treiberbausteine enthalten. Das gesamte Wandler-Array 24 einschließlich Trägeraufbau (Substrat) 25 ist mit einer biokompatiblen Ummantelung ausgestattet, die zum Beispiel aus bekannten Polymeren aus der Implantattechnologie besteht (Polytetrafluorethylen, Polyurethan, Silikone).

Weitere Einzelheiten eines Hörsystems zur Schaffung einer die Art der Wanderwellenausbildung eines gesunden Innenohres approximierenden Wanderwellenkonfiguration auf der Basilarmembran sind in der älteren EP-Patentanmeldung 00 119 195.6 erläutert und vorliegend ebenfalls vorteilhaft anwendbar.

Fig. 4 zeigt einen bevorzugten Aufbau der Signalverarbeitungseinheit 34 für das implantierbare Hörsystem gemäß Fig. 3. Auch in diesem Fall wird das externe Schallsignal über einen oder mehrere Schallsensoren (Mikrofone) 40 aufgenommen und in elektrische Signale umgewandelt. Die analogen elektrischen Sensorsignale werden an die Module 41 geleitet, wo sie vorverarbeitet, insbesondere vorverstärkt, und in digitale Signale umgewandelt (A/D) werden. Diese Vorverarbeitung kann beispielsweise in einer analogen linearen oder nichtlinearen Vorverstärkung und Filterung (zum Beispiel Antialiasing-Filterung) bestehen.

Die digitalisierten Sensorsignale werden in dem digitalen Signalprozessor 42 (DSP) weiterverarbeitet. Der Signalprozessor 42 enthält im vorliegenden Ausführungsbeispiel einen nicht überschreibbaren Festspeicherbereich So, in welchem die für einen "Minimalbetrieb" des Systems erforderlichen Anweisungen und Parameter gespeichert sind, sowie Speicherbereiche S₁ und S₂, in denen die Betriebssoftware der bestimmungsgemäßen Funktion beziehungsweise Funktionen des Implantatsystems abgelegt sind. Die wiederholt beschreibbaren Programmspeicher S₁ und S₂ zur Aufnahme der Betriebssoftware können auf EEPROM-Basis oder statischen RAM-Zellen basieren, wobei im letztgenannten Fall dafür gesorgt werden sollte, dass dieser RAM-Bereich immer durch das implantatinterne Energieversorgungssystem "gepuffert" ist.

Die digitalen Ausgangssignale des Signalprozessors 42 werden in den Digital-Endverstärker-Modulen 43 in Analogsignale umgewandelt und verstärkt sowie dann den ausgangsseitigen elektromechanischen Wandlern (Aktoren) 14 zugeführt. Die Module 43 können gegebenenfalls entfallen, wenn beispielsweise bei einem Hörsystem mit elektromagnetischem Ausgangswandler ein zum Beispiel pulsweitenmoduliertes, serielles digitales Ausgangssignal des Signalprozessors 42 direkt an die Aktoren 14 übermittelt wird.

Der Signalprozessor 42 führt die bestimmungsgemäße Funktion des Hörimplantates aus. Dazu gehören eine Audiosignalverarbeitung für die Rehabilitation einer Hörstörung, die anhand der Fig. 1 diskutierte Sprachanalyse und -synthese und gegebenenfalls auch eine Signalgenerierung im Fall eines Systems mit zusätzlicher Tinnitusmaskierer- oder Noiser-Funktion. Der Signalprozessor 42 beinhaltet wiederum die in Fig. 4 und den weiteren Figuren nicht mehr im einzelnen dargestellten Softwaremodule 18, 19 und vorzugsweise auch das Softwaremodul 21 gemäß Fig. 1. Weiterhin enthält der digitale Signalprozessor 42 Softwaremodule, die eine solche Ansteuerung der ausgangsseitigen elektromechanischen Wandler 14 bewirken, dass die spektralen, zeitlichen, amplituden- und phasenbezogenen Wandlersignaleigenschaften so bemessen sind, dass auf der Basilarmembran des geschädigten Innenohres eine Wanderwelle erzeugt wird, die der des gesunden Gehörs möglichst nahe kommt. Die genannten Softwaremodule können statisch und dynamisch ausgelegt sein. Unter statischer Auslegung soll dabei verstanden werden, dass die Softwaremodule aufgrund wissenschaftlicher Erkenntnisse einmalig im Programmspeicher des Signalprozessors 42 abgelegt werden und unverändert bleiben. Dynamisch bedeutet, dass die Softwaremodule "lernfähig" sind, um die Sprachanalyse und Spracherkennung beziehungsweise die angestrebte Wanderwellenkonfiguration zeitlich iterativ zu optimieren. Dies bedeutet, dass die Softwaremodule adaptiv ausgelegt sein können und die Parameteranpassung durch "Training" durch den Implantatträger und gegebenenfalls weitere Hilfsmittel, wie Rehabilitationsprogramme, vorgenommen wird. Die Sprachanalyse und die Spracherkennung können vorteilhaft auf einem digital realisierten neuronalen Netzwerk beruhen. Entsprechend kann auch ein Softwaremodul enthalten sein, welches eine möglichst optimale Simulation des "gesunden" cochleären Verstärkers auf der Basis eines lernfähigen neuronalen Netzwerkes approximiert. Das Training der neuronalen Netze kann wieder durch den Implantatträger und/oder unter Zuhilfenahme weiterer externer Hilfsmittel erfolgen.

Eine Methode zur möglichst optimalen Simulation des "gesunden" cochleären Verstärkers kann die Implementierung des Prinzips der "Time-Reversed Acoustics" (TRA) sein (Fink, M.: "Time-Reversed Acoustics", Scientific American 281:5 (1999), p. 67-73). Die Ansteuerung der ausgangsseitigen Aktoren 14 erfolgt mittels TRA so, dass örtlich begrenzte Bereiche der Cochlea mechanisch stimuliert werden. Während in herkömmlichen Anwendungen von TRA die Registrierung des verteilten Schallereignisses und die Aussendung des time-reversed Signals im selben Präparat erfolgen, werden diese beiden Schritte im vorliegenden Fall getrennt. Die verteilten Ereignisse können zum Beispiel in einem adäquaten Tiermodell intracochleär ermittelt werden; anschließend werden die time-reversed Stimuli bei der vorliegenden Applikation eines Hörsystems für den Menschen, gegebenenfalls unter Parameteranpassung an die veränderte Geometrie der menschlichen Cochlea, appliziert.

Damit die softwarebasierten Algorithmen für die Sprachanalyse und -erkennung sowie zur möglichst optimalen Simulation des cochleären Verstärkers insbesondere in einem Vollimplantat auch postoperativ implementiert werden können, enthält das System nach Fig. 4 einen weiteren Mikroprozessorbaustein, zum Beispiel einen Mikrocontroller (µC) 44 mit zugehörigen Speichern S₃, S₄ und S₅. Bei dem Speicher S₃ handelt es sich um einen wiederholt beschreibbaren Speicher, in welchem ein Arbeitsprogramm für den Mikrocontroller 44 abgelegt ist. In den Speicherbereichen S₄ und S₅ können insbesondere die Betriebsoftwareanteile des Implantatmanagementsystems abgelegt sein (zum Beispiel Verwaltungsüberwachungs- und Telemetriefunktionen). In den Speichern S₁, und/oder S₂ und/oder S₄ und/oder S₅ können auch von außen veränderliche, patientenspezifische, wie zum Beispiel audiologische, Anpassparameter, abgelegt sein.

Der Microcontroller 44 kommuniziert einerseits über den bidirektionalen Datenbus 45 und ein Telemetriesystem (TS) 46 drahtlos (beispielsweise mittels induktiver Kopplung) durch die bei 47 angedeutete geschlossene Haut mit dem externen Programmiersystem (PS) 48. Das Programmiersystem 48 kann ein PC-basiertes System mit entsprechender Programmier-, Bearbeitungs-, Darstellungs- und Verwaltungssoftware sein. Über diese Telemetrieschnittstelle wird die zu verändernde beziehungsweise ganz auszutauschende Betriebssoftware des Implantatsystems übertragen und zunächst in dem Speicherbereich S₄ und/oder S₅ des Microcontrollers 44 zwischengespeichert. So kann zum Beispiel eine einfache Verifikation der Softwareübertragung durch einen Lesevorgang über die Telemetrieschnittstelle durchgeführt werden, bevor die Betriebssoftware oder die entsprechenden Signalverarbeitungsanteile dieser Software in die Programmspeicherbereiche S₁, und S₂ des digitalen Signalprozessors 42 über einen Datenbus 50 übertragen werden.

Ferner kann auch das Arbeitsprogramm für den Microcontroller 44 über die Telemetrieschnittstelle ganz oder teilweise mit Hilfe der externen Einheit 48 geändert oder ausgetauscht werden.

Andererseits steuert der Microcontroller 44 implantatintern über den bidirektionalen Datenbus 50 die A/D-Konvertermodule 41 der Sensor-Vorverarbeitung, die D/A-Konvertermodule 43 zur Ansteuerung der ausgangsseitigen elektromechanischen Wandler 14 und den Signalprozessor 42 selbst. Über den Datenbus 50 werden auch Programmteile oder ganze Softwaremodule zwischen Außenwelt, Microcontroller 44 und Signalprozessor 42 übermittelt.

Das Implantatsystem enthält bei vollimplantierter Ausführungsform als Energieversorgungseinheit 52 eine primäre oder sekundäre Batteriezelle, die die einzelnen Module mit elektrischer Betriebsenergie versorgt.

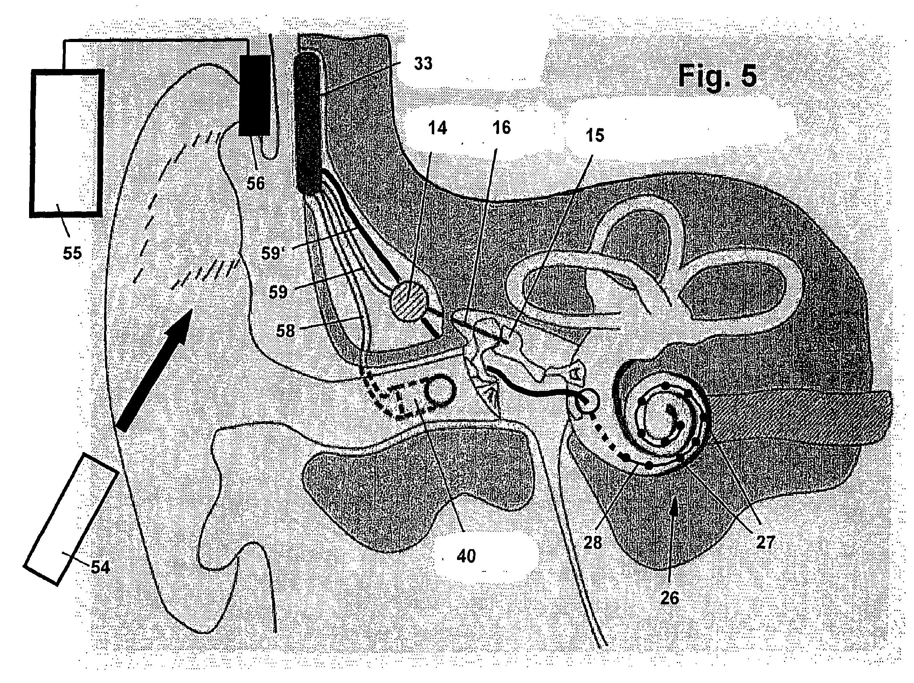

Fig. 5 zeigt schematisch den Aufbau eines vollständig implantierbaren Hörsystems, dessen ausgangsseitige aktorische Stimulationsanordnung ein elektrisches, intracochleäres Array 26 mit mehreren Reizelektroden 27 und einen elektromechanischen Wandler 14 aufweist, der hier beispielhaft über die Koppelstange 16 an den Amboss 15 angekoppelt ist.

Das intracochleäre Reizelektrodenarray 26 kann in beliebiger für Cochlea-Implantate bekannter Weise zum Beispiel als unipolare oder bipolare Anordnung aufgebaut sein. Es weist einen Elektrodenträger 28 aus elektrisch isolierendem, flexiblem Werkstoff auf, entlang dem die an Zuleitungen 59' angeschlossenen Reizelektroden 27 in gegenseitigem Abstand verteilt angeordnet sind. Die Reizelektroden 27 sind so in den Träger 28 eingebettet oder an dem Träger 28 fixiert, dass ein Oberflächenanteil je Reizelektrode in direktem galvanischem Kontakt zu der lymphatischen Flüssigkeit des Innenohres oder direkt zu einer der zu reizenden neuronalen Strukturen steht.

Der Aktor 14 kann in der oben unter Bezugnahme auf die Fig. 2 erläuterten Weise ausgelegt und an das Mittel- oder Innenohr angekoppelt sein. Auch bezüglich des Schallsensors 40, der drahtlosen Fernbedienung 54 und des Ladegerätes 55 wird auf die vorangegangene Beschreibung der Fig. 2 verwiesen.

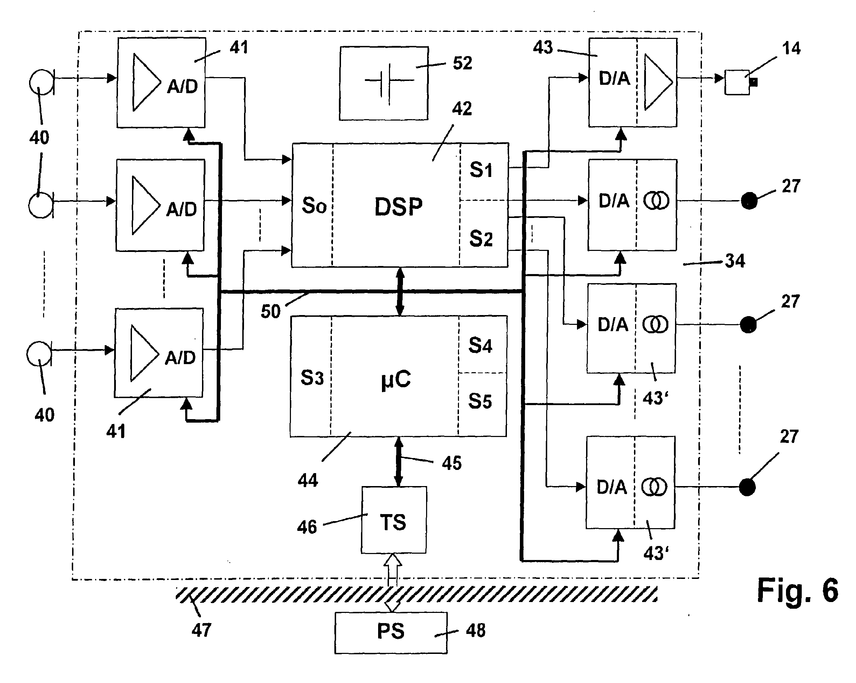

Fig. 6 zeigt einen bevorzugten Aufbau der Signalverarbeitungseinheit 34 für das implantierbare Hörsystem gemäß Fig. 5. Der digitale Signalprozessor 42 enthält Softwaremodule, die eine duale Ansteuerung des Reizelektrodenarrays 26 beziehungsweise der einzelnen Reizelektroden 27 sowie des elektromechanischen Wandlers 14 so vornehmen, dass die spektralen, zeitlichen, amplituden- und phasenbezogenen Wandler- beziehungsweise Reizelektroden-Signaleigenschaften derart konfiguriert sind, dass bei dem betreffenden Patienten ein optimaler Hörerfolg approximiert wird. Den Reizelektroden 27 vorgeschaltete D/A-Konvertermodule 43', die ebenso wie das dem elektromechanischen Wandler 14 zugeordnete D/A-Konvertermodul 43 mit digitalen Ausgangssignalen des Signalprozessors 42 beaufschlagt sind, umfassen, wie in Fig. 6 gezeigt, jeweils eine digital gesteuerte Stromquelle zur Strombeaufschlagung der betreffenden Reizelektrode 27. Im übrigen stimmen Aufbau und Funktion der Schaltungsanordnung nach Fig. 6 mit denjenigen des Ausführungsbeispiels gemäß Fig. 4 überein. Weitere Einzelheiten eines Hörsystems mit kombinierter Stimulation des Mittel- oder Innenohres über einen Aktor in Form eines elektromagnetischen Wandlers sowie elektrischer Stimulation des Innenohres mittels eines intracochleären, elektrisch wirkenden Reizelektrodenarrays sind in der älteren EP-Patentanmeldung 01 109 192.3 erläutert und vorliegend ebenfalls vorteilhaft anwendbar.

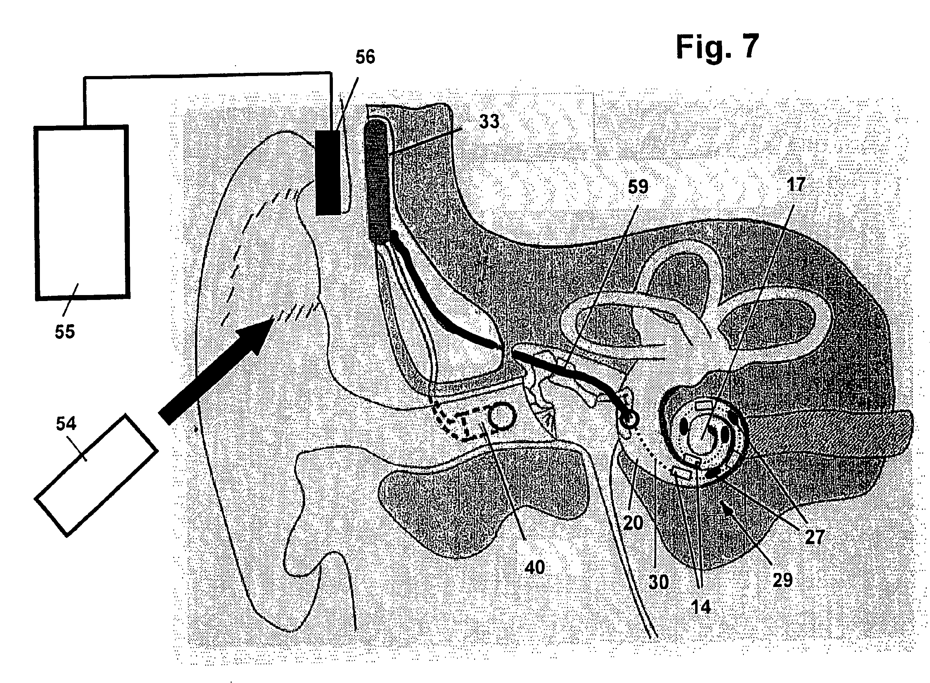

Fig. 7 zeigt schematisch ein implantierbares Hörsystem ähnlich denjenigen der Figuren 3 und 5, bei dem jedoch abweichend von dort als Aktoranordnung ein intracochleäres, duales Stimulationsarray 29 mit mehreren Cochlea-Implant-Reizelektroden 27 zur unmittelbaren elektrischen Stimulation des Innenohres und mit mehreren intracochleären Aktoren 14 in Form von elektromechanischen Wandlern zur unmittelbaren mechanischen Stimulation des Innenohres in einem gemeinsamen mechanischen Träger 30 vorgesehen ist. Prinzipiell ist dieses Array 29 ähnlich wie ein mehrkanaliges, intracochleäres Cochlea-Implantat-Elektrodenarray aufgebaut, und es ist über die Wandlerarray-Zuleitung 59 an das Elektronikmodul 33 angeschlossen. Der Träger 30 besteht bevorzugt im wesentlichen aus einem flexiblen Silikonformteil von vorzugsweise kreisförmigem Querschnitt. Das Formteil wird durch das ovale Fenster, das runde Fenster oder eine artifizielle Eröffnung der Cochlea 12 oder in die flüssigkeitsgefüllten Innenohrräume geschoben. Die elektromechanischen Wandler 14 sind in Fig. 7 schematisch als zylinderförmige Elemente mit ebenfalls kreisförmigem Querschnitt gezeigt. Innerhalb des Trägers 30 befinden sich elektrische Zuleitungen zu den Cochlea-Implant-Elektroden 27 und den Wandlern 14; diese einzelnen Zuleitungen sind nicht näher dargestellt.

Die elektromechanischen Wandler 14 des dualen Arrays 29 arbeiten wiederum vorzugsweise nach dem Prinzip der dynamischen Volumenänderung aufgrund einer dynamischen Oberflächenvergrößerung beziehungsweise -verkleinerung entsprechend einem ansteuernden elektrischen Wandlerwechselspannungssignal.

Die Wandler 14 sind beispielsweise äquidistant längs des Trägers 30 verteilt oder in logarithmischen Distanzen entsprechend der tonotopen Orts-Frequenz-Zuordnung längs der Basilarmembran des Innenohres. Der Gesamtdurchmesser der Wandler-Elektroden-Anordnung 29 liegt vorzugsweise im Bereich von 0,4 mm (apikaler Bereich 17 gemäß Fig. 7) bis 2,0 mm (basaler Bereich 20 gemäß Fig. 7). Die Gesamtlänge des Stimulationsarrays 29 liegt zweckmäßig zwischen 5 mm und 50 mm. Die elektromechanischen Wandlerelemente 14 sind aus Biokompatibilitätsgründen vorzugsweise so in den Träger 30 eingebettet, dass sie vollständig von einer dünnen Schicht des Trägermaterials umgeben sind. Der Träger 30 des Stimulationsarrays 29 besteht aus einem biokompatiblen und im Innenohr biostabilen Material, vorzugsweise Polymere wie entsprechende Silikone.

Die Cochlea-Implant-Elektroden 27 sind, wie bei Cochlea-Implantaten bekannt, so in den Träger 30 eingebettet beziehungsweise in oder an dem Träger 30 fixiert, dass ein Oberflächenanteil je Reizelektrode in direktem galvanischem Kontakt zu der lymphatischen Flüssigkeit des Innenohres oder direkt zu einer zu reizenden neuronalen Struktur steht. Die Cochlea-Implant-Elektroden 27 können aus allen bekannten biokompatiblen Metallen bestehen, insbesondere aus reinem Platin, vorzugsweise aus Platin-Iridium-Legierungen (vorzugsweise 90 % Pt, 10 % Ir), reinem Gold, Goldlegierungen, Tantal, Tantallegierungen, Niob, Nioblegierungen sowie Edelstählen.

Vorteilhaft sind die elektromechanischen Wandler 14 und die Cochlea-Implant- Elektroden 27 in Längsrichtung des Trägers 30 einander abwechselnd angeordnet, damit die mechanische Wellenausbreitung innerhalb des Trägers 30 zwischen benachbarten elektromechanischen Wandlerelementen 14 bedämpft wird und somit eine örtlich konzentrierte mechanische Stimulation je Wandler erreicht wird.

Weitere Einzelheiten von vorliegend geeigneten dualen intracochleären Stimulationsarrays 29 sind in der älteren EP-Patentanmeldung 01 109 191.5 erläutert. Im übrigen entspricht die Anordnung der Fig. 7 den anhand der Figuren 3 und 5 beschriebenen Ausführungsbeispielen.

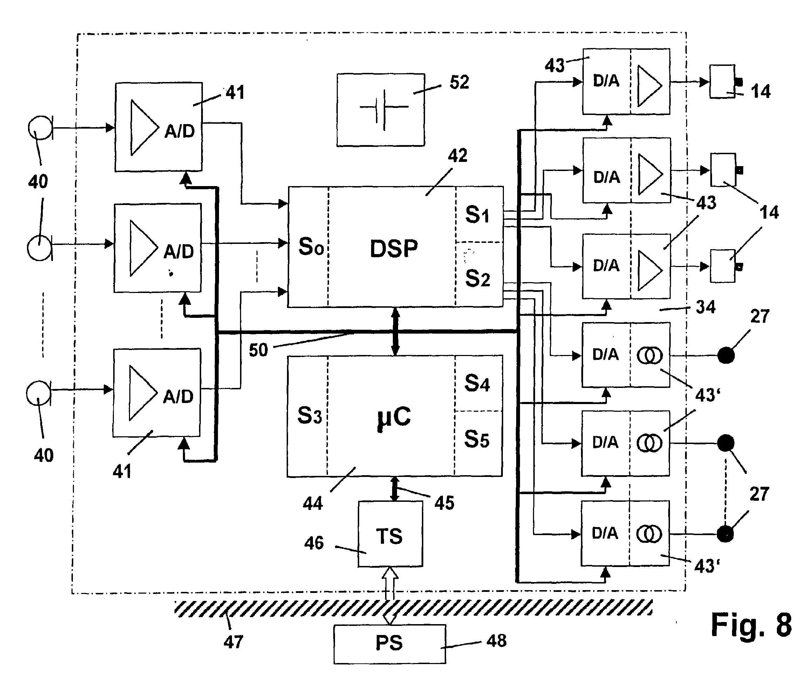

Eine für das Ausführungsbeispiel der Fig. 7 geeignete Auslegung der in dem Elektronikmodul 33 enthaltenen Signalverarbeitungseinheit 34 ist in Fig. 8 veranschaulicht. Wie dort gezeigt ist, werden die als elektromechanische Wandler ausgebildeten Aktoren 14 des dualen intracochleären Stimulationsarrays 29 jeweils von einem Digital/Anal og-Wandler-Endverstärkungs-Modul 43 angesteuert, während zur Strombeaufschlagung der Reizelektroden 27 des Arrays 29 jeweils ein Digital/Analog-Wandler-Modul 43' mit digital gesteuerter Stromquelle vorgesehen ist. Im übrigen stimmen Aufbau und Funktion der Schaltungsanordnung nach Fig. 8 mit denjenigen der Ausführungsformen gemäß den Figuren 4 und 6 überein.

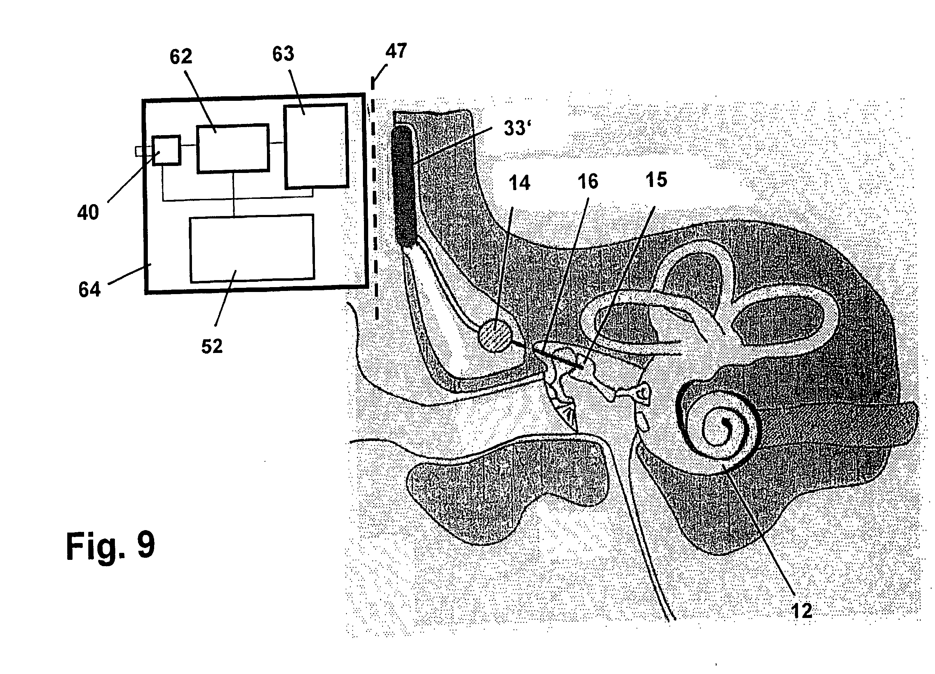

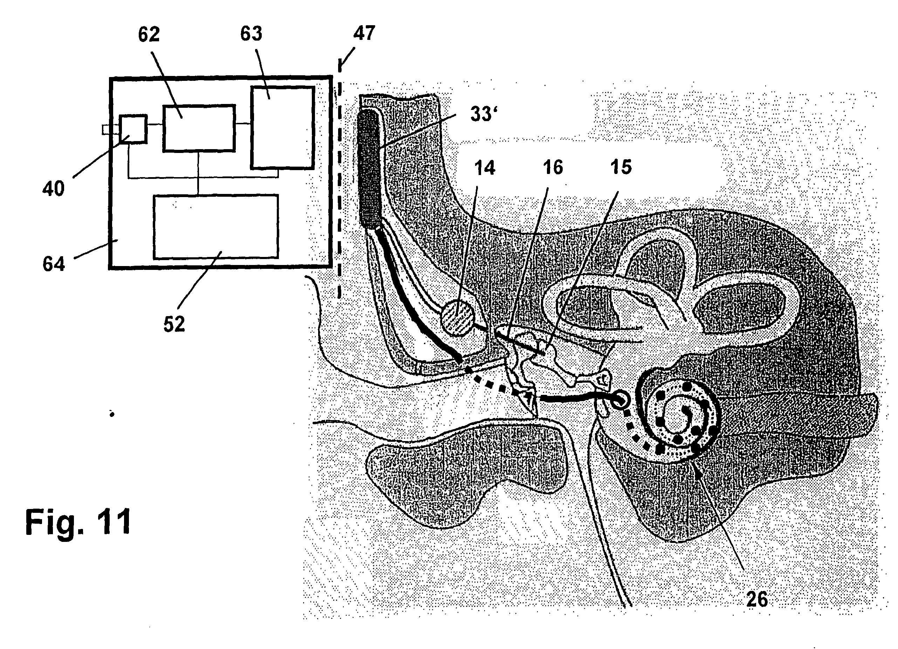

Die Figuren 9, 10 und 11 zeigen schematisch den Aufbau eines teilimplantierbaren Hörsystems mit einer Aktoranordnung gemäß Fig. 2 beziehungsweise Fig. 3 beziehungsweise Fig. 5. Bei diesen teilimplantierbaren Systemen sind mindestens ein Schallsensor (Mikrofon) 40, ein Elektronikmodul 62 für eine elektronische Signalverarbeitung weitestgehend entsprechend Fig. 1 beziehungsweise Fig. 4 beziehungsweise Fig. 6 (aber ohne das Telemetriesystem 46), die Energieversorgung 52 sowie eine Modulator/Sender-Einheit 63 in einem extern am Körper, vorzugsweise am Kopf über dem Implantat, zu tragenden externen Modul 64 enthalten. Das Implantat ist wie bei bekannten Teilimplantaten energetisch passiv. Sein Elektronikmodul 33' (ohne Energieversorgung 52) empfängt seine Betriebsenergie und Wandlersteuerdaten über die Modulator/Sender-Einheit 63 im externen Teil 64. Es versteht sich, dass bei einem solchen teilimplantierbaren Hörsystem auch das duale intracochleäre Stimulationsarray 29 gemäß Fig. 7 vorgesehen sein kann und dass auch eine binaurale Anwendung analog den nachstehend erläuterten Ausführungsbeispielen möglich ist.

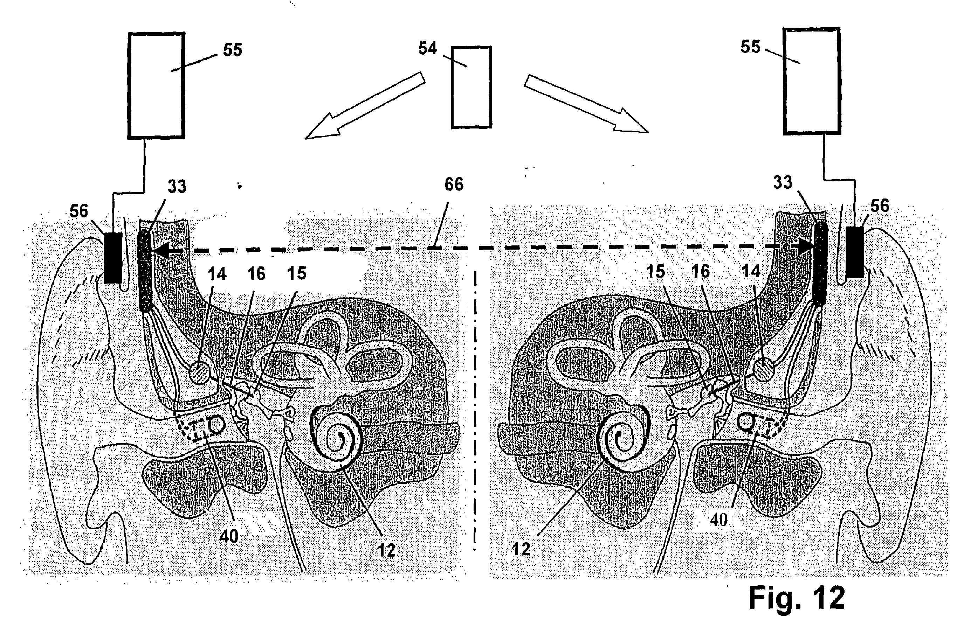

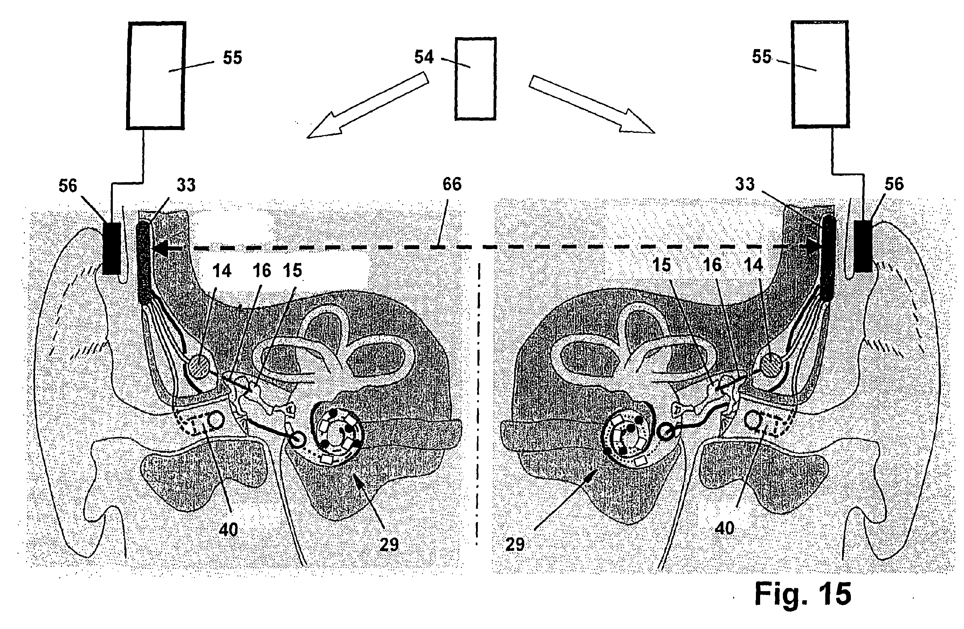

Die Figuren 12, 13, 14 und 15 zeigen jeweils eine binaurale Anwendung eines Hörimplantates, bei dem die Signalverarbeitungseinheiten 34 in den Elektronikmodulen 33 auf beiden Seiten des Schädels über eine drahtgebundene implantierbare Leitungsverbindung 66 so miteinander kommunizieren, dass eine optimale binaurale Signalverarbeitung und Aktor-Ansteuerung in beiden mit dem Implantat versehenen Innenohren erreicht wird. Weiterhin sind auch hier transkutane Ladevorrichtungen 55, 56 für den Fall implantatseitiger sekundärer Energiespeicherelemente (Batterien 52) vorgesehen sowie eine beide Elektronikmodule 33 synchron steuernde drahtlose Fernbedienung 54 zur Anwendung durch den Implantatträger. Im Falle der Ausführungsbeispiele der Figuren 12, 13 und 14 sind die Aktoranordnungen gemäß Fig. 2 beziehungsweise Fig. 3 beziehungsweise Fig. 7 ausgelegt. Fig. 15 zeigt ein Ausführungsbeispiel, bei dem das duale intracochleäre Stimulationsarray 29 gemäß Fig. 7 mit einem Aktor 14 nach Fig. 2 kombiniert ist.

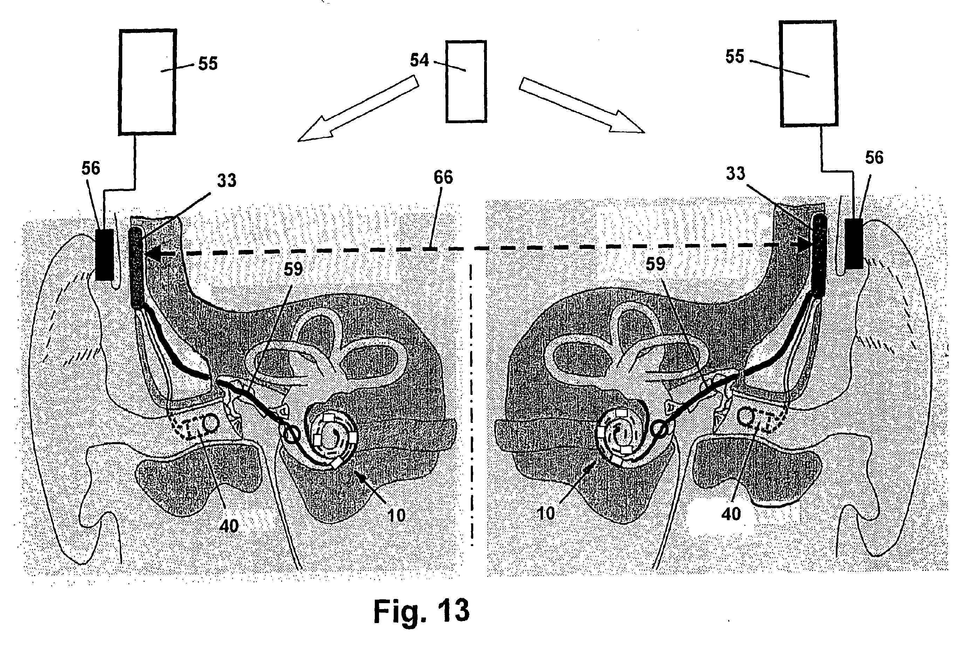

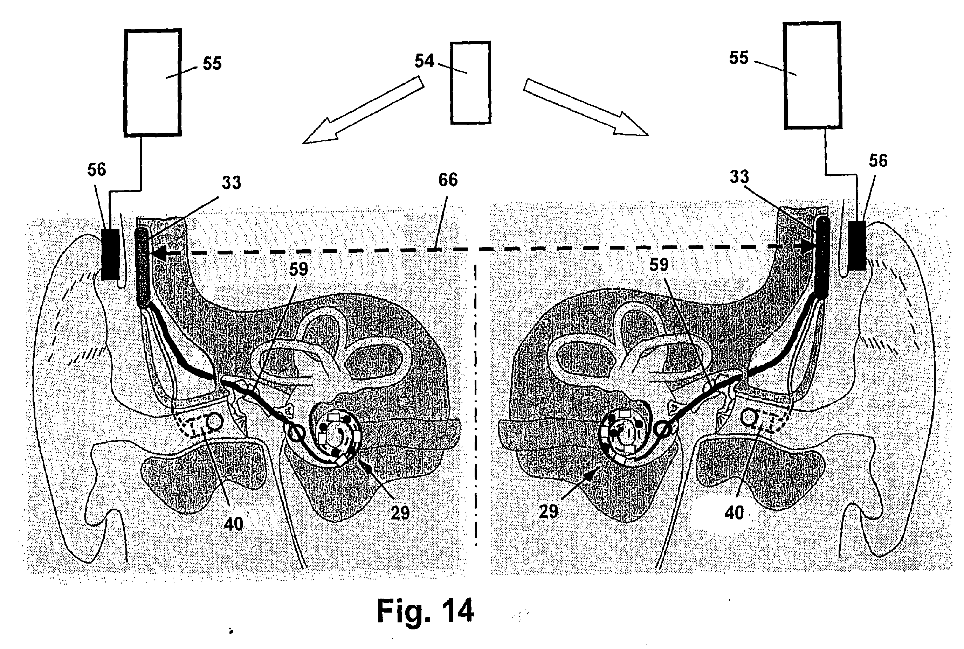

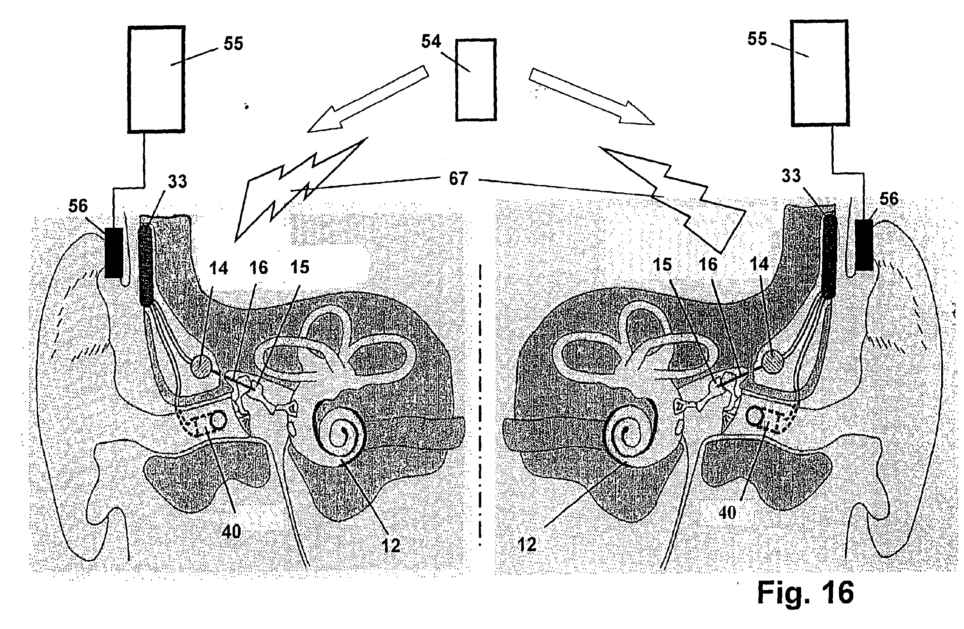

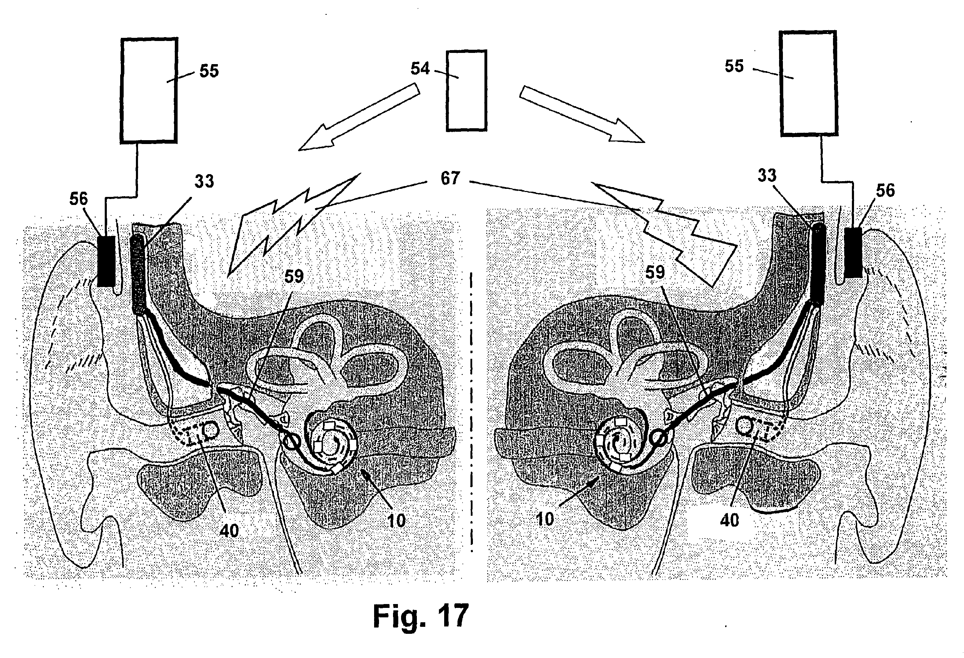

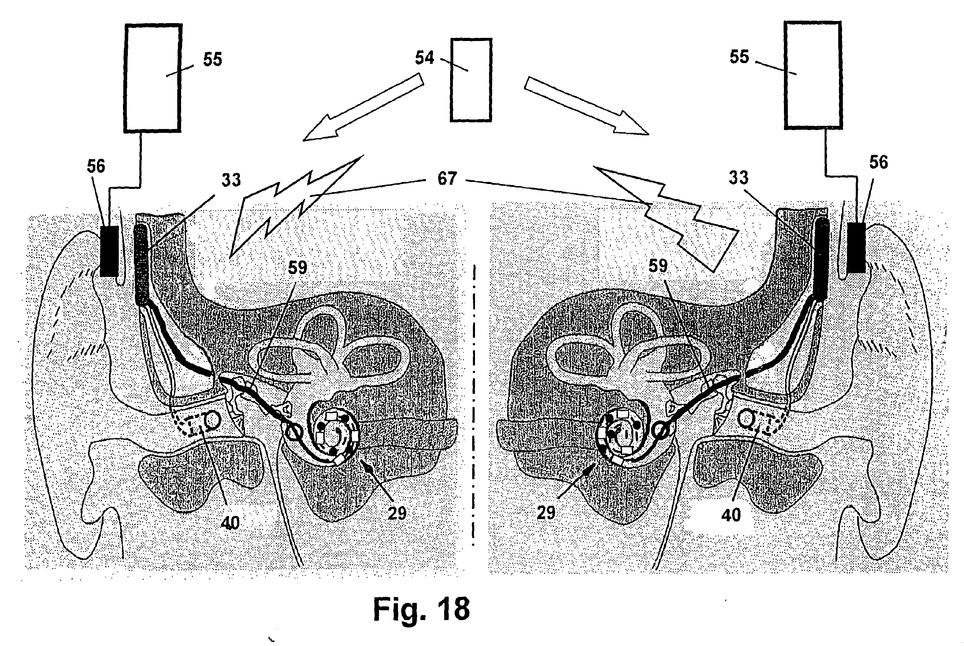

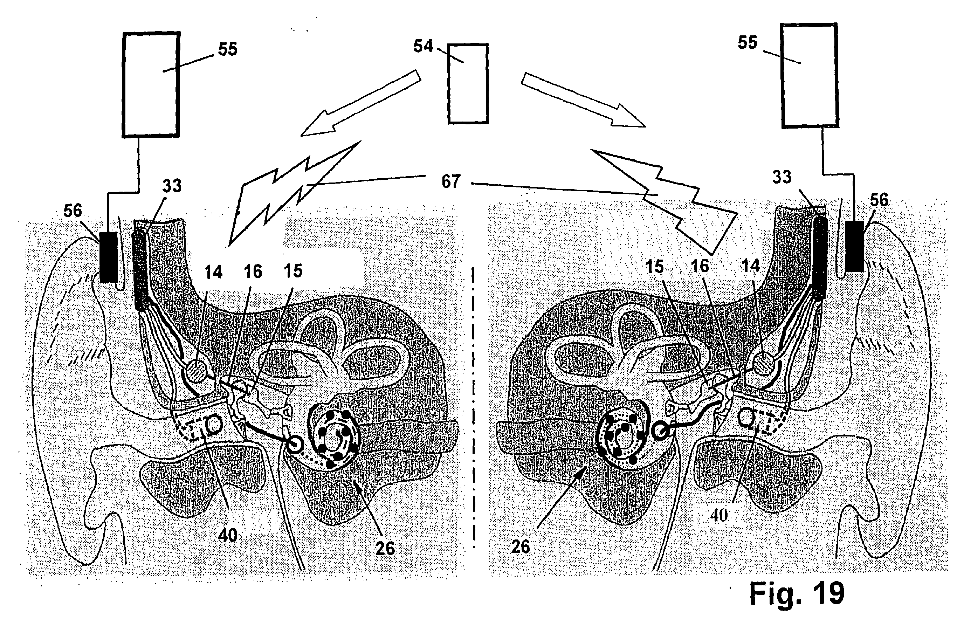

Die Figuren 16, 17, 18 und 19 zeigen jeweils eine binaurale Anwendung eines Hörimplantates, bei dem die Signalverarbeitungseinheiten 34 in den Elektronikmodulen 33 auf beiden Seiten des Schädels über eine drahtlose Verbindung, zum Beispiel eine bei 67 angedeutete bidirektionale Hochfrequenzstrecke, so miteinander kommunizieren, dass eine optimale binaurale sensorische Signalverarbeitung und Aktor-Ansteuerung in beiden implantierten Innenohren erreicht wird. Transkutane Ladevorrichtungen 55, 56 für den Fall implantatseitiger sekundärer Energiespeicherelemente (Batterien 52) sowie eine beide Elektronikmodule 33 synchron steuernde drahtlose Fernbedienung 54 zur Anwendung durch den Implantatträger sind auch hier vorgesehen. Die Ausführungsbeispiele der Figuren 16 bis 19 unterscheiden sich voneinander nur hinsichtlich der Aktoranordnungen. Die Aktoranordnungen gemäß den Figuren 16, 17 und 18 entsprechen denjenigen der Fig. 12 beziehungsweise Fig. 13 beziehungsweise Fig. 14. Die Aktoranordnung gemäß Fig. 19 entspricht derjenigen der Fig. 5.

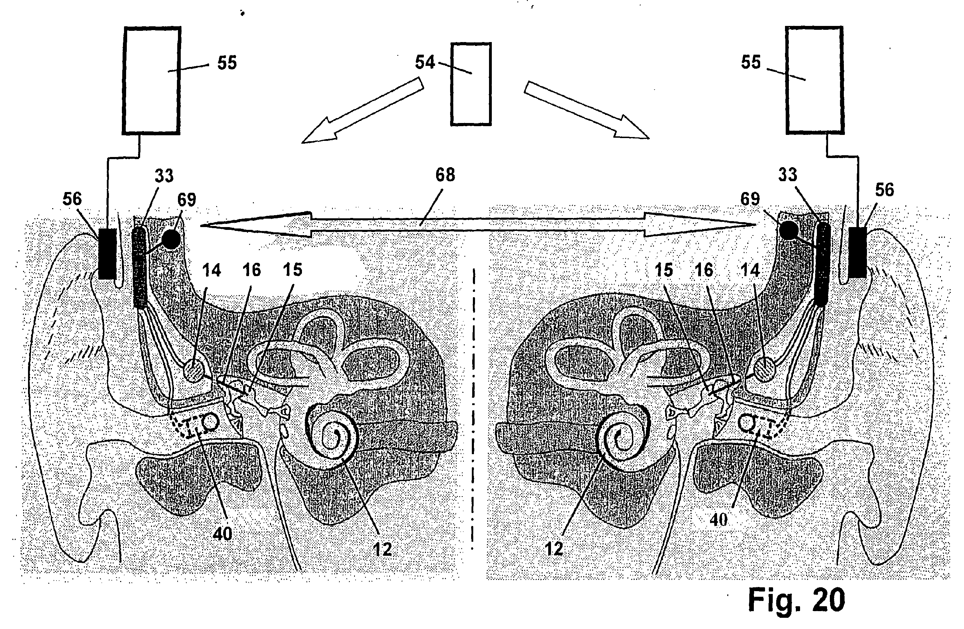

Die binaurale Ausbildung der Hörimplantate gemäß den Figuren 20, 21, 22 und 23 unterscheidet sich von denjenigen der Figuren 16 beziehungsweise 17 beziehungsweise 18 beziehungsweise 19 nur dadurch, dass für die drahtlose Kommunikation zwischen den Elektronikmodulen 33 beider Systemeinheiten eine körperschallgekoppelte Ultraschallstrecke 68 mit Ultraschall-Kopplern 69 vorgesehen ist. Dabei sind die zum Beispiel digitalen, bidirektionalen Informationen auf einen Träger im Ultraschallbereich vorzugsweise amplituden- oder frequenzmoduliert. Die Ultraschall-Koppler 69 können, wie in den Figuren 20 bis 23 dargestellt, von dem Elektronikmodul 33 örtlich getrennte und über elektrische Leitungen angeschlossene Ultraschallempfänger und -sender sein, die vorzugsweise im Mastoidbereich an den Schädelknochen fest angekoppelt sind. Die Ultraschall-Koppler können aber auch in den Elektronikmodulen 33 integriert sein (nicht dargestellt), wenn die Elektronikmodule so im Mastoidbereich implantiert werden, dass eine Ultraschall-Körperübertragung durch den Schädelknochen stattfinden kann.

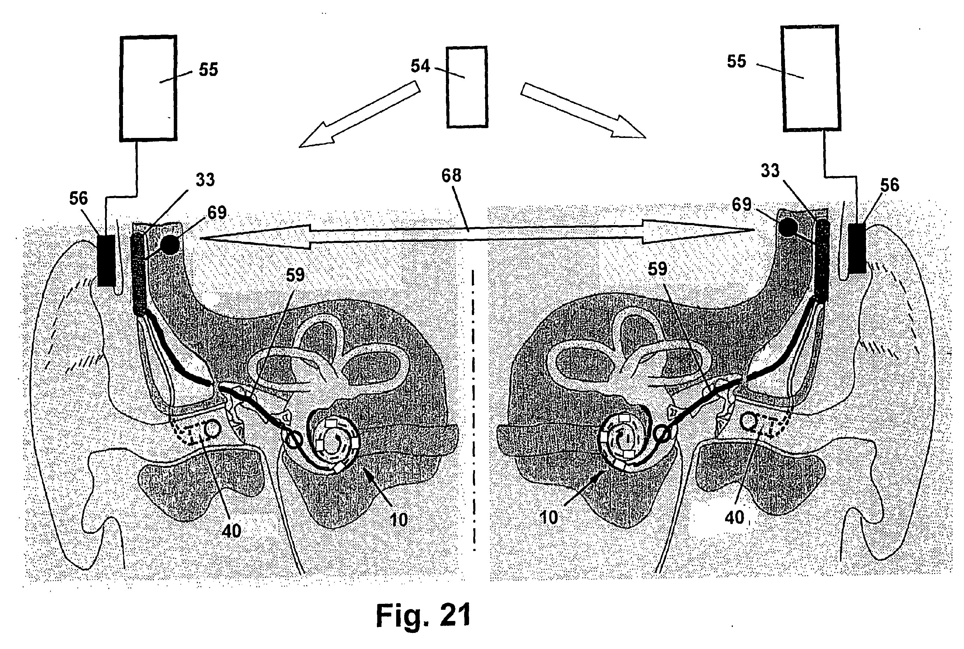

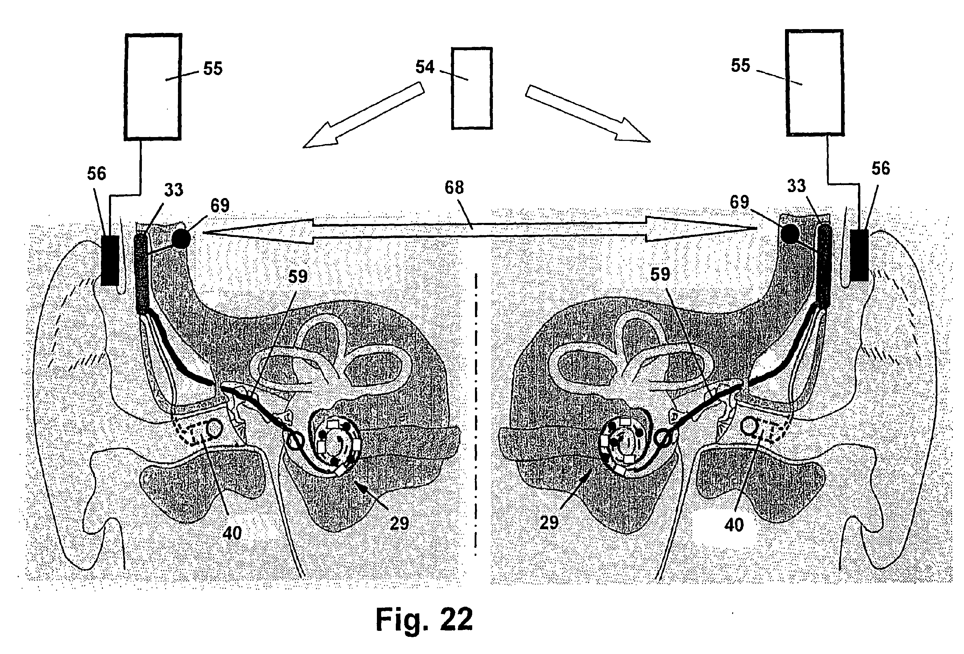

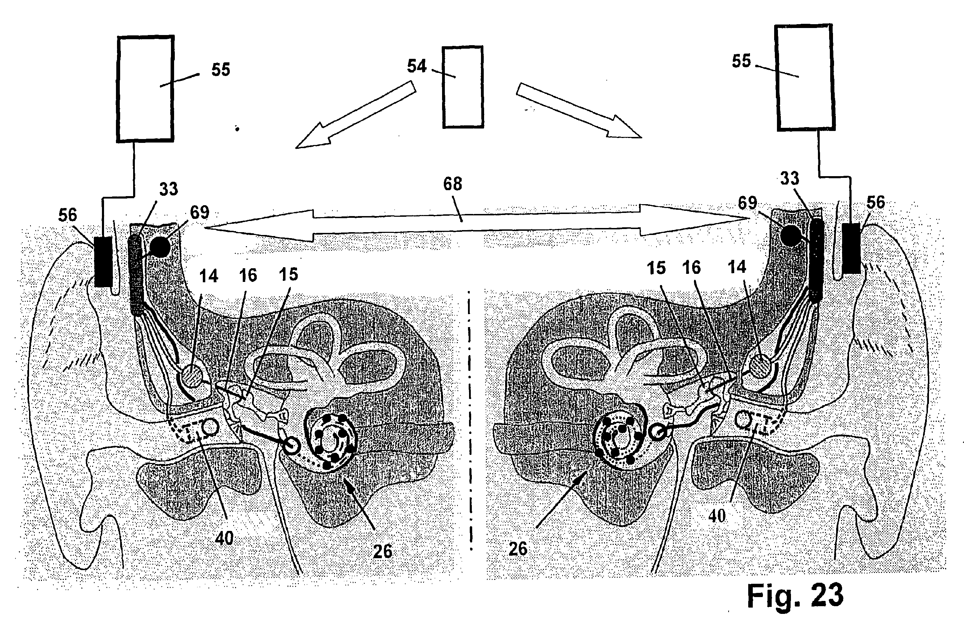

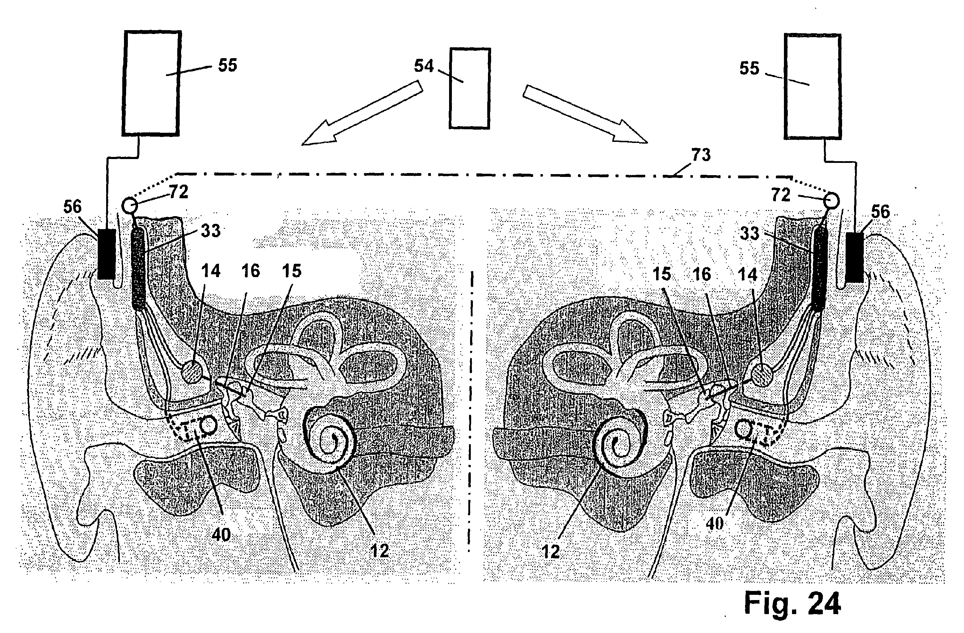

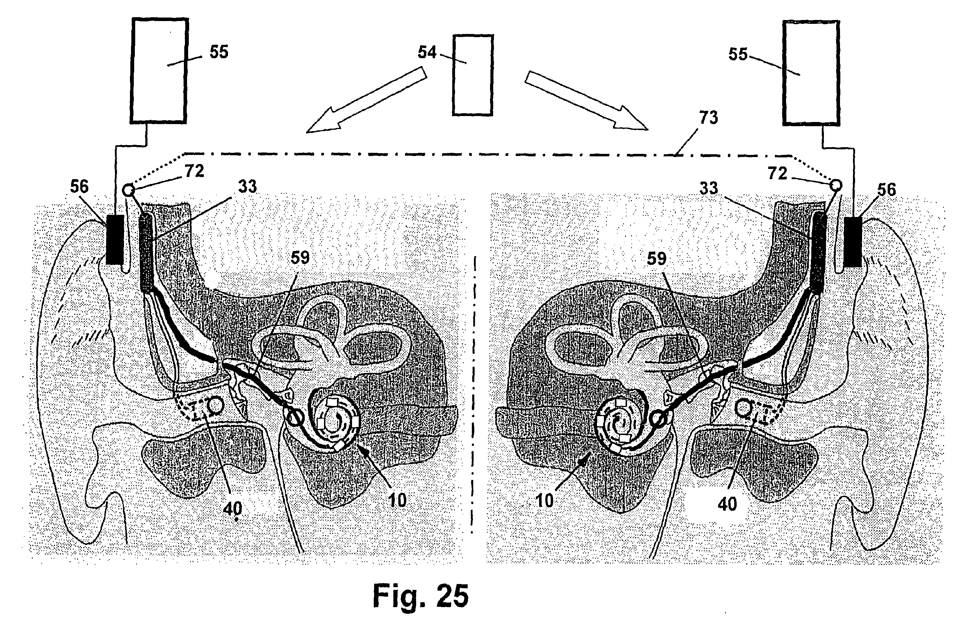

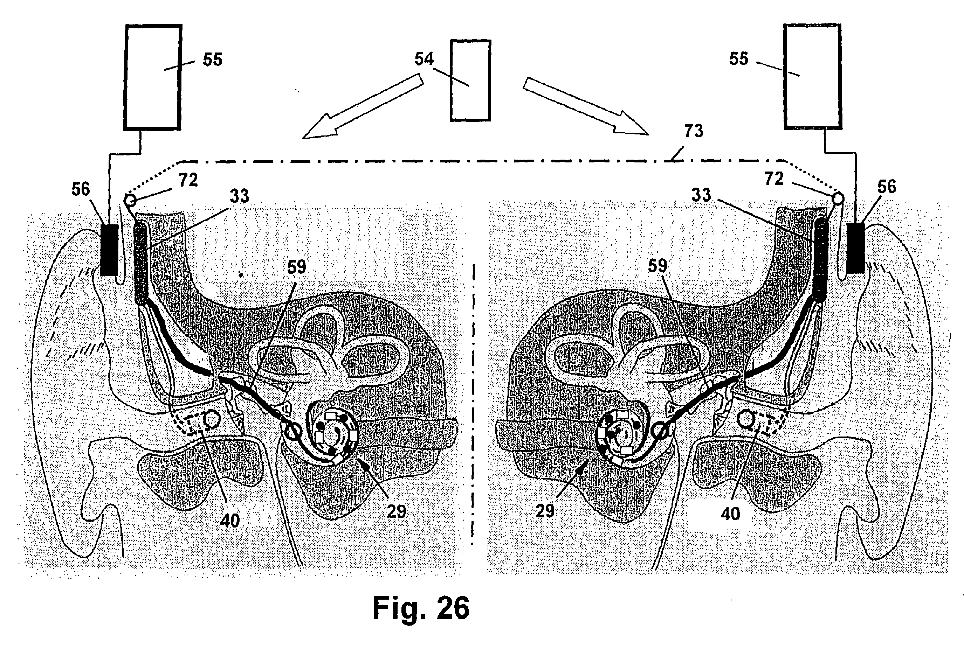

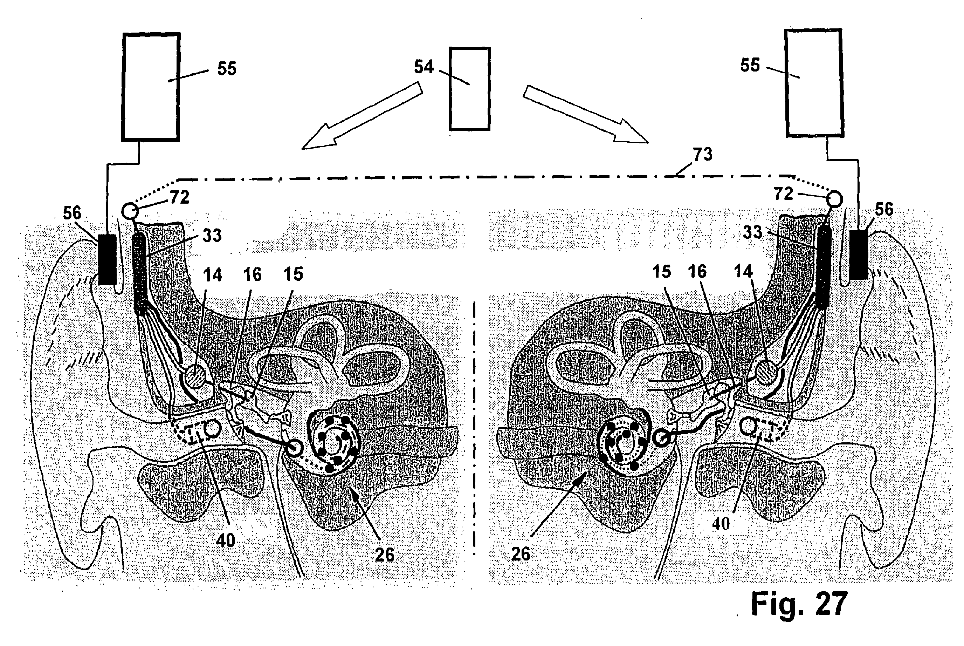

Weiter abgewandelte Ausführungsbeispiele eines binaural ausgebildeten Hörimplantates sind in den Figuren 24, 25, 26 und 27 dargestellt. Bei diesen Ausführungsformen werden abweichend von den Ausführungsbeispielen der Figuren 12 bis 23 die zum Beispiel digitalen, bidirektionalen Informationen implantatseitig auf einen Träger vorzugsweise amplituden- oder frequenzmoduliert und an implantierte Elektroden 72 angelegt, die Teil einer durch Körpergewebe des Implantatträgers führenden Datenübertragungsstrecke 73 sind. Es wird so ein modulierter Gewebestrom erhalten, der in an sich bekannter Weise (DE-A-38 31 809) für die gewünschte Kommunikation zwischen den Signalverarbeitungsmodulen 34 beider Systemeinheiten sorgt. Die Ausführungsbeispiele der Figuren 24 bis 27 unterscheiden sich voneinander wiederum nur hinsichtlich der Aktoranordnungen. Letztere entsprechen der Aktoranordnung gemäß Fig. 20 beziehungsweise Fig. 21 beziehungsweise Fig. 22 beziehungsweise Fig. 23.

Es versteht sich, dass auch ein teilimplantierbares System binaural appliziert werden kann und dass dann für eine Kommunikation zwischen den beiden Systemeinheiten vorzugsweise entsprechend den in den Figuren 12 bis 27 veranschaulichen Ausführungsbeispielen von binauralen Anwendungen vollimplantierbarer Systeme gesorgt werden kann.

## Patentansprüche

1. System zur Rehabilitation einer Hörstörung, das mindestens einen Schallsensor (40) zur Aufnahme des Schallsignals und dessen Umwandlung in ein elektrisches Audiosignal, eine elektronische Signalverarbeitungseinheit (34, 62) zur Audiosignalverarbeitung und -verstärkung, eine elektrische Energieversorgungseinheit (52), welche einzelne Komponenten des Systems mit Strom versorgt, und eine Aktoranordnung (10, 14, 24, 26, 29) umfasst, die mit einem oder mehreren elektroakustischen, elektromechanischen oder rein elektrischen ausgangsseitigen Aktoren oder einer beliebigen Kombination solcher Aktoren zur Stimulation des geschädigten Gehöres versehen ist, wobei die Signalverarbeitungseinheit (34, 62) ein Sprachanalyse- beziehungsweise -erkennungsmodul (18) und ein Sprachsynthesemodul (19) aufweist; **dadurch gekennzeichnet, dass** das Sprachanalysebeziehungsweise -erkennungsmodul (18) eine Anordnung zum Erkennen und Extrahieren von zusätzlichen Informationen bezüglich der Prosodie der Sprache aufweist, und dass das Sprachsynthesemodul (19) mit einer Anordnung zum Berücksichtigen dieser Prosodieinformationen bei der Sprachsynthese versehen ist.

2. System nach Anspruch 1, **dadurch gekennzeichnet, dass** die Anordnung zum Erkennen und Extrahieren der Prosodieinformationen zur Extraktion der Höhe und des Verlaufs der Sprachgrundfrequenz bei stimmhaften Lauten ausgelegt ist, und die Anordnung zum Berücksichtigen der Prosodieinformationen bei der Sprachsynthese auf der Syntheseseite des Systems eine entsprechende Modulation des Ausgangssignals bewirkt.

3. System nach Anspruch 1 oder 2, **dadurch gekennzeichnet, dass** die Signalverarbeitungseinheit (34, 62) einen digitalen Signalprozessor (42) aufweist, der in Software realisierte Module (18, 19) zur Sprachanalyse und -synthese enthält.

4. System nach Anspruch3, **dadurch gekennzeichnet, dass** die Sprachanalyse- beziehungsweise -erkennungs- und Sprachsynthesemodule (18, 19) lernfähig aufgebaut sind.

5. System nach Anspruch 3 oder 4, **dadurch gekennzeichnet, dass** die Sprachanalysebeziehungsweise -erkennungs- und Sprachsynthesemodule (18, 19) umprogrammierbar sind.

6. System nach einem der vorhergehenden Ansprüche, **dadurch gekennzeichnet, dass** die Sprachanalyse- beziehungsweise -erkennungs- und Sprachsynthesemodule (18, 19) ein digital realisiertes neuronales Netzwerk beinhalten.

7. System nach einem der vorhergehenden Ansprüche, **dadurch gekennzeichnet, dass** die Sprachanalyse- beziehungsweise -erkennungs- und Sprachsynthesemodule (18, 19) derart ausgelegt sind, dass zwischen ihnen phonetische Kategorien übertragen werden.

8. System nach einem der vorhergehenden Ansprüche, **dadurch gekennzeichnet, dass** die Sprachanalyse- beziehungsweise -erkennungs- und Sprachsynthesemodule (18, 19) derart ausgelegt sind, dass zwischen ihnen lexikalische Kategorien übertragen werden.

9. System nach einem der vorhergehenden Ansprüche, **dadurch gekennzeichnet, dass** die Sprachanalyse- beziehungsweise -erkennungs- und Sprachsynthesemodule (18, 19) ausschaltbar sind, um das Audiosignal ohne Sprachanalyse- und -synthese zu verarbeiten, wenn kein oder nur wenig Störschall vorliegt.

10. System nach Anspruch 9, **dadurch gekennzeichnet, dass** Mittel (21) zum automatischen Ausschalten der Sprachanalyse- beziehungsweise -erkennungs- und Sprachsynthesemodule (18, 19) bei geringem Störschallpegel vorgesehen sind.

11. System nach Anspruch 9 oder 10, **dadurch gekennzeichnet, dass** Mittel (21, 48, 54) zum Ausschalten der Sprachanalyse- beziehungsweise -erkennungs- und Sprachsynthesemodule (18, 19) durch eine Fernbedienungsfunktion durch den Anwender vorgesehen sind.

12. System nach einem der vorhergehenden Ansprüche, **dadurch gekennzeichnet, dass** die Signalverarbeitungseinheit (34, 62) Softwaremodule enthält, die parallel zum Hörgerätebetrieb die Maskierung eines Tinnitus ermöglichen.

13. System nach einem der vorhergehenden Ansprüche, **dadurch gekennzeichnet, dass** die Signalverarbeitungseinheit (34, 62) eine Vorverarbeitungsanordnung (41) zum Vorverstärken und/oder Filtern sowie zum Analog/Digital- (A/D-) Wandeln der Schallsensorsignale aufweist.

14. System nach Anspruch 13, **dadurch gekennzeichnet, dass** die Vorverarbeitungsanordnung (41) einen Antialiasing-Filter umfasst.

15. System nach einem der vorhergehenden Ansprüche, **dadurch gekennzeichnet, dass** bei Vorhandensein mehrerer Schallsensoren (40) jedem der Schallsensoren ein Analog/Digital-Wandler (41) nachgeschaltet ist.

16. System nach einem der vorhergehenden Ansprüche, **gekennzeichnet durch** mindestens einen der Aktoranordnung (10, 14, 24, 26, 29) vorgeschalteten Digital/Analog-Wandler (43).

17. System nach Anspruch 16, **dadurch gekennzeichnet, dass** den ausgangsseitigen Aktoren (14, 27) jeweils ein eigener Digital/Analog-Wandler (43) vorgeschaltet ist.

18. System nach einem der vorhergehenden Ansprüche, **dadurch gekennzeichnet, dass** die Signalverarbeitungseinheit (34, 62) einen digitalen Signalprozessor (42) zum Verarbeiten der A/D-gewandelten und gegebenenfalls mittels der Vorverarbeitungsanordnung (41) vorverarbeiteten Schallsensorsignale und/oder zum Generieren von digitalen Signalen für eine Tinnitusmaskierung aufweist.

19. System nach einem der vorhergehenden Ansprüche, **dadurch gekennzeichnet, dass** es mindestens teilweise implantierbar ausgebildet ist und dass eine vorzugsweise PC-basierte, drahtlose Telemetrieeinrichtung (46) zur Übertragung von Daten zwischen einem implantierten Teil (33) des Systems und einer externen Einheit, insbesondere einem externen Programmiersystem (48), vorgesehen ist.

20. System nach Anspruch 19, **dadurch gekennzeichnet, dass** dem Signalprozessor (42) zur Aufnahme und Wiedergabe eines Betriebsprogramms eine wiederholt beschreibbare, implantierbare Speicheranordnung (S₁, S₂) zugeordnet ist, und mindestens Teile des Betriebsprogramms durch von der externen Einheit (48) über die Telemetrieeinrichtung (46) übermittelte Daten geändert oder ausgetauscht werden können.

21. System nach Anspruch 20, **dadurch gekennzeichnet, dass** ferner eine Zwischenspeicheranordnung (S₄, S₅) vorgesehen ist, in welcher von der externen Einheit (48) über die Telemetrieeinrichtung (46) übermittelte Daten vor dem Weiterleiten an den Signalprozessor (42) zwischengespeichert werden können.

22. System nach Anspruch 21, **dadurch gekennzeichnet, dass** ferner eine Überprüfungslogik (44) vorgesehen ist, um in der Zwischenspeicheranordnung (S₄, S₅) gespeicherte Daten vor dem Weiterleiten an den Signalprozessor (42) einer Überprüfung zu unterziehen.

23. System nach einem der Ansprüche 19 bis 22, **gekennzeichnet durch** einen Mikroprozessorbaustein (44), insbesondere einen Microcontroller, zum implantatinternen Steuern der Analog/Digital-Wandler (41) und/oder der Digital/Analog-Wandler (43) und/oder des Signalprozessors (42) über einen Datenbus (50).

24. System nach den Ansprüchen 22 und 23, **dadurch gekennzeichnet, dass** die Überprüfungslogik und die Zwischenspeicheranordnung (S₄, S₅) in dem Mikroprozessorbaustein (44) implementiert sind.

25. System nach Anspruch 23 oder 24, **dadurch gekennzeichnet, dass** über den Datenbus (50) und die Telemetrieeinrichtung (46) auch Programmteile oder ganze Softwaremodule zwischen der Außenwelt, dem Mikroprozessorbaustein (44) und dem Signalprozessor (42) übermittelbar sind.

26. System nach einem der Ansprüche 23 bis 25, **dadurch gekennzeichnet, dass** dem Mikroprozessorbaustein (44) eine implantierbare Speicheranordnung (S₃) zum Speichern eines Arbeitsprogramms für den Mikroprozessorbaustein zugeordnet ist, und mindestens Teile des Arbeitsprogramms für den Mikroprozessorbaustein durch von der externen Einheit (48) über die Telemetrieeinrichtung (46) übermittelte Daten geändert oder ausgetauscht werden können.

27. System nach einem der Ansprüche 20 bis 26, **dadurch gekennzeichnet, dass** mindestens zwei Speicherbereiche (S₁, S₂) zur Aufnahme und Wiedergabe mindestens des Betriebsprogramms des Signalprozessors (42) vorgesehen sind.

28. System nach einem der Ansprüche 21 bis 27, **dadurch gekennzeichnet, dass** die Zwischenspeicheranordnung mindestens zwei Speicherbereiche (S₄, S₅) zur Aufnahme und Wiedergabe von von der externen Einheit (48) über die Telemetrieeinrichtung (46) übermittelten Daten aufweist.

29. System nach einem der Ansprüche 18 bis 28, **dadurch gekennzeichnet, dass** dem Signalprozessor (42) ferner ein vorprogrammierter, nicht überschreibbarer Festspeicherbereich (S₀) zugeordnet ist.

30. System nach einem der Ansprüche 18 bis 29, **dadurch gekennzeichnet, dass** die Telemetrieeinrichtung (46) zur Übermittlung auch von Betriebsparametern zwischen dem implantierbaren Teil (33) des Systems und der externen Einheit (48) ausgelegt ist.

31. System nach einem der vorhergehenden Ansprüche, **dadurch gekennzeichnet, dass** es vollimplantierbar ausgebildet und mit mindestens einem implantierbaren Schallsensor (40) versehen ist, die elektrische Energieversorgungseinheit implantatseitig ein nachladbares elektrisches Speicherelement (52) aufweist und eine drahtlose, transkutane Ladevorrichtung (55, 56) zum Laden des Speicherelements vorgesehen ist.

32. System nach Anspruch 31, **gekennzeichnet durch** eine drahtlose Fernbedienung (54) zur Steuerung der Implantatfunktionen **durch** den Implantatträger.

33. System nach einem der Ansprüche 1 bis 31, **dadurch gekennzeichnet, dass** es teilimplantierbar ausgebildet ist, wobei mindestens ein Schallsensor (40), die elektronische Signalverarbeitungseinheit (62) zur Audiosignalverarbeitung und -verstärkung, die Energieversorgungseinheit (52) sowie eine Modulator/Sender-Einheit (63) in einem extern am Körper, vorzugsweise am Kopf über dem Implantat (33'), zu tragenden externen Modul (64) enthalten sind, sowie das Implantat energetisch passiv ist und seine Betriebsenergie und Wandlersteuerdaten über die Modulator/Sender-Einheit im externen Modul empfängt.

34. System nach einem der vorhergehenden Ansprüche, **dadurch gekennzeichnet, dass** als ausgangsseitige Aktoren (14) elektromechanische Wandler zur Anregung der flüssigkeitsgefüllten Innenohrräume des geschädigten Innenohrs vorgesehen sind, und dass die Signalverarbeitungseinheit (34, 62) eine treibende Signalverarbeitungselektronik (42, 44) aufweist, die jeden der Wandler derart elektrisch ansteuert, dass auf der Basilarmembran des geschädigten Innenohres eine Wanderwellenkonfiguration entsteht, die die Art der Wanderwellenausbildung eines gesunden, nicht geschädigten Innenohres approximiert.

35. System nach Anspruch 34, **dadurch gekennzeichnet, dass** die ausgangsseitigen Aktoren (14) für eine direkte Anregung der flüssigkeitsgefüllten Innenohrräume des geschädigten Innenohrs ausgelegt sind.

36. System nach Anspruch 35, **gekennzeichnet durch** ein intracochleäres Array (10) von ausgangsseitigen Aktoren (14) in Form von elektromechanischen Wandlern.

37. System nach Anspruch 36, **dadurch gekennzeichnet, dass** das intracochleäre Wandler-Array (10) einen Träger (11) aus einem biokompatiblen und im Innenohr biostabilen Material, vorzugsweise einem Polymer, insbesondere einem Silikon, aufweist.

38. System nach Anspruch 37, **dadurch gekennzeichnet, dass** die einzelnen ausgangsseitigen Aktoren (14) so in den Träger (11) eingebettet sind, dass sie von einer dünnen Schicht des Trägermaterials vollständig umgeben sind.

39. System nach Anspruch 37 oder 38, **dadurch gekennzeichnet, dass** zwischen den einzelnen ausgangsseitigen Aktoren (14) mechanische Dämpfungselemente (22) in den Träger (11) eingebettet sind, die die mechanische Wellenausbreitung innerhalb des Trägers zu benachbarten Wandlern minimieren.

40. System nach Anspruch 39, **dadurch gekennzeichnet, dass** das Material der Dämpfungselemente (22) bei ähnlicher Querschnittsgeometrie wie die des Trägers (11) so gewählt ist, dass zur Erzielung hoher Dämpfungswerte ein großer mechanischer Impedanzunterschied zu dem Trägermaterial besteht.

41. System nach Anspruch 34 oder 35, **gekennzeichnet durch** ein vorzugsweise mikrosystemtechnisch hergestelltes extracochleäres Array (24) von ausgangsseitigen Aktoren (14) in Form von elektromechanischen Wandlern.

42. System nach Anspruch 41, **dadurch gekennzeichnet, dass** für das extracochleäre Wandler-Array (24) ein Substrat (25) vorgesehen ist, das ein mikrosystemtechnisch mitgefertigtes elektrisches Anschlussfeld (36) enthält, das für den Anschluss einer mehrpoligen, biokompatiblen Aktorzuleitung (59) zu der Signalverarbeitungseinheit (34) ausgelegt ist.

43. System nach Anspruch 41 oder 42, **dadurch gekennzeichnet, dass** für das extracochleäre Wandler-Array (24) ein Substrat (25) mit einem mikrosystemtechnisch mitgefertigten Elektronikmodul (37) vorgesehen ist.

44. System nach Anspruch 43, **dadurch gekennzeichnet, dass** das Elektronikmodul (37) Treiberstufen zum Ansteuern der ausgangsseitigen Aktoren (14) und/oder eine Decodierlogik und Umsetzerbausteine für den Anschluss einer polreduzierten Aktorzuleitung (59) enthält.

45. System nach Anspruch 44, **dadurch gekennzeichnet, dass** der Array-Anschluss aus nur drei Leitungen, insbesondere einer Masseleitung, einer Datenleitung und einer Taktsignalleitung, besteht und die Versorgung mit elektrischer Betriebsenergie durch Phantomspeisung auf der Taktsignalleitung erfolgt.

46. System nach einem der Ansprüche 42 bis 45, **dadurch gekennzeichnet, dass** das Elektronikmodul (37) einen Schnittstellenbaustein für eine digitale Datenübertragung über die Aktorzuleitung (59), vorzugsweise mittels einer Lichtleitfaser, enthält.

47. System nach einem der Ansprüche 42 bis 46, **dadurch gekennzeichnet, dass** das Elektronikmodul (37) bei serieller Datenübertragung auf der Aktorzuleitung (59) entsprechende den Aktoren (14) zugeordnete D/A-Wandler und Treiberbausteine enthält.

48. System nach Anspruch 3 und einem der Ansprüche 42 bis 47, **dadurch gekennzeichnet, dass** die Aktoren (14) des extracochleären Wandler-Arrays (24) jeweils ein ausgangsseitiges Koppelelement (28) aufweisen, das so ausgebildet ist, dass es durch eine Öffnung der Cochlea-Wand in einen flüssigkeitsgefüllten Innenohrraum ragen kann.

49. System nach einem der Ansprüche 36 bis 48, **dadurch gekennzeichnet, dass** die ausgangsseitigen Aktoren (14) in dem Wandler-Array (10, 24) äquidistant oder in logarithmischen Distanzen - entsprechend der tonotopen Frequenz-Orts-Zuordnung längs der Basilarmembran des Innenohres - verteilt angeordnet sind.

50. System nach einem der Ansprüche 1 bis 33, **dadurch gekennzeichnet, dass** die Aktoranordnung in Kombination einen elektromechanischen Wandler (14) zur mechanischen Stimulation des Mittel- oder Innenohres und ein intracochleäres, elektrisch wirkendes Reizelektrodenarray (26) mit mindestens einer Reizelektrode (27) zur elektrischen Stimulation des Innenohres aufweist.

51. System nach einem der Ansprüche 1 bis 33, **gekennzeichnet durch** eine duale intracochleäre Anordnung (29), die in Kombination eine Aktororanordnung mit mindestens einem Aktorelement (14) zur mindestens mittelbaren mechanischen Stimulation des Innenohres und eine elektrisch wirkende Reizelektrodenanordnung mit mindestens einer Cochlea-Implant-Elektrode (27) zur elektrischen Stimulation des Innenohres aufweist.

52. System nach Anspruch 51, **dadurch gekennzeichnet, dass** die Aktoranordnung mindestens einen intracochleären elektromechanischen Wandler (14) zur unmittelbaren mechanischen Stimulation des Innenohres aufweist.

53. System nach Anspruch 51, **dadurch gekennzeichnet, dass** die Aktoranordnung mindestens eine intracochleäre Haarzellen-Reizelektrode (27) zur mittelbaren mechanischen Stimulation des Innenohres durch elektrische Reizung von äußeren Haarzellen aufweist.

54. System nach Ansprüchen 52 und 53, **dadurch gekennzeichnet, dass** elektromechanische Wandler (14) und Cochlea-Implant-Elektroden (27) entlang einem gemeinsamen Träger (30) einander abwechselnd angeordnet sind.

55. System nach einem der Ansprüche 34 bis 54, **dadurch gekennzeichnet, dass** der oder die ausgangsseitige(n) elektromechanische(n) Wandler (14) als elektromagnetische(r), elektrodynamische(r), piezoelektrische(r), magnetostriktiv(r)e oder dielektrische(r) (kapazitive(r)) Wandler ausgebildet ist beziehungsweise sind.

56. System nach Anspruch 55, **dadurch gekennzeichnet, dass** der oder die ausgangsseitige(n) elektromechanische(n) Wandler (14) bei Anwendung des piezoelektrischen Wandlerprinzips unter Verwendung von PZT-Keramik (Blei-Zirkonat-Titanat) oder PVDF (Polyvinylidenfluorid) aufgebaut ist beziehungsweise sind.

57. System nach einem der Ansprüche 34 bis 56, **dadurch gekennzeichnet, dass** der oder die ausgangsseitige(n) elektromechanische(n) Wandler (14), vorzugsweise mit Ausnützung von geometrischen Gestalttransformationen, insbesondere des Bimorph-Prinzips, des Unimorph-Prinzips oder des Heteromorph-Prinzips mit passiven Materialpartnern, so ausgeführt ist beziehungsweise sind, dass bei gegebener Wandlerspannung eine maximale Auslenkung bei minimaler elektrischer Leistungsaufnahme erhalten wird.

58. System nach einem der vorhergehenden Ansprüche, **dadurch gekennzeichnet, dass** es als binaurales System zur Rehabilitation einer Hörstörung beider Ohren ausgelegt ist, das zwei Systemeinheiten aufweist, die jeweils einem der beiden Ohren zugeordnet sind.

59. System nach Anspruch 58, **dadurch gekennzeichnet, dass** die beiden Systemeinheiten einander im wesentlichen gleich sind.

60. System nach Anspruch 58, **dadurch gekennzeichnet, dass** die eine Systemeinheit als Master-Einheit und die andere Systemeinheit als von der Master-Einheit gesteuerte Slave-Einheit ausgelegt ist.

61. System nach einem der Ansprüche 58 bis 60, **gekennzeichnet durch** eine drahtgebundene implantierbare Leitungsverbindung (66), über welche die Signalverarbeitungsmodule (33) so miteinander kommunizieren, dass in beiden Systemeinheiten eine optimierte binaurale Signalverarbeitung und Wandler-Array-Ansteuerung erreicht wird.

62. System nach einem der Ansprüche 58 bis 60, **gekennzeichnet durch** eine drahtlose Verbindung (67), vorzugsweise eine bidirektionale Hochfrequenzstrecke, über welche die Signalverarbeitungsmodule (33) so miteinander kommunizieren, dass in beiden Systemeinheiten eine optimierte binaurale Signalverarbeitung und Wandler-Array-Ansteuerung erreicht wird.

63. System nach einem der Ansprüche 58 bis 60, **dadurch gekennzeichnet, dass** die Signalverarbeitungsmodule (33) unter Verwendung von Ultraschall-Kopplern (69) über eine körperschallgekoppelte Ultraschallstrecke (68) so miteinander kommunizieren, dass in beiden Systemeinheiten eine optimierte binaurale Signalverarbeitung und Wandler-Array-Ansteuerung erreicht wird.

64. System nach einem der Ansprüche 58 bis 60, **dadurch gekennzeichnet, dass** den Signalverarbeitungsmodulen (33) implantierbare Elektroden (72) zugeordnet sind, die im implantierten Zustand Teil einer durch Körpergewebe des Implantatträgers führenden Datenübertragungsstrecke (73) für eine Kommunikation der Signalverarbeitungsmodule der beiden Systemeinheiten sind.

## Claims

1. A system for rehabilitation of a hearing disorder comprising at least one acoustic sensor (40) for picking up the acoustic signal and converting the latter into an electrical audio signal, an electronic signal processing unit (34, 62) for audio signal processing and amplification, an electrical power supply unit (52) which supplies individual components of the system with current, and an actuator arrangement (10, 14, 24, 26, 29) which is provided with one or more electroacoustic, electromechanical or purely electrical output side actuator(s) or any combination of such actuators for stimulation of the damaged hearing, the signal processing unit (34, 62) having a speech analysis and recognition module (18) and a speech synthesis module (19); **characterised in that** the speech analysis and recognition module (18) has an arrangement for detecting and extracting additional information with regard to the prosody of the speech, and that the speech synthesis module (19) is provided with an arrangement for taking into account this prosody information in the speech synthesis.

2. The system according to Claim 1, **characterised in that** the arrangement for detecting and extracting the prosody information is designed for extraction of the level and development of the fundamental speech frequency for voiced sounds, and the arrangement for taking into account the prosody information in speech synthesis brings about a corresponding modulation of the output signal on the synthesis side of the system.

3. The system according to Claim 1 or 2, **characterised in that** the signal processing unit (34, 62) has a digital signal processor (42) which contains software modules (18, 19) for speech analysis and synthesis.

4. The system according to Claim 3, **characterised in that** the speech analysis and recognition module (18) and the speech synthesis module (19) are adaptive.

5. The system according to Claim 3 or 4, **characterised in that** the speech analysis and recognition module (18) and the speech synthesis module (19) are re-programmable.

6. The system according to any of the preceding claims, **characterised in that** the speech analysis and recognition module (18) and the speech synthesis module (19) include a digitally implemented neuronal network.

7. The system according to any of the preceding claims, **characterised in that** the speech analysis and recognition module (18) and the speech synthesis module (19) are designed to transmit phonetic categories between them.

8. The system according to any of the preceding claims, **characterised in that** the speech analysis and recognition module (18) and the speech synthesis module (19) are designed to transmit lexical categories between them.

9. The system according to any of the preceding claims, **characterised in that** the speech analysis and recognition module (18) and the speech synthesis module (19) can be turned off in order to process the audio signal without speech analysis and synthesis if there is no or only a little interfering sound.

10. The system according to Claim 9, **characterised in that** means (21) are provided for automatically turning off the speech analysis and recognition module (18) and the speech synthesis module (19) at a low level of interfering sound.

11. The system according to Claim 9 or 10, **characterised in that** means (21, 48, 54) are provided so that the user can turn off the speech analysis and recognition module (18) and the speech synthesis module (19) with a remote control function.

12. The system according to any of the preceding claims, **characterised in that** the signal processing unit (34, 62) contains software modules which enable masking of tinnitus parallel to operation of the hearing aid.

13. The system according to any of the preceding claims, **characterised in that** the signal processing unit (34, 62) has a pre-processing arrangement (41) for pre-amplifying and/or filtering and for the analogue/digital (A/D) conversion of the acoustic sensor signals.

14. The system according to Claim 13, **characterised in that** the pre-processing arrangement (41) comprises an anti-aliasing filter.

15. The system according to any of the preceding claims, **characterised in that** with a plurality of acoustic sensors (40) provided, an analogue/digital converter (41) is connected downstream of each of the acoustic sensors.

16. The system according to any of the preceding claims, **characterised by** at least one digital/analogue converter connected upstream of the actuator arrangement (10, 14, 24, 26, 29).

17. The system according to Claim 16, **characterised in that** a respective digital/analogue converter (43) is connected upstream of each of the actuators (14, 27) on the output side.

18. The system according to any of the preceding claims, **characterised in that** the signal processing unit (34, 62) has a digital signal processor (42) for processing the A/D-converted acoustic sensor signals optionally pre-processed by means of the pre-processing arrangement (41) and/or for generating digital signals for tinnitus masking.

19. The system according to any of the preceding claims, **characterised in that** it is designed to be at least partially implantable and that a preferably PC-based, wireless telemetry means (46) is provided for transmitting data between an implanted part (33) of the system and an external unit, in particular an external programming system (48).

20. The system according to Claim 19, **characterised in that** a rewritable implantable storage arrangement (S₁, S₂) is associated with the signal processor (42) for accommodating and reproducing an operating programme, and at least parts of the operating programme can be changed or replaced by data conveyed from the external unit (48) via the telemetry means (46).

21. The system according to Claim 20, **characterised in that** a buffer storage arrangement (S₄, S₅) is furthermore provided in which data conveyed from the external unit (48) via the telemetry means (46) can be intermediately stored before being relayed to the signal processor (42).

22. The system according to Claim 21, **characterised in that** a checking logic (44) is furthermore provided for checking data stored in the buffer storage arrangement (S₄, S₅) before being relayed to the signal processor (42).

23. The system according to any of Claims 19 to 22, **characterised by** a microprocessor module (44), in particular a microcontroller, for implant-internal control of the analogue/digital converter (41) and/or the digital/analogue converter (43) and/or the signal processor (42) via a data bus (50).

24. The system according to Claims 22 and 23, **characterised in that** the checking logic and the buffer storage arrangement (S₄, S₅) are implemented in the microprocessor module (44).

25. The system according to Claim 23 or 24, **characterised in that** programme parts or whole software modules can also be conveyed between the outside world, the microprocessor module (44) and the signal processor (42) via the data bus (50) and the telemetry means (46).

26. The system according to any of Claims 23 to 25, **characterised in that** an implantable storage arrangement (S₃) for storing a working programme for the microprocessor module is associated with the microprocessor module (44), and at least parts of the working programme for the microprocessor module can be changed or replaced by data conveyed from the external unit (48) via the telemetry means (46).

27. The system according to any of Claims 20 to 26, **characterised in that** at least two storage areas (S₁, S₂) are provided for holding and reproducing at least the operating programme of the signal processor (42).

28. The system according to any of Claims 21 to 27, **characterised in that** the buffer storage arrangement has at least two storage areas (S₄, S₅) for holding and reproducing data conveyed from the external unit (48) via the telemetry means (46).

29. The system according to any of Claims 18 to 28, **characterised in that** a pre-programmed read-only memory area (S₀) which can not be overwritten is furthermore associated with the signal processor (42).

30. The system according to any of Claims 18 to 29, **characterised in that** the telemetry means (46) is designed to also convey operating parameters between the implantable part (33) of the system and the external unit (48).

31. The system according to any of the preceding claims, **characterised in that** it is designed to be completely implantable and is provided with at least one implantable acoustic sensor (40), the electric energy power unit has a rechargeable electrical storage element (52) on the implant side, and a wireless, transcutaneous charging device (55, 56) is provided for charging the storage element.

32. The system according to Claim 31, **characterised by** a wireless remote control (54) so that the person wearing the implant can control the implant functions.

33. The system according to any of Claims 1 to 31, **characterised in that** it is designed to be partially implantable, at least one acoustic sensor (40), the electronic signal processing unit (62) for audio signal processing and amplification, the power supply unit (52) and a modulator/transmitter unit (63) being contained in an external module (64) to be worn externally on the body, preferably on the head over the implant (33'), and the implant being passive in terms of energy, and receiving its operating energy and converter control data via the modulator/transmitter unit in the external module.

34. The system according to any of the preceding claims, **characterised in that** electromechanical converters are provided as output side actuators (14) for excitation of the fluid-filled inner-ear spaces of the damaged inner ear, and that the signal processing unit (34, 62) has driving signal processing electronics (42, 44) that electrically trigger each of the convertors such that a travelling wave configuration is formed on the basilar membrane of the damaged inner ear which approximates the type of travelling wave formation of a healthy, undamaged inner ear.

35. The system according to Claim 34, **characterised in that** the output side actuators (14) are designed for direct excitation of the fluid-filled inner ear spaces of the damaged inner ear.

36. The system according to Claim 35, **characterised by** an intracochlear array (10) of output side actuators (14) in the form of electromechanical converters.

37. The system according to Claim 36, **characterised in that** the intracochlear converter array (10) has a carrier (11) made of a biocompatible material which is biostable in the inner ear, preferably a polymer, in particular a silicone.

38. The system according to Claim 37, **characterised in that** the individual output side actuators (14) are embedded in the carrier (11) such that they are completely surrounded by a thin layer of the carrier material.

39. The system according to Claim 37 or 38, **characterised in that** mechanical attenuation elements (22) are embedded in the carrier (11) between the individual output side actuators (14), and these minimise mechanical wave propagation within the carrier to adjacent converters.

40. The system according to Claim 39, **characterised in that** the material of the attenuation elements (22) with a similar cross-sectional geometry to that of the carrier (11) is chosen such that there is a large mechanical impedance difference as compared to the carrier material in order to achieve high attenuation values.

41. The system according to Claim 34 or 35, **characterised by** an extracochlear array (24) of output side actuators (14), preferably produced using microsystem engineering, in the form of electromechanical converters.

42. The system according to Claim 41, **characterised in that** for the extracochlear converter array (24) a substrate (25) is provided which contains an electrical termination panel (36) co-produced using microsystem engineering and which is designed for the connection of a multi-pin, biocompatible actuator feed line (59) to the signal processing unit (34).

43. The system according to Claim 41 or 42, **characterised in that** for the extracochlear converter array (24) a substrate (25) is provided with an electronic module (37) co-produced using microsystems engineering.

44. The system according to Claim 43, **characterised in that** the electronic module (37) includes driver stages for triggering the output side actuators (14) and/or a decoding logic and converter modules for connection of a pin-reduced actuator feed line (59).

45. The system according to Claim 44, **characterised in that** the array connection consists of just three lines, in particular a ground line, a data line and a clock signal line, and the supply with electrical operating energy is implemented by phantom feed on the clock signal line.

46. The system according to any of Claims 42 to 45, **characterised in that** the electronic module (37) includes an interface module for digital data transmission via the actuator feed line (59), preferably by means of an optical fibre.

47. The system according to any of Claims 42 to 46, **characterised in that** the electronic module (37) includes D/A converters and driver modules associated with the actuators for serial data transmission on the actuator feed line (59).

48. The system according to Claim 3 and any of Claims 42 to 47, **characterised in that** the actuators (14) of the extracochlear converter array (24) each have an output side coupling element (28) that is designed such that it can project through an opening in the cochlear wall into a fluid-filled inner ear space.

49. The system according to any of Claims 36 to 48, **characterised in that** the output side actuators (14) in the converter array (10, 24) are arranged distributed equidistantly or logarithmic distances apart - according to the tonotopic frequency-location assignment - along the basilar membrane of the inner ear.

50. The system according to any of Claims 1 to 33, **characterised in that** the actuator arrangement has in combination an electromechanical converter (14) for mechanically stimulating the middle or inner ear and an intracochlear, electrically acting stimulation electrode array (26) with at least one stimulation electrode (27) for electrical stimulation of the inner ear.

51. The system according to any of Claims 1 to 33, **characterised by** a dual intracochlear arrangement (29) which has in combination an actuator arrangement with at least one actuator element (14) for at least indirect mechanical stimulation of the inner ear and an electrically acting stimulation electrode arrangement with at least one cochlear implant electrode (27) for electrical stimulation of the inner ear.

52. The system according to Claim 51, **characterised in that** the actuator arrangement has at least one intracochlear electromechanical converter (14) for direct mechanical stimulation of the inner ear.

53. The system according to Claim 51, **characterised in that** the actuator arrangement has at least one intracochlear hair cell stimulation electrode (27) for indirect mechanical stimulation of the inner ear by electrical stimulation of external hair cells.

54. The system according to Claims 52 and 53, **characterised in that** electromechanical converters (14) and cochlear implant electrodes (27) are arranged alternating with one another along a common carrier (30).

55. The system according to any of Claims 34 to 54, **characterised in that** the output side electromechanical converter(s) (14) is/are in the form of (an) electromagnetic, electrodynamic, piezoelectric, magnetostrictive or dielectric (capacitive) converter(s).

56. The system according to Claim 55, **characterised in that** the output side electromechanical converter(s) (14) is/are designed in application of the piezoelectric converter principle and using PZT ceramic (lead zirconate titanate) or PVDF (polyvinylidene fluoride).

57. The system according to any of Claims 34 to 56, **characterised in that** the output side electromechanical converter(s) (14) is/are preferably designed utilising geometric form transformations, in particular the bimorph principle, the unimorph principle or the heteromorph principle with passive material partners such that a maximum deflection at a given converter voltage with minimum electric power consumption is obtained.

58. The system according to any of the preceding claims, **characterised in that** it is designed as a binaural system for rehabilitation of a hearing disorder of both ears which has two system units which are each associated with one of the two ears.

59. The system according to Claim 58, **characterised in that** the two system units are essentially identical to one another.

60. The system according to Claim 58, **characterised in that** one system unit is designed as the master unit and the other system unit as the slave unit controlled by the master unit.

61. The system according to any of Claims 58 to 60, **characterised by** a wired implantable line connection (66) by means of which the signal processing modules (33) communicate with one another such that in both system units optimised binaural signal processing and converter array triggering are achieved.

62. The system according to any of Claims 58 to 60, **characterised by** a wireless connection (67), preferably a bidirectional high frequency section, via which the signal processing modules (33) communicate with one another such that in both system units optimised binaural signal processing and converter array triggering are achieved.

63. The system according to any of Claims 58 to 60, **characterised in that** the signal processing modules (33) communicate with one another using ultrasonic couplers (69) via a solid-borne sound-coupled ultrasonic section (68) such that in both system units optimised binaural signal processing and converter array triggering are achieved.

64. The system according to any of Claims 58 to 60, **characterised in that** implantable electrodes (72) are associated with the signal processing modules (33), and in the implanted state these are part of a data transmission section (73) which leads through body tissue of the wearer of the implant for communication of the signal processing modules of the two system units.

## Revendications

1. Système de réhabilitation d'un trouble auditif, qui comprend au moins un capteur acoustique (40) pour recevoir le signal acoustique et convertir celui-ci en signal audio électrique, une unité électronique de traitement de signaux (34, 62) pour traiter et amplifier le signal audio, une unité électrique d'alimentation (52) qui alimente en courant les composants individuels du système, et un dispositif actionneur (10, 14, 24, 26, 29) qui est pourvu d'un ou plusieurs actionneurs électroacoustiques, électromécaniques ou uniquement électriques, situés côté sortie, ou de n'importe quelle combinaison de ces actionneurs pour stimuler l'audition atteinte, étant précisé que l'unité de traitement de signaux (34, 62) comporte un module d'analyse ou de reconnaissance vocale (18) et un module de synthèse vocale (19) ; **caractérisé en ce que** le module d'analyse ou de reconnaissance vocale (18) comporte un dispositif pour reconnaître et extraire des informations supplémentaires concernant la prosodie de la parole, et **en ce que** le module de synthèse vocale (19) est pourvu d'un dispositif pour tenir compte de ces informations de prosodie lors de la synthèse vocale.

2. Système selon la revendication 1, **caractérisé en ce que** le dispositif pour reconnaître et extraire les informations de prosodie est conçu pour extraire la hauteur et la courbe de la fréquence de base vocale dans le cas de sons sonores, et le dispositif pour tenir compte des informations de prosodie lors de la synthèse vocale du côté sortie du système provoque une modulation correspondante du signal de sortie.

3. Système selon la revendication 1 ou 2, **caractérisé en ce que** l'unité de traitement de signaux (34, 62) comporte un processeur numérique de signaux (42) qui contient des modules (18, 19) réalisés comme des logiciels, pour l'analyse et la synthèse vocale.

4. Système selon la revendication 3, **caractérisé en ce que** les modules d'analyse ou de reconnaissance vocale et de synthèse vocale (18, 19) sont conçus pour être autodidactiques.

5. Système selon la revendication 3 ou 4, **caractérisé en ce que** les modules d'analyse ou de reconnaissance vocale et de synthèse vocale (18, 19) sont reprogrammables.

6. Système selon l'une des revendications précédentes, **caractérisé en ce que** les modules d'analyse ou de reconnaissance vocale et de synthèse vocale (18, 19) contiennent un réseau neuronal à réalisation numérique.

7. Système selon l'une des revendications précédentes, **caractérisé en ce que** les modules d'analyse ou de reconnaissance vocale et de synthèse vocale (18, 19) sont conçus pour que des catégories phonétiques soient transmises entre eux.

8. Système selon l'une des revendications précédentes, **caractérisé en ce que** les modules d'analyse ou de reconnaissance vocale et de synthèse vocale (18, 19) sont conçus pour que des catégories lexicales soient transmises entre eux.

9. Système selon l'une des revendications précédentes, **caractérisé en ce que** les modules d'analyse ou de reconnaissance vocale et de synthèse vocale (18, 19) sont aptes à être arrêtés pour traiter le signal audio sans analyse ni synthèse vocale, s'il n'y a pas de son parasite, ou que très peu.

10. Système selon la revendication 9, **caractérisé en ce qu'**il est prévu des moyens (21) pour arrêter automatiquement les modules d'analyse ou de reconnaissance vocale et de synthèse vocale (18, 19) en présence d'un niveau de son parasite faible.

11. Système selon la revendication 9 ou 10, **caractérisé en ce que** des moyens (21, 48, 54) pour arrêter les modules d'analyse ou de reconnaissance vocale et de synthèse vocale (18, 19) sont prévus grâce à une fonction de télécommande par l'utilisateur.

12. Système selon l'une des revendications précédentes, **caractérisé en ce que** l'unité de traitement de signaux (34, 62) contient des modules de logiciel qui permettent, parallèlement au fonctionnement de la prothèse auditive, de masquer un acouphène.

13. Système selon l'une des revendications précédentes, **caractérisé en ce que** l'unité de traitement de signaux (34, 62) comporte un dispositif de traitement préalable (41) pour l'amplification préalable et/ou le filtrage ainsi que pour la conversion analogique/numérique (A/N) des signaux de capteur acoustique.

14. Système selon la revendication 13, **caractérisé en ce que** le dispositif de traitement préalable (41) comprend un filtre antirepliement.

15. Système selon l'une des revendications précédentes, **caractérisé en ce que** s'il y a plusieurs capteurs acoustiques (40), un convertisseur analogique/numérique (41) est monté en aval de chacun des capteurs acoustiques.

16. Système selon l'une des revendications précédentes, **caractérisé par** au moins un convertisseur numérique/analogique (43) monté en aval du dispositif actionneur (10, 14, 24, 26, 29).

17. Système selon la revendication 16, **caractérisé en ce qu'**un convertisseur numérique/analogique (43) propre est monté en aval de chacun des actionneurs prévus côté sortie (14, 27).

18. Système selon l'une des revendications précédentes, **caractérisé en ce que** l'unité de traitement de signaux (34, 62) comporte un processeur numérique de signaux (42) pour traiter les signaux de capteur acoustique convertis A/N et éventuellement traités préalablement à l'aide du dispositif de traitement préalable (41) et/ou pour générer des signaux numériques pour un masquage d'acouphène.

19. Système selon l'une des revendications précédentes, **caractérisé en ce qu'**il est conçu pour être au moins en partie implantable et **en ce qu'**il est prévu un dispositif de télémétrie (46) sans fil, de préférence basé sur PC, pour transmettre des données entre une partie implantée (33) du système et une unité externe, en particulier un système de programmation externe (48).

20. Système selon la revendication 19, **caractérisé en ce qu'**il est prévu, associé au processeur de signaux (42), pour recevoir et reproduire un programme de commande, un dispositif de mémoire implantable (S₁, S₂) à inscription multiple, et des parties au moins du programme de commande peuvent être modifiées par des données transmises par l'unité externe (48) par l'intermédiaire du dispositif de télémétrie (46), ou échangées.

21. Système selon la revendication 20, **caractérisé en ce qu'**il est également prévu un dispositif de mémoire intermédiaire (S₄, S₅) dans lequel des données transmises par l'unité externe (48) par l'intermédiaire du dispositif de télémétrie (46) peuvent être stockées provisoirement avant d'être retransmises au processeur de signaux (42).

22. Système selon la revendication 21, **caractérisé en ce qu'**il est également prévu une logique de contrôle (44) pour soumettre les données stockées dans le dispositif de mémoire intermédiaire (S₄, S₅) à un contrôle avant leur retransmission au processeur de signaux (42).

23. Système selon l'une des revendications 19 à 22, **caractérisé par** un composant de microprocesseur (44), en particulier un microcontrôleur, pour commander à l'intérieur de l'implant les convertisseurs analogiques/numériques (41) et/ou les convertisseurs numériques/analogiques (43) et/ou le processeur de signaux (42) par l'intermédiaire d'un bus de données (50).

24. Système selon les revendications 22 et 23, **caractérisé en ce que** la logique de contrôle et le dispositif de mémoire intermédiaire (S₄, S₅) sont implémentés dans le composant de microprocesseur (44).

25. Système selon la revendication 23 ou 24, **caractérisé en ce que** par l'intermédiaire du bus de données (50) et du dispositif de télémétrie (46), des parties de programme ou des modules de logiciel entiers peuvent être transmis eux aussi entre le monde extérieur, le composant de microprocesseur (44) et le processeur de signaux (42).

26. Système selon l'une des revendications 23 à 25, **caractérisé en ce qu'**il est prévu, associé au composant de microprocesseur (44), un dispositif de mémoire implantable (S₃) pour stocker un programme de travail pour le composant de microprocesseur, et des parties au moins du programme de travail pour le composant de microprocesseur peuvent être modifiées ou échangées grâce à des données transmises par l'unité externe (48) par l'intermédiaire du dispositif de télémétrie (46).

27. Système selon l'une des revendications 20 à 26, **caractérisé en ce qu'**il est prévu au moins deux zones de stockage (S₁, S₂) pour recevoir et reproduire au moins le programme de commande du processeur de signaux (42).

28. Système selon l'une des revendications 21 à 27, **caractérisé en ce que** le dispositif de mémoire intermédiaire comporte au moins deux zones de mémoire (S₄, S₅) pour recevoir et reproduire des données transmises par l'unité externe (48) par l'intermédiaire du dispositif de télémétrie (46).

29. Système selon l'une des revendications 18 à 28, **caractérisé en ce qu'**il est également prévu, associée au processeur de signaux (42), une zone de mémoire morte (S₀) préprogrammée et non accessible en écriture.

30. Système selon l'une des revendications 18 à 29, **caractérisé en ce que** le dispositif de télémétrie (46) est conçu pour transmettre aussi des paramètres de fonctionnement entre la partie implantable (33) du système et l'unité externe (48).

31. Système selon l'une des revendications précédentes, **caractérisé en ce qu'**il est conçu pour être entièrement implantable et est pourvu d'au moins un capteur acoustique implantable (40), l'unité d'alimentation électrique comporte côté implant un élément de stockage électrique rechargeable (52), et il est prévu un chargeur transcutané sans fil (55, 56) pour charger l'élément de stockage.

32. Système selon la revendication 31, **caractérisé par** une télécommande sans fil (54) pour la commande, par le porteur de l'implant, des fonctions de l'implant.

33. Système selon l'une des revendications 1 à 31, **caractérisé en ce qu'**il est conçu pour être en partie implantable, étant précisé qu'au moins un capteur acoustique (40), l'unité électronique de traitement de signaux (62) pour le traitement et l'amplification de signaux audio, l'unité d'alimentation (52) et une unité modulateur/émetteur (63) sont contenus dans un module externe (64) à porter sur le corps, de préférence sur la tête au-dessus de l'implant (33'), et l'implant est énergétiquement passif et reçoit son énergie de fonctionnement et ses données de commande de convertisseur par l'intermédiaire de l'unité modulateur/émetteur prévue dans le module externe.

34. Système selon l'une des revendications précédentes, **caractérisé en ce qu'**il est prévu comme actionneurs côté sortie (14) des convertisseurs électromécaniques pour stimuler les espaces de l'oreille interne atteinte qui sont remplis de liquide, et **en ce que** l'unité de traitement de signaux (34, 62) comporte une électronique pilote de traitement de signaux (42, 44) qui commande électriquement chacun des convertisseurs de telle sorte qu'il se forme sur la membrane basilaire de l'oreille interne atteinte une configuration d'onde progressive qui approche du type de forme d'onde progressive d'une oreille interne saine, non atteinte.

35. Système selon la revendication 34, **caractérisé en ce que** les actionneurs côté sortie (14) sont conçus pour une stimulation directe des espaces de l'oreille interne atteinte qui sont remplis de liquide

36. Système selon la revendication 35, **caractérisé par** un ensemble intracochléaire (10) d'actionneurs côté sortie (14) sous la forme de convertisseurs électromécaniques.

37. Système selon la revendication 36, **caractérisé en ce que** l'ensemble intracochléaire de convertisseurs (10) comporte un support (11) composé d'un matériau biocompatible et biostable dans l'oreille interne, de préférence un polymère, en particulier un silicone.

38. Système selon la revendication 37, **caractérisé en ce que** les actionneurs individuels côté sortie (14) sont insérés dans le support (11) de telle sorte qu'ils sont complètement entourés par une fine couche du matériau de support.

39. Système selon la revendication 37 ou 38, **caractérisé en ce qu'**il est prévu, insérés dans le support (11) entre les actionneurs individuels côté sortie (14), des éléments d'amortissement mécaniques (22) qui minimalisent la propagation mécanique des ondes à l'intérieur du support vers des convertisseurs voisins.

40. Système selon la revendication 39, **caractérisé en ce que** le matériau des éléments d'amortissement (22), pour une géométrie de section transversale semblable à celle du support (11), est choisi pour qu'il y ait une grande différence d'impédance mécanique par rapport au matériau de support, pour obtenir des valeurs d'amortissement élevées.

41. Système selon la revendication 34 ou 35, **caractérisé par** un ensemble extracochléaire (24), fabriqué de préférence selon la technique des microsystèmes, d'actionneurs côté sortie (14) sous la forme de convertisseurs électromécaniques.

42. Système selon la revendication 41, **caractérisé en ce qu'**il est prévu pour l'ensemble extracochléaire de convertisseurs (24) un substrat (25) qui contient une zone de connexion électrique (36), fabriquée en même temps suivant la technique des microsystèmes, qui est conçue pour la connexion d'un fil biocompatible multipolaire d'alimentation d'actionneur (59) vers l'unité de traitement de signaux (34).

43. Système selon la revendication 41 ou 42, **caractérisé en ce qu'**il est prévu pour l'ensemble extracochléaire de convertisseurs (24) un substrat (25) avec un module électronique (37) fabriqué en même temps selon la technique des microsystèmes.

44. Système selon la revendication 43, **caractérisé en ce que** le module électronique (37) contient des étages d'attaque pour commander les actionneurs côté sortie (14) et/ou une logique de décodage et des composants convertisseurs pour la connexion d'un fil d'alimentation d'actionneur (59) à pôle réduit.

45. Système selon la revendication 44, **caractérisé en ce que** la connexion d'ensemble se compose de trois fils seulement, en particulier un fil de masse, un fil de données et un fil de signaux d'horloge, et l'alimentation en énergie électrique de fonctionnement se fait grâce à une alimentation fantôme sur le fil de signaux d'horloge.

46. Système selon l'une des revendications 42 à 45, **caractérisé en ce que** le module électronique (37) contient un composant d'interface pour une transmission numérique de données par l'intermédiaire du fil d'alimentation d'actionneur (59), de préférence à l'aide d'un conducteur de lumière.

47. Système selon l'une des revendications 42 à 46, **caractérisé en ce que** le module électronique (37), dans le cas d'une transmission de données en série sur le fil d'actionneur (59), contient des convertisseurs N/A et des composants d'attaque correspondants associés aux actionneurs (14).

48. Système selon la revendication 3 et l'une des revendications 42 à 47, **caractérisé en ce que** les actionneurs (14) de l'ensemble extracochléaire de convertisseurs (24) comportent chacun un élément de couplage côté sortie (28) qui est conçu pour pouvoir dépasser dans un espace de l'oreille interne rempli de liquide, à travers une ouverture de la paroi cochléaire.

49. Système selon l'une des revendications 36 à 48, **caractérisé en ce que** les actionneurs côté sortie (14) sont répartis dans l'ensemble de convertisseurs (10, 24) à égale distance ou à des distances logarithmiques - suivant la tonotopie fréquence-lieu le long de la membrane basilaire de l'oreille interne.

50. Système selon l'une des revendications 1 à 33, **caractérisé en ce que** la disposition combinée des actionneurs comporte un convertisseur électromécanique (14) pour la stimulation mécanique de l'oreille moyenne ou interne, et un ensemble intracochléaire d'électrodes d'excitation à action électrique (26) avec au moins une électrode d'excitation (27) pour la stimulation électrique de l'oreille interne.

51. Système selon l'une des revendications 1 à 33, **caractérisé par** un dispositif intracochléaire double (29) qui comporte une combinaison d'un dispositif actionneur avec au moins un élément actionneur (14) pour la stimulation mécanique au moins indirecte de l'oreille interne, et un dispositif à électrode d'excitation à action électrique avec au moins une électrode d'implant cochléaire (27) pour la stimulation électrique de l'oreille interne.

52. Système selon la revendication 51, **caractérisé en ce que** le dispositif actionneur comporte au moins un convertisseur intracochléaire électromécanique (14) pour la stimulation mécanique directe de l'oreille interne.

53. Système selon la revendication 51, **caractérisé en ce que** le dispositif actionneur comporte au moins une électrode intracochléaire d'excitation de cellules ciliées (27) pour la stimulation mécanique indirecte de l'oreille interne grâce à une excitation électrique de cellules ciliées extérieures.

54. Système selon l'une des revendications 52 et 53, **caractérisé en ce que** des convertisseurs électromécaniques (14) et des électrodes d'implant cochléaire (27) sont disposés en alternance le long d'un support commun (30).

55. Système selon l'une des revendications 34 à 54, **caractérisé en ce que** le ou les convertisseurs électromécaniques côté sortie (14) sont conçus comme des convertisseurs électromagnétiques, électrodynamiques, piézoélectriques, magnétostrictifs ou diélectriques (capacitifs).

56. Système selon la revendication 55, **caractérisé en ce que** le ou les convertisseurs électromécaniques côté sortie (14), dans le cas de l'application du principe de convertisseur piézoélectrique, sont construits à l'aide de céramique PZT (plomb-zirconate-titanate) ou de PVDF (polyfluorure de vinylidène).

57. Système selon l'une des revendications 34 à 56, **caractérisé en ce que** le ou les convertisseurs électromécaniques côté sortie (14), de préférence à l'aide de transformations de forme géométrique, en particulier du principe bimorphe, du principe unimorphe ou du principe hétéromorphe avec des partenaires de matériau passifs, sont réalisés de telle sorte que pour une tension de convertisseur donnée, on obtienne une déviation maximale pour une puissance absorbée minimale.

58. Système selon l'une des revendications précédentes, **caractérisé en ce qu'**il est conçu comme un système binaural pour la réhabilitation d'un trouble auditif des deux oreilles, qui comporte deux unités de système associées chacune à l'une des deux oreilles.

59. Système selon la revendication 58, **caractérisé en ce que** les deux unités de système sont globalement identiques.

60. Système selon la revendication 58, **caractérisé en ce qu'**une unité de système est conçue comme une unité maître, et l'autre comme une unité esclave commandée par l'unité maître.

61. Système selon l'une des revendications 58 à 60, **caractérisé par** une liaison implantable à fil (66) par l'intermédiaire de laquelle les modules de traitement de signaux (33) communiquent entre eux de telle sorte qu'on obtienne dans les deux unités de système un traitement de signal binaural et une commande d'ensemble de convertisseurs optimisés.

62. Système selon l'une des revendications 58 à 60, **caractérisé par** une liaison sans fil (67), de préférence une liaison haute fréquence bidirectionnelle, par l'intermédiaire de laquelle les modules de traitement de signaux (33) communiquent entre eux de telle sorte qu'on obtienne dans les deux unités de système un traitement de signal binaural et une commande d'ensemble de convertisseurs optimisés.

63. Système selon l'une des revendications 58 à 60, **caractérisé en ce que** les modules de traitement de signaux (33) communiquent entre eux à l'aide de coupleurs à ultrasons (69) par l'intermédiaire d'une liaison à ultrasons couplée par bruit de corps (68) de telle sorte qu'on obtienne dans les deux unités de système un traitement de signal binaural et une commande d'ensemble de convertisseurs optimisés.

64. Système selon l'une des revendications 58 à 60, **caractérisé en ce qu'**il est prévu, associées aux modules de traitement de signaux (33), des électrodes implantables (72) qui, à l'état implanté, font partie d'une liaison de transmission de données (73) qui traverse les tissus du corps du porteur d'implant, pour une communication des modules de traitement de signaux des deux unités de système.
